# EUROPEAN PATENT APPLICATION

(11) **EP 4 631 951 A1**
(43) Date of publication of application: **15.10.2025**
(21) Application number: 24170124.2
(22) Date of filing: 13.04.2024
(51) Int. Cl.: C07H 15/04, C07H 15/12, C07K 16/12, A61P 1/02, A61P 29/00, A61K 31/70

(54) **SYNTHETIC VACCINES AGAINST PORPHYROMONAS GINGIVALIS**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a synthetic saccharide of general formula (**I**) that is related to *Porphyromonas gingivalis* lipopolysaccharide specifically the O-antigen, a conjugate thereof and the use of said saccharide and conjugate for raising a protective immune response in a human and/or animal host. Furthermore, the synthetic saccharide of general formula (**I**) is useful as marker in immunological assays for detection of antibodies against *Porphyromonas gingivalis* bacteria. Another aspect of the present invention is directed to a monoclonal antibody having specificity for a synthetic saccharide of general formula (**I**).

## Description

### Field of the invention

The present invention relates to a synthetic saccharide of general formula (**I**) that is related to *Porphyromonas gingivalis* lipopolysaccharide specifically the O-antigen, a conjugate thereof and the use of said saccharide and conjugate for raising a protective immune response in a human and/or animal host. Furthermore, the synthetic saccharide of general formula (**I**) is useful as marker in immunological assays for detection of antibodies against *Porphyromonas gingivalis* bacteria. Another aspect of the present invention is directed to a monoclonal antibody having specificity for a synthetic saccharide of general formula (**I**).

### Background of the invention

*Porphyromonas gingivalis (P. gingivalis)* is a Gram-negative, anaerobic, rod-shaped bacterium, that colonizes the oral epithelium and is an important component of subgingival microbiomes (Mei et al., Pathogens 2020, 9, 944). Besides *Tannerella forsythia* and *Treponema denticola,* it is part of the so called "red complex" that includes the main pathogens in the development of chronic periodontitis (CP). Studies have demonstrated that *P. gingivalis* strains vary in their virulence, with some strains being classified as virulent, e.g. strains W50, ATCC 49417 and A7A1 and others being classified as avirulent, e.g. strains 381, 33277 and 23A4. W50 is worldwide the most common type of *P. gingivalis* (Igboin et al., J. Clin. Microbiol. 2009, 47, 3073-3081).

Among surface components of the bacterial envelope, polysaccharides such as the K-antigen (capsule) and the lipopolysaccharide (LPS) are essential for virulence and immunogenicity of *P. gingivalis.* The main virulence factors include its own structural components (lipopolysaccharide (LPS), fimbriae, heat shock proteins) and secretory components (gingipains and outer membrane vesicles).

Outer membrane vesicles of *P. gingivalis* are enriched in many virulence factors including the LPS and proteins such as well-studied gingipains and the peptidyl-arginine deiminase (PPAD). Outer membrane vesicles participate in biofilm development, host interaction, colonization and immune defense evasion. They are double-layer spherical membrane-like structures with a diameter of about 50-250 nm and due to their size, they can easily penetrate deep tissues compared to the parent bacterium. They are released into the hosts tissue and can be internalized into host-cells (Gui et al. Mol. Oral Microbiol. 2016, 31, 365-378).

The peptidoglycan layer of the gram-negative bacterium *P. gingivalis* is surrounded by an outer membrane that contain, among others, lipopolysaccharides (LPS) organized in vesicles. Those outer membrane vesicles can be internalized into host cells. After lysis of the outer membrane vesicle, various antigens from the outer membrane and the underlying periplasm may be processed by antigen-presenting cells leading to the induction of an adaptive immune response including *P. gingivalis-*specific antibody production.

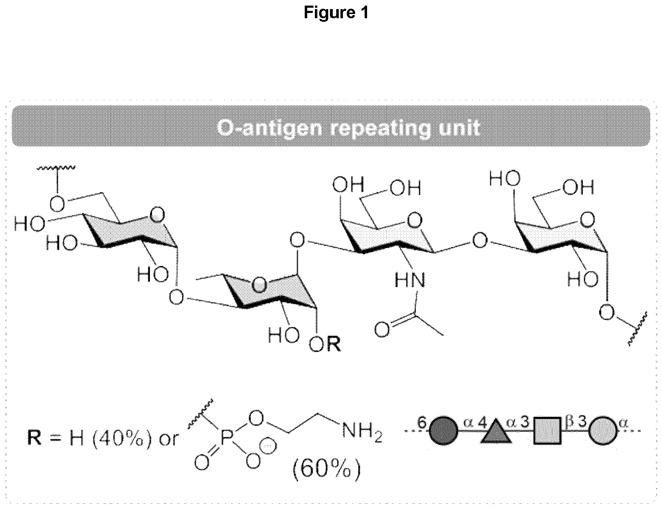

The LPS is the major component of the outer bacterial membrane. It is a complex glycolipid that is composed of three covalently linked domains: The lipid A or endotoxin, the central oligosaccharide core and the O-antigen, which is the most external part of this molecule (Raetz et al. Annu. Rev. Biochem. 2002, 71, 635-700). The glycan part in the O-antigen (O-LPS) of *P. gingivalis* W50 is built up of the tetrasaccharide repeating sequence [→6)-α-D-Glcp-(1→4)-α-L-Rhap-(1→3)-β-D-GalNAc- (1→3)-α-D-Galp-(1→] and bears a monophosphoethanolamine residue at position C2 of the α-rhamnose in a nonstoichiometric (approximately 60%) amount (see Figure 1). The LPS of *P. gingivalis* is a significant antigen in periodontitis patients, which offers opportunities for the development of a carbohydrate-based conjugate vaccine.

Periodontal diseases are very prevalent and constitute a major public health problem. According to the World Health Organization, 10-15% of adults worldwide suffer from severe periodontal diseases. Regarding the described connection to systemic diseases, periodontal disease has immense social impact and poses a huge challenge towards global health care. Since *P. gingivalis* is the major cause for periodontal diseases, it is an important target in research on systemic diseases. Therefore, a significant amount of research and extensive analysis of *P. gingivalis* over the past decades resulted in evidences of its role in atherosclerosis, Alzheimer's disease, rheumatoid arthritis, cancer, diabetes, and adverse pregnancy outcomes. The ability of *P. gingivalis* to travel to distant sites might participate in *P. gingivalis-*associated systemic disorder. *P. gingivalis* in local periodontal tissue can enter the vasculature through ulcerated epithelium and lymph vessels by brushing and chewing. This was indicated by the detection of *P. gingivalis* in synovial fluid and plasma as well as by isolation of *P. gingivalis* from human atherosclerotic plaque tissues, mouse lungs, human Alzheimer's brains, aortic endothelial cells, pancreatic tumor cells, and human myeloid dendritic cells. Periodontal bacteria also have been found in cardiovascular disease plaques and the detection rate of *P. gingivalis* has been 100%.

Alzheimer's disease (AD), the most common type of dementia, is a chronic neurodegenerative disease where patients suffer from progressive memory loss, disorientation, and cognitive performance deficits. The number of dementia cases is projected to reach more than 140 million by 2050 and thus AD is a major challenge for global health and social care. Until now, the current understanding of AD pathogenesis is limited, which hinders efficient treatment and prevention. However, epidemiological studies, animal experiments and other evidence have strengthened support for the possible relevance of *P. gingivalis* in AD pathogenesis. For example, DNA, LPS and gingipains of *P. gingivalis* have been identified in AD brains and oral *P. gingivalis* infection in mice resulted in an increased production of amyloid-β₁₋₄₂ (Aβ₁₋₄₂) plaques. The pathology of AD is based on Aβ plaques, neurofibrillary tangles and microglia-mediated neuroinflammation. Studies suggest that infection with *P*. *gingivalis* can lead to direct disruption of the blood-brain barrier, or bacteria find leaky regions lacking of blood-brain barrier, invade peripheric nerves or invade immune cells followed by brain recruitment and finally enter into the brain causing neuroinflammation. In addition to the direct role of *P. gingivalis,* the release of inflammatory molecules from infected host cells, such as a set of cytokines and their immune response may also be involved in AD pathogenesis. Another theory is the suppression of the host's adaptive immune system, which could allow *P. gingivalis* to prevent the entry of immune cells into the brain, or an increased blood-brain barrier permeability, or the inhibition of the local IFN-γ response.^{]}

The current CP control measures heavily rely on the mechanical removal of dental plaque, which disrupts the disease-triggering dysbiotic biofilm. This includes toothbrushing, interdental cleaning, frequent professional dental cleaning, but also pocket reduction surgery, regenerative surgery, laser therapy and local or systemic antimicrobials.

Many researchers try to explore whether controlling *P. gingivalis* can be a potential treatment of systemic diseases. Proposed approaches include broad-spectrum antibiotics and specific inhibitors. Doxycycline and metronidazole resulted in significant reduction in atherosclerotic lesions in mice. Small molecule gingipain inhibitors have been found to block gingipain-induced neurodegeneration, suggesting that it could be used to treat *P. gingivalis* brain colonization and neurodegeneration in AD.

However, pre-immunization could reduce the ability of the bacteria to translocate to remote tissues. For a successful periodontal vaccine, immunity in the oral cavity should be induced, which is difficult with traditional vaccination methods. Advances in mucosal vaccination strategies might give the option for periodontal disease control. The intranasal route is a major mucosal route and the nasal influenza vaccine was the pioneering application in humans. Sublingual immunization with a recombinant vaccine that contains *P. gingivalis* heat shock protein 60 or nasal immunization with outer membrane vesicles prior to *P. gingivalis* injection significantly reduced arteriosclerotic lesion in mice (Koizumi et al. Infect. Immun. 2008, 76, 2958-2965; Hagiwara et al. J. Dent. Res. 2014, 93, 382-387). The nasal immunization with outer membrane vesicles in mice resulted in the detection of a significant increase in *P*. *gingivalis* specific IgA in the nasal lavage fluid and saliva of mice, as well as serum IgG and IgA. In view of the encouraging results, it is of high interest to provide novel therapeutic strategies for systemic diseases caused by *P. gingivalis.*

It is the objective of the present invention to provide a saccharide of general formula (**I**) that is related to the *Porphyromonas gingivalis* lipopolysaccharide, specifically the O-antigen, as well as a conjugate of the saccharide of general formula (**I**) with an immunogenic carrier, such as a carrier protein, as well as an antibody having specificity for the saccharide of general formula (**I**). The saccharide of general formula (**I**), and particularly the conjugate of said saccharide with an immunogenic carrier is able to raise a protective immune response against *P. gingivalis* LPS O-antigen in a human and/or animal host. Thus, a vaccine composition for immunization against *P. gingivalis* comprising the saccharide of general formula (**I**), and/or a conjugate thereof is provided. Furthermore, the synthetic saccharide of general formula (**I**) is useful as marker in immunological assays for detection of antibodies against *P. gingivalis* bacteria.

The objective of the present invention is solved by the teaching of the independent claims. Further advantageous features, aspects and details of the invention are evident from the dependent claims, the description, the figures, and the examples of the present application.

### Description of the invention

### Definitions

The term *"linker"* as used herein encompasses molecular fragments capable of connecting the reducing-end monosaccharide of a saccharide with an immunogenic carrier or a solid support, optionally by binding to at least one interconnecting molecule. Thus, the function of the linker *per se* or together with the interconnecting molecule is to establish, keep and/or bridge a special distance between the reducing-end monosaccharide and an immunogenic carrier or a solid support. More specifically, one extremity of the linker is connected to the exocyclic oxygen atom at the anomeric center of the reducing-end monosaccharide and the other extremity is connected *via* the nitrogen atom with the interconnecting molecule, or directly with the immunogenic carrier or the solid support.

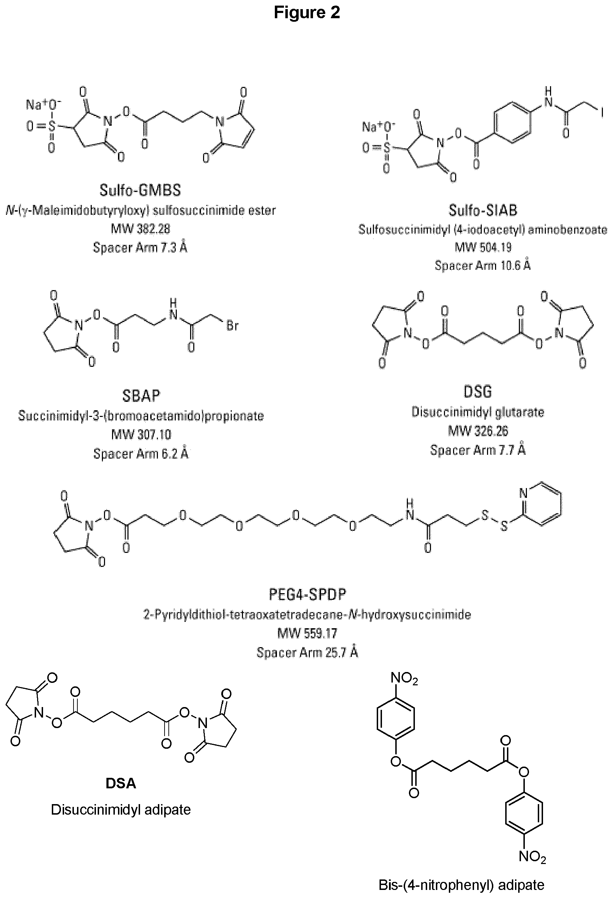

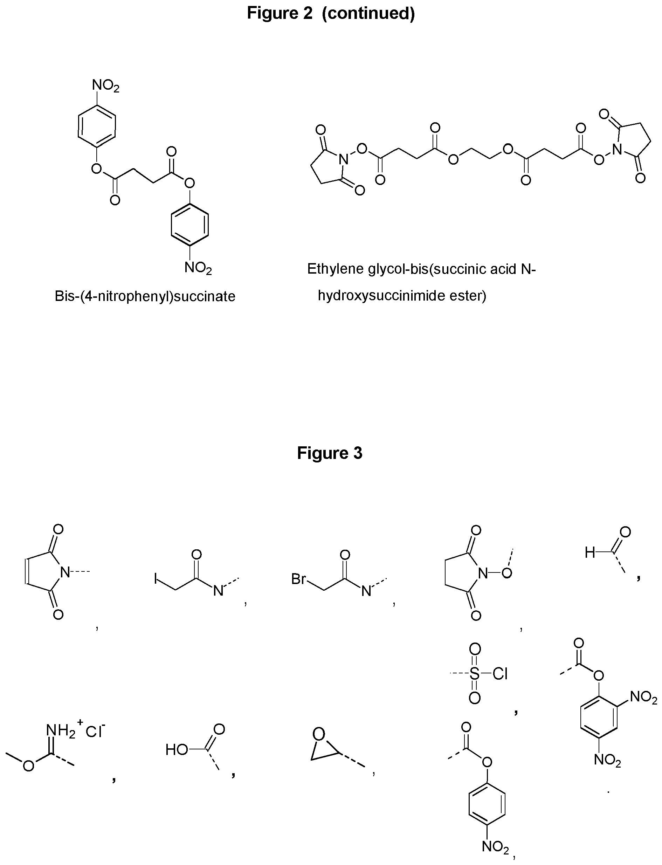

As used herein, the term *"interconnecting molecule"* refers to a bifunctional molecule containing functional group X and functional group Y, wherein functional group X is capable of reacting with the terminal amino group on the linker L and the functional group Y is capable of reacting with a functionality present on an immunogenic carrier or on a solid support. Figure 2 displays examples of commercially available interconnecting molecules, but does not restrict the interconnecting molecules that can be used according to the present invention to the examples displayed herein.

The term *"adjuvant'* as used herein refers to an immunological adjuvant i.e. a material used in a vaccine composition that modifies or augments the effects of said vaccine by enhancing the immune response to a given antigen contained in the vaccine without being antigenically related to it. For the person skilled in the art, classically recognized examples of adjuvants include:
- mineral-containing compositions, including calcium salts and aluminium salts (or mixtures thereof). Calcium salts include calcium phosphate. Aluminium salts include hydroxides, phosphates, sulfates, etc., with the salts taking any suitable form (e.g. gel, crystalline, amorphous, etc.). Adsorption to these salts is preferred. The mineral containing compositions may also be formulated as a particle of metal salt. The adjuvants known as aluminium hydroxide and aluminium phosphate may be also used. The invention can use any of the "hydroxide" or "phosphate" adjuvants that are in general used as adjuvants. The adjuvants known as "aluminium hydroxide" are typically aluminium oxyhydroxide salts, which are usually at least partially crystalline. The adjuvants known as "aluminium phosphate" are typically aluminium hydroxyphosphates, often also containing a small amount of sulfate (i. e. aluminium hydroxyphosphate sulfate). They may be obtained by precipitation, and the reaction conditions and concentrations during precipitation influence the degree of substitution of phosphate for hydroxyl in the salt. Mixtures of both an aluminium hydroxide and an aluminium phosphate can be employed in the formulation according to the present invention;
- saponins, which are a heterologous group of sterol glycosides and triterpenoid glycosides that are found in the bark, leaves, stems, roots and even flowers of a wide range of plant species. Saponins from the bark of the *Quillaia saponaria,* Molina tree have been widely studied as adjuvants. Saponins can also be commercially obtained from Smilax ornata (sarsaprilla), Gypsophilla paniculata (brides veil), and Saponaria oficianalis (soap root). Saponin adjuvant formulations include purified formulations, such as QS21, as well as lipid formulations, such as ISCOMs. Saponin compositions have been purified using HPLC and RP-HPLC. Specific purified fractions using these techniques have been identified, including QS7, QS 17, QS 18, QS2 1, QH-A, QH-B and QH-C. Saponin formulations may also comprise a sterol, such as cholesterol. Combinations of saponins and cholesterols can be used to form unique particles called immunostimulating complexes (ISCOMs). ISCOMs generally include a phospholipid such as phosphatidylethanolamine or phosphatidylcholine. Any known saponin can be used in ISCOMs. Preferably, the ISCOM includes one or more of QuilA, QHA & QHC;

- microparticles (i.e. a particle of 100 nm to 150 pm in diameter, more preferably 200 nm to 30 pm in diameter, or 500 nm to 10 pm in diameter) formed from materials that are biodegradable and non-toxic. Such non-toxic and biodegradable materials include, but are not restricted to poly(α-hydroxy acid), polyhydroxybutyric acid, polyorthoester, polyanhydride, polycaprolactone;
- CD1d ligands, such as an α-glycosylceramide, phytosphingosine-containing α-glycosylceramides, OCH, KRN7000 [(2*S*,3*S*,4*R*)-1-O-(α-D-galactopyranosyl)-2-(*N-*hexacosanoylamino)-1,3,4-octadecanetriol], CRONY- 101, 3"-sulfo-galactosyl-ceram ide;
- immunostimulatory oligonucleotides, such CpG motif containing ones (a dinucleotide sequence containing an unmethylated cytosine residue linked by a phosphate bond to a guanosine residue), or Cpl motif containing ones (a dinucleotide sequence containing cytosine linked to inosine), or a double-stranded RNA, or an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded;
- compounds containing lipids linked to a phosphate-containing acyclic backbone, such as the TLR4 antagonist E5564;
- oil emulsions (e.g. Freund's adjuvant).

Theoretically, each molecule or substance that is able to favor or amplify a particular situation in the cascade of immunological events, ultimately leading to a more pronounced immunological response, can be defined as an adjuvant.

In principle, through the use of adjuvants in vaccine formulations, one can
- direct and optimize immune responses that are appropriate or desirable for the vaccine;
- enable mucosal delivery of vaccines, i.e. administration that results in contact of the vaccine with a mucosal surface such as buccal or gastric or lung epithelium and the associated lymphoid tissue;
- promote cell-mediated immune responses;
- enhance the immunogenicity of weaker immunogens, such as highly purified or recombinant antigens;
- reduce the amount of antigen or the frequency of immunization required to provide protective immunity; and
- improve the efficacy of vaccines in individuals with reduced or weakened immune responses, such as newborns, the aged, and immunocompromised vaccine recipients.

Although little is known about their mode of action, it is currently believed that adjuvants augment immune responses by one of the following mechanisms:
- increasing the biological or immunologic half-life of antigens;
- improving antigen delivery to antigen-presenting cells (APCs), as well as antigen processing and presentation by the APCs e.g., by enabling antigen to cross endosomal membranes into the cytosol after ingestion of antigen-adjuvant complexes by APC;
- mimicking danger inducing signals from stressed or damaged cells, which serve to initiate an immune response;
- inducing the production of immunomodulatory cytokines;
- biasing the immune response towards a specific subset of the immune system; and
- blocking the rapid dispersal of the antigen challenge.

Saccharides are known by the person skilled in the art as TI-2 (T cell independent-2) antigens and poor immunogens. Therefore, to produce a saccharide-based vaccine, said saccharide is conjugated to an immunogenic carrier to provide a conjugate, which presents an increased immunogenicity in comparison with the saccharide. In this context the term "immunogenic carrier" is defined as a structure, which is conjugated to the saccharide to form a conjugate that presents an increased immunogenicity in comparison with the saccharide *per se*. Thus, the conjugation of the saccharides to the immunogenic carrier has as effect the stimulation of the immune response against said saccharide, without inducing an immune response against the said immunogenic carrier.

Surprisingly, it was found that a pure saccharide of general formula (**I**) according to the present invention contains a protective immunogenic glycan epitope and is able to induce a protective immune response against *Porphyromonas gingivalis* bacteria in a human and/or animal host. The saccharide of general formula (**I**) elicits antibodies that are cross-reacting with the *P. gingivalis* lipopolysaccharide, recognize specifically *P. gingivalis* bacteria.

Thus, the present invention relates to a saccharide of general formula (**I**)

**H-Uₓ₊₂-Uₓ₊₁-Uₓ-T-O-L-NH₂** (**I**)

wherein
x is an integer selected from 1 and 3;
U₁ = U₅ = 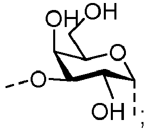 U₂ = U₆ = 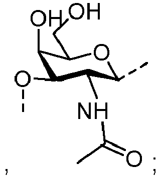
U3 = 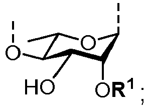 U₄ = 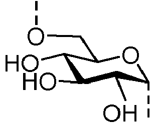
-T- represents a bond, -Uₓ₊₃-, -Uₓ₊₃-Uₓ₊₂-, -Uₓ₊₃-Uₓ₊₂-Uₓ₊₁-, or -[Uₓ₊₃-Uₓ₊₂-Uₓ₊₁-Uₓ]ₙ- with n being an integer selected from 1, 2 and 3;
R¹ represents -H or -PO₂-O-CH₂CH₂-NH₂;
L represents a linker; or a pharmaceutically acceptable salt thereof
-L- is defined as a linker and is part of the fragment -O-L-NH₂. Thus, the linker -L- is bound to an oxygen atom and to the nitrogen atom of the NH₂-group. It is preferred that at least two carbon atoms of the linker are between the oxygen atom and the NH₂-group, like -O-C-C-NH₂. The linker -L- can be an aliphatic chain, wherein the aliphatic chain can optionally include an aromatic chain inserted in it, or a number of heteroatoms oscillating from 0 to 10.

The linker L preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms.

The shortest atom chain between the oxygen atom (i.e. the oxygen of -O-L-NH₂) and the NH₂-group consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the NH₂-group) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker -L-, or the shortest chain is fully or partially fluorinated. The linker -L- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker -L- may also contain amide (-NH-CO-, -CO-NH-) and/or urea (-NH-CO-NH-) residues and preferably only one amide or urea residue. The linker may also contain substituents and preferably two substituents, such as R¹⁰ and R¹¹, or four substituents such as R¹⁰, R¹¹, R¹⁵ and R¹⁴, which have the meanings as defined herein and which are preferably selected from: -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂H₅)₂;

In case the linker -L- is fluorinated, more than two substituents -F are preferred.

Preferably the linker -L- is selected from: -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -CF₂-, -(CF₂)₂-, -(CF₂)₃-, -(CF₂)₄-, -(CF₂)₅-, -(CF₂)₆-, -(CF₂)₇-, -(CF₂)₈-, -(CF₂)₉-, -(CF₂)₁₀-, -(CH₂)₂-O-(CH₂)₂-, -CH₂-O-(CH₂)₃-, -(CH₂)₃-O-CH₂-, -CH₂-O-(CH₂)₂-, -(CH₂)₂-O-CH₂-, -(CH₂)₃-O-(CH₂)₂-, -(CH₂)₂-O-(CH₂)₃-, -(CH₂)₄-O-CH₂-, -CH₂-O-(CH₂)₄-, -L^{a}-, -L^{a}-L^{e}-, -L^{a}-L^{b}-L^{e}-, -L^{a}-L^{b}-L^{d}-L^{c}-L^{e}-, -L^{a}-L^{d}-L^{e}-;
wherein
-L^{a}- is selected from: -(CH₂)ₒ-, -(CF₂)ₒ-, -(CH₂-CH₂-O)ₒ-C₂H₄-, -(CH₂-CH₂-O)ₒ-CH₂-, -(CR¹⁰R¹¹)ₒ-, 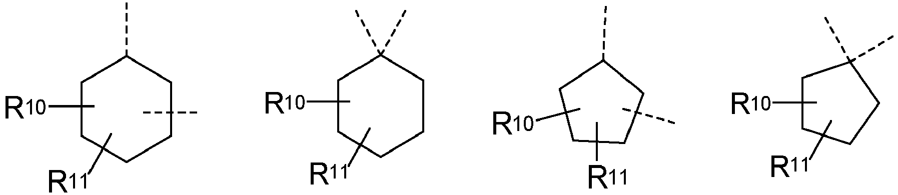 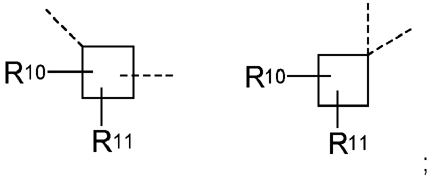
-L^{b}- and -L^{c}- are independently of each other selected from: -O-, -NH-C(O)-NH-, -NH-C(S)-NH-, -NH-C(O)-, -C(O)-NH-, -NH-C(O)-O-, -NR⁹-, -NR¹⁸-, -SO₂-, 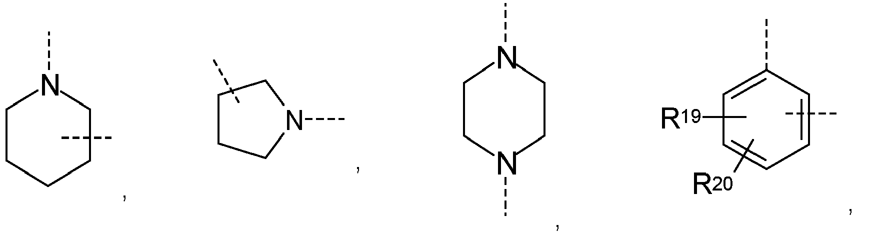 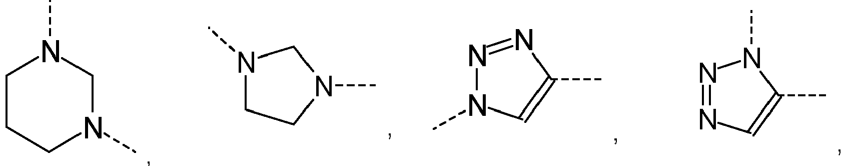 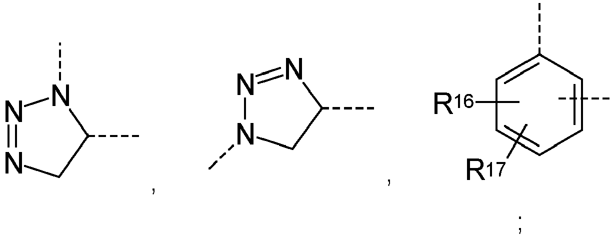
-L^{d}- represents -(CH₂)_{q}-, -(CF₂)_{q}-, -(CR¹²R¹³)_{q}-, -(CH₂-CH₂-O)_{q}-C₂H₄-, -(CH₂-CH₂-O)_{q}-CH₂-, 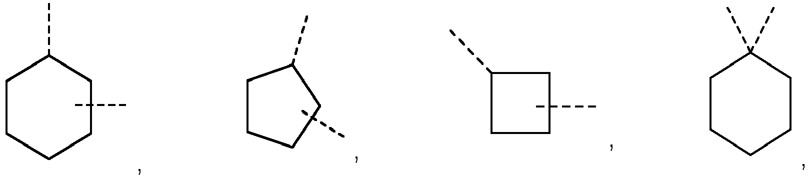 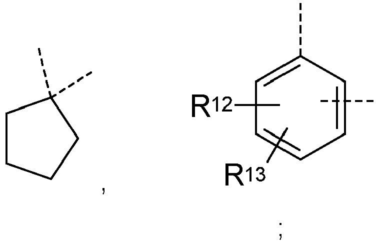
-L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁-, -(CH₂)ₚ₁-O-(CH₂)ₚ₂-, -(CR¹⁴R^{1S})ₚ₁-, -(CR¹⁴R¹⁵)ₚ₁-O-(CR²¹R²²)ₚ₂-, 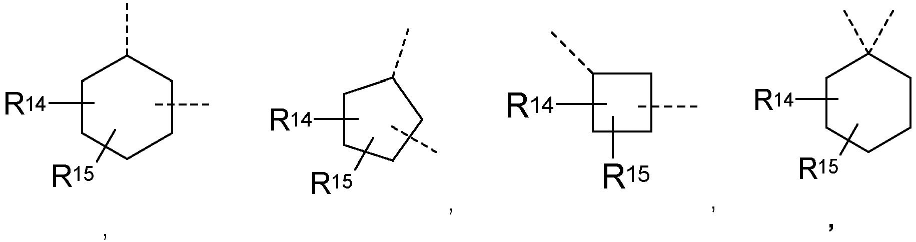 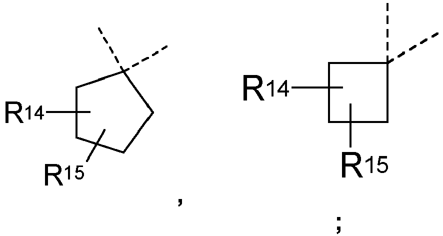
R⁹ and R¹⁸ are independently of each other selected from: -CH₃, -C₂H₅, -C₃H₇, and -C(O)CH₃;
R¹⁰, R¹¹, R¹², R¹³, R¹⁴, R¹⁵, R¹⁶, R¹⁷, R¹⁹, R²⁰, R²¹ and R²² are independently of each other selected from: -H, -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂ and -N(C₂H₅)₂;
o, q, p1 and p2 are independently of each other an integer selected from 1, 2, 3, 4, 5, 6, 7, 8, 9, and 10.

Preferably, the linker -L- is selected from: -L^{a}-, -L^{a}-L^{e}-, -L^{a}-L^{b}-L^{e}- and
-L⁸-L^{d}-L^{e}-;
-L^{a}- represents -(CH₂)ₒ-, -(CH₂-CH₂-O)ₒ-C₂H₄-, -(CH₂-CH₂-O)ₒ-CH₂-;
-L^{b}- represents -O-; -NH-CO-NH-, -NH-CO-CH₂-NH-, -NH-CO-;
-L^{d}- represents -(CH₂)_{q}-, -(CF₂)_{q}-, -(CH₂-CH₂-O)_{q}-C₂H₄-, or -(CH₂-CH₂-O)_{q}-CH₂-;
-L^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-;
o, q, p1 and p2 are independently of each other an integer selected from 1, 2, 3, 4, 5, and 6.

Preferably, the linker -L- represents -(CH₂)ₒ- and o is an integer selected from 1, 2, 3, 4, 5, 6, 7 and 8.

The saccharides of the present invention bear basic and/or acidic substituents and they may form salts with organic or inorganic acids or bases.

Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples of suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (**I**) with a solution of a base, selected out of the group mentioned above.

It is clear for the skilled person in the art of carbohydrate chemistry that the saccharides of general (**I**) are not containing -O-O- bonds and or sugar fragments (Uₓ, Uₓ₊₁, Uₓ₊₂, Uₓ₊₃) connected or bound to each other *via* their anomeric or C-1 carbons. It is also clear for the person skilled in the art that the stereochemistry of the glycosidic bond is the stereochemistry indicated for the anomeric center of the sugar fragment in the general formula. Hence, the stereochemistry of the anomeric center for sugar fragment U₁ and U₅ is α, for sugar fragment U₂ and U₆ is β, for sugar fragment U₃ is α and for sugar fragment U₄ is α.

The saccharide of general formula (**I**) contains a protective immunogenic epitope and is able to induce a protective immune response against *P. gingivalis* bacteria in a human and/or animal host. The saccharide of general formula (**I**) elicits antibodies that are cross-reacting with the *P. gingivalis* lipopolysaccharide, recognize specifically *P. gingivalis* bacteria. Additionally, the inventive saccharides have the advantage that these are pure synthesized compounds, which can be easily manufactured in accordance with GMP regulations.

Thus, the vaccine composition of the present invention contains most preferably only one single compound of the general formula (**I**) bound to an immunogenic carrier, preferably a carrier protein and more preferably CRM₁₉₇. Thus, the compound of the general formula (**I**) is useful for the preparation of well defined, well characterized and pure vaccines containing only one synthetically prepared and well characterized tri-, tetra-, penta-, hexa-, hepta- or octasaccharide preferably linked to an immunogenic carrier, preferably a carrier protein and more preferably CRM₁₉₇. Consequently, the vaccines of the present invention contain only one synthetically synthesized compound of general formulae (**I**) preferably linked to an immunogenic carrier, preferably a carrier protein and more preferably CRM₁₉₇.

Preferred is a saccharide of general formula (**I**), wherein x represents 1 and a pharmaceutically acceptable salt thereof. Hence, a saccharide of general formula (**I-a**) 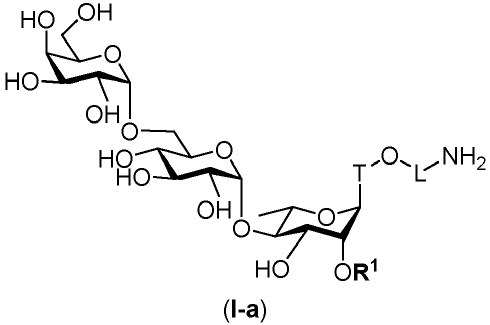 with L, T, and R¹ having the meaning defined herein, or a pharmaceutically acceptable salt thereof is particularly preferred.

Also preferred is a saccharide of general formula (**I**), wherein x represents 3, and a pharmaceutically acceptable salt thereof. Thus, a saccharide of general formula (**I-b**), 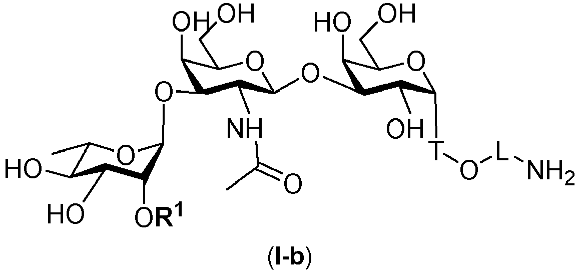 with L, T, and R¹ having the meaning defined herein, or a pharmaceutically acceptable salt thereof is also preferred.

Also preferred is a saccharide of general formula (**I**), wherein x represents 1 or 3, R¹ represents -H, and a pharmaceutically acceptable salt thereof. Thus, a saccharide of general formulae (**I-c**) and (**I-d**), 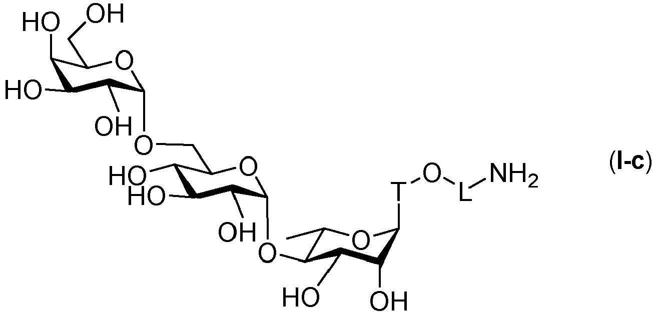 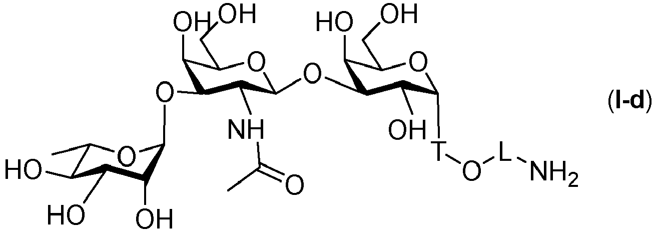 with L and T having the meaning defined herein, or a pharmaceutically acceptable salt thereof are also preferred.

Also preferred is a saccharide of general formula (**I**), wherein x represents 1 or 3, R¹ represents -H, T represents a bond or -Uₓ₊₃-, and a pharmaceutically acceptable salt thereof. Thus, a saccharide of general formula (**II-a**), (**II-b**), (**II-c**), or (**II-d**),

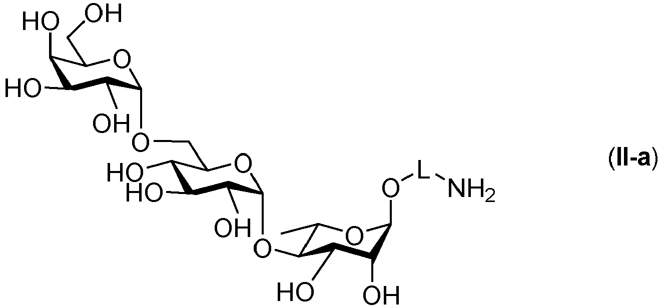

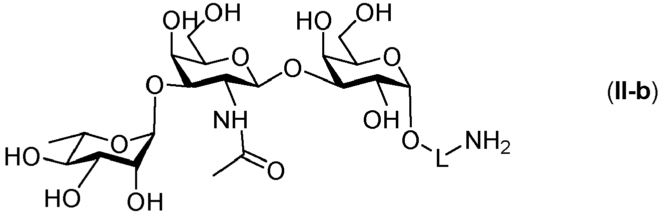

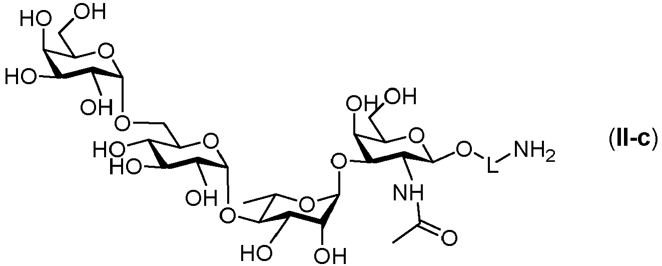

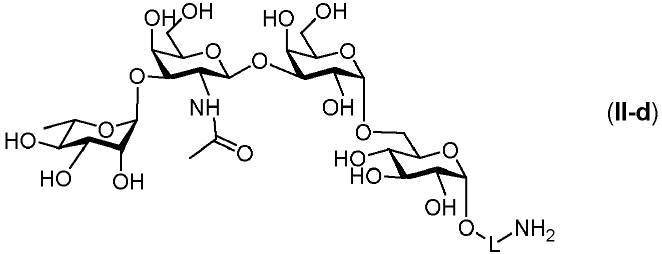

Preferably, T represents a bond, -Uₓ₊₃-, -Uₓ₊₃-Uₓ₊₂-, or -Uₓ₊₃-Uₓ₊₂-Uₓ₊₁-. Thus, a saccharide of general formula (**I**), (**I-a**) or (**I-b**), wherein T represents a bond, -Uₓ₊₃-, -Uₓ₊₃-Uₓ₊₂-, or -Uₓ₊₃-Uₓ₊₂-Uₓ₊₁- is preferred.

Preferably, T represents a bond or -Uₓ₊₃-, wherein -Uₓ₊₃- is either -U₄- or -U₆-. Thus, a saccharide of general formula (**I**), (**I-a**) or (**I-b**), wherein T represents a bond or -Uₓ₊₃- , wherein -Uₓ₊₃- is either -U₄- or -U₆- is preferred.

Also, preferred is a saccharide of general formula (**III**),

**H-Uₓ₊₂-Uₓ₊₁-Uₓ-[Uₓ₊₃-Uₓ₊₂-Uₓ₊₁-Uₓ]ₙ-O-L-NH₂** (**III**)

wherein Uₓ, Uₓ₊₁, Uₓ₊₂, Uₓ₊₃, x, L, and n have the meanings as defined herein.

Particularly preferred is a saccharide of general formula (**III**), wherein n represents 1 and R¹ represents -H.

Preferably the **linker-L-** represents **-L^{a}-, -L^{a}-L^{e}-, -L^{a}-L^{b}-L^{e}-,** or **-L^{a}-L^{d}-L^{e}-**
-L^{a}- represents -(CH₂)ₒ-, -(CHrCHrO)ₒ-C₂H₄-, or -(CH₂-CH₂-O)ₒ-CH₂;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
and o, q, p1 and p2 are independently of each other an integer selected from 1, 2, 3, 4, 5, and 6.

Therefore, a saccharide of general formula (**I**), (**I-a**), (**I-b**), (**I-c**), (**I-d**), (**II-a**), (**II-b**), (**II-c**), (**II-d**), or (**III**) wherein
-L- represents -L^{a}-, -L^{a}-L^{e}-, -L^{a}-L^{b}-L^{e}-, or -L^{a}-L^{d}-L^{e}- ;
-L^{a}- represents -(CH₂)ₒ-, -(CH₂CH₂O)ₒ-C₂H₄-, or -(CH₂-CH₂-O)ₒ-CH₂;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
-L^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
and o, q, p1 and p2 are independently of each other an integer selected from 1, 2, 3, 4, 5, and 6 is especially preferred.

Particularly preferred is a saccharide of general formula (**I**), (**I-a**), (**I-b**), (**I-c**), (**I-d**), (**II-a**), (**II-b**), (**II-c**), (**II-d**), or (**III**), wherein
-L- represents -L^{a}-, -L^{a}-L^{e}-, -L^{a}-L^{b}-L^{e}-, or -L^{a}-L^{d}-L^{e}- ;
-L^{a}- represents -(CH₂)ₒ-, -(CH₂-CH₂-O)ₒ-C₂H₄-, or -(CH₂-CH₂-O)ₒ-CH₂;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
-L^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
and o, q, p1 and p2 are independently of each other an integer selected from 1, 2, 3, 4, 5, and 6; and R¹ represents -H.

It is particularly preferred that -L- represents -(CH₂)ₒ- and o is an integer selected from 2, 3, 4, 5, 6, 7 and 8. Thus, a particularly preferred saccharide is a saccharide of general formula (**I**), (**I-a**), (**I-b**), (**I-c**), (**I-d**), (**II-a**), (**II-b**), (**II-c**), (**II-d**), or (**III**) wherein -L- represents -(CH₂)ₒ- and o is an integer selected from 2, 3, 4, 5, 6, 7 and 8.

Even more preferred is a saccharide of general formula (**I**), (**I-a**), (**I-b**), (**I-c**), (**I-d**), (**II-a**), (**II-b**), (**II-c**), (**II-d**), or (**III**) wherein -L- represents -(CH₂)ₒ- and o is an integer selected from 2, 3, 4, 5, 6, 7 and 8 and R¹ represents -H.

Preferably, the inventive saccharide is selected from: 5-amino pentyl *α*-D-galactopyranosyl-(1→6)-*α*-D-glucopyranosyl--(1→4)-*α*-L-rhamnopyranosyl-(1→3)-*β-*D-N-acetylgalactopyranoside; 6-amino hexyl *α*-D-galactopyranosyl-(1→6)-*α*-D-glucopyranosyl--(1→4)-*α*-L-rhamnopyranosyl-(1→3)-*β*-D-N-acetylgalactopyranoside-, 7-amino heptyl *α*-D-galactopyranosyl-(1→6)-*α*-D-glucopyranosyl--(1→4)-*α*-L-rhamnopyranosyl-(1→3)-*β*-D-N-acetylgalactopyranoside; 4-amino butyl *α*-D-galactopyranosyl-(1→6)-*α*-D-glucopyranosyl--(1→4)-*α*-L-rhamnopyranosyl-(1→3)-*β-*D-N-acetylgalactopyranoside; 3-amino propyl *α*-D-galactopyranosyl-(1→6)-*α*-D-glucopyranosyl--(1→4)-*α*-L-rhamnopyranosyl-(1→3)-*β*-D-N-acetylgalactopyranoside-, 5-amino pentyl *α*-D-galactopyranosyl-(1→6)-*α*-D-glucopyranosyl--(1→4)-*α*-L-rhamnopyranoside; 6-amino hexyl *α*-D-galactopyranosyl-(1→6)-*α*-D-glucopyranosyl-(1→4)-*α*-L-rhamnopyranoside; 7-amino heptyl *α*-D-galactopyranosyl-(1→6)-*α*-D-glucopyranosyl-(1→4)-*α*-L-rhamnopyranoside; 4-amino butyl *α*-D-galactopyranosyl-(1→6)-*α*-D-glucopyranosyl--(1→4)-*α*-L-rhamnopyranoside; 3-amino propyl *α*-D-galactopyranosyl-(1→6)-*α*-D-glucopyranosyl--(1→4)-*α*-L-rhamnopyranoside; 5-amino pentyl *α*-L-rhamnopyranosyl-(1→3)-*β*-D-N-acetylgalactopyranosyl-(1→3)-*α*-D-galactopyranosyl-(1→6)-*α*-D-glucopyranoside; 6-amino hexyl *α*-L-rhamnopyranosyl-(1→3)-*β*-D-N-acetylgalactopyranosyl-(1→3)-*α*-D-galactopyranosyl-(1→6)-*α*-D-glucopyranoside; 7-amino heptyl *α*-L-rhamnopyranosyl-(1→3)-*β*-D-N-acetylgalactopyranosyl-(1→3)-*α*-D-galactopyranosyl-(1→6)-*α*-D-glucopyranoside; 4-amino butyl *α*-L-rhamnopyranosyl-(1→3)-*β*-D-N-acetylgalactopyranosyl--(1→3)-*α*-D-galactopyranosyl-(1→6)-*α*-D-glucopyranoside; 3-amino propyl *α*-L-rhamnopyranosyl-(1→3)-β-D-N-acetylgalactopyranosyl--(1→3)-*α*-D-galactopyranosyl-(1→6)-*α*-D-glucopyranoside; 5-amino pentyl *α*-L-rhamnopyranosyl-(1→3)-β-D-N-acetylgalactopyranosyl-(1→3)-*α*-D-galactopyranoside; 6-amino hexyl *α*-L-rhamnopyranosyl-(1→3)-*β*-D-N-acetylgalactopyranosyl-(1→3)-*α*-D-galactopyranoside; 7-amino heptyl *α*-L-rhamnopyranosyl-(1→3)-*β*-D-N-acetylgalactopyranosyl-(1→3)-*α*-D-galactopyranoside; 4-amino butyl *α*-L-rhamnopyranosyl-(1→3)-*β*-D-N-acetylgalactopyranosyl--(1→3)-*α*-D-galactopyranoside; and 3-amino propyl *α*-L-rhamnopyranosyl-(1→3)-*β*-D-N-acetylgalactopyranosyl--(1→3)-*α*-D-galactopyranoside.

### Conjugate

Another aspect of the present invention is directed to a conjugate comprising a synthetic saccharide of general formula (**I**) covalently bound or covalently linked to an immunogenic carrier through the nitrogen atom of the -O-L-NH₂ group. In other words, another aspect of the present invention is directed to a saccharide of any of the general formulae (**I**), (**I-a**), (**I-b**), (**I-c**), (**I-d**), (**II-a**), (**II-b**), (**II-c**), (**II-d**), or (**III**) conjugated with an immunogenic carrier through the nitrogen atom of the -O-L-NH₂ group. The inventive conjugate comprising a synthetic saccharide of the general formula (**I**), (**I-a**), (**I-b**), (**I-c**), (**I-d**), (**II-a**), (**II-b**), (**II-c**), (**II-d**), or (**III**) covalently bound or covalently linked to an immunogenic carrier through the nitrogen atom of the -O-L-NH₂ group is also defined as a conjugate obtained by reacting a saccharide of any of the general formulae (**I**), (**I-a**), (**I-b**), (**I-c**), (**I-d**), (**II-a**), (**II-b**), (**II-c**), (**II-d**), or (**III**) with an immunogenic carrier. Said conjugate proved to be efficient as a vaccine for immunization against diseases associated with *Porphyromonas gingivalis* bacteria.

Saccharides are known by the person skilled in the art as generally TI-2 (T cell independent-2) antigens and poor immunogens. TI-2 antigens are antigens, which are recognized only by mature B cells through the cross linking of surface exposed immunoglobulin receptors. Without T cell help, no immunological memory is generated and neither isotype switching from IgM to other IgG subclasses, nor B cells affinity maturation occurs. Moreover, saccharides are known as poor immunogens in humans due to the structural homology to human glycolipids and glycoproteins. Due to their poor immunogenic properties, saccharides manifest poor ability to produce both antibody production by B cells, as well as the formation of memory cells, features which are essential for the production of potent vaccines.

Therefore, to produce a potent saccharide-based vaccine, a saccharide of general formula (**I**), (**I-a**), (**I-b**), (**I-c**), (**I-d**), (**II-a**), (**II-b**), (**II-c**), (**II-d**), or (**III**) is conjugated to an immunogenic carrier to provide a conjugate presenting increased immunogenicity in comparison with the saccharide.

Said conjugate consists of at least one synthetic saccharide of the general (**I**), (**I-a**), (**I-b**), (**I-c**), (**I-d**), (**II-a**), (**II-b**), (**II-c**), (**II-d**), or (**III**) and an immunogenic carrier to which the at least one saccharide of the general formula (**I**), (**I-a**), (**I-b**), (**I-c**), (**I-d**), (**II-a**), (**II-b**), (**II-c**), (**II-d**), or (**III**) is covalently bound.

Surprisingly, it was found that immunization with a conjugate according to the present invention results in the production of high titers of antibodies specific to the carbohydrate part of the saccharide according to the present invention. Said antibodies are cross-reacting with the natural *Porphyromonas gingivalis* lipopolysaccharide, thus conferring protection against *Porphyromonas gingivalis* bacteria.

In this context the term "immunogenic carrier" is defined as a structure, which is conjugated to the saccharide to form a conjugate that presents an increased immunogenicity in comparison with the saccharide *per se*. Thus, the conjugation of a saccharides of the general formula (**I**), (**I-a**), (**I-b**), (**I-c**), (**I-d**), (**II-a**), (**II-b**), (**II-c**), (**II-d**), or (**III**) to the immunogenic carrier has as effect the stimulation of the immune response against the saccharide of the general formula (**I**), (**I-a**), (**I-b**), (**I-c**), (**I-d**), (**II-a**), (**II-b**), (**II-c**), (**II-d**), or (**III**) without inducing an immune response against the said immunogenic carrier.

Preferred immunogenic carriers are carrier proteins, *P. gingivalis* outer membrane proteins, or glycosphingolipids with immunomodulatory properties. For the person skilled in the art, a carrier protein is a protein selected from the group comprising or consisting of: a diphtheria toxoid, a mutated diphtheria toxoid, a modified diphtheria toxoid, a mutated and modified diphtheria toxoid, a tetanus toxoid, a modified tetanus toxoid, a mutated tetanus toxoid, outer membrane protein (OMP), bovine serum albumin (BSA), keyhole limpet hemocyanine (KLH), cholera toxoid (CT) and protein D (a non-typeable *Haemophilus influenzae* protein).

*"P. gingivalis* membrane proteins" are understood herein as proteins that are part of or are present in the outer membrane of *P. gingivalis* (Mol Oral Microbiol. 2021, 36(1):25-36) which can be recognized as virulence factors. Examples are but not restricted to fimbriae including long fimbriae, short fimbriae, cell surface proteins (e.g. 40-kDa, 53-kDa and 67-kDa), hemagglutinin, porins such as RagA, RagB, PG1626, PG1414, PG1028, PG0694, and PG0695, and protease enzymes (gingipains) such as arginine-specific gingipains (RgpA, RgpB) and lysine-specific gingipain (Kgp).

The term "toxoid" as used herein refers to a bacterial toxin (usually an exotoxin), whose toxicity has been inactivated or suppressed either by chemical (formalin) or heat treatment, while other properties, typically immunogenicity, are maintained. A mutated toxoid as used herein is a recombinant bacterial toxin, which has been amended to be less toxic or even non-toxic by amending the wild-type amino acid sequence. Such a mutation could be a substitution of one or more amino acids. Such a mutated toxoid presents on its surface a functionality that can react with the functional group Y of the interconnecting molecule to provide a modified toxoid. Said functionality is known to the person skilled in the art and includes but is not restricted to the primary amino functionality of a lysine residue that can react with activated esters, an isocyanate group or an aldehyde in presence of a reducing agent, to the carboxylate functionality of a glutamate or aspartate residue that can be activated by carbodiimides or to the thiol functionality of a cysteine residue.

Activated esters include, but are not restricted to N-(γ-maleimidobutyryloxy) sulfosuccinimide ester (sulfo-GMBS), succinimidyl (4-iodoacetyl) aminobenzoate (sulfo-SIAB), succinimidyl-3-(bromoacetamido)propionate (SBAP), disuccinimidyl glutarate (DSG), disuccinimidyl adipate (DSA), 2-pyridyldithiol-tetraoxatetradecane-N-hydroxysuccinimide (PEG-4-SPDP), bis-(4-nitrophenyl) adipate and bis-(4-nitrophenyl) succinate (see **Figure 2****).** Preferred activated esters are disuccinimidyl adipate (DSA), disuccinimidyl glutarate (DSG), bis-(4-nitrophenyl) adipate and bis-(4-nitrophenyl) succinate.

Preferred is a conjugate of general formula **(IV)**

**[H-Uₓ₊₂-Uₓ₊₁-Uₓ-T-O-L-NH-W]ₘ-IM** **(IV)**

wherein m is comprised between about 2 and about 18;
-W- is selected from: 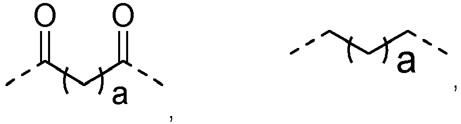 and 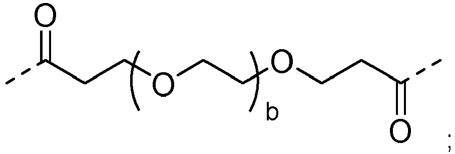
a represents an integer from 1 to 10; b represents an integer from 1 to 4;
**IM** represents an immunogenic carrier, and
Uₓ, Uₓ₊₁, Uₓ₊₂, Uₓ₊₃, x, n and L have the meanings defined herein.

As well known to the skilled person, "m" in structure **IV** corresponds to the average load of saccharide units per unit of immunogenic carrier as determined by MALDI-TOF MS method using the molecular weight of the immunogenic carrier as reference. By varying the reaction conditions for the coupling of the saccharide according to the present invention to the immunogenic carrier any conjugate of structure **IV** with m being comprised between about 2 and about 18 can be obtained.

Preferably, the linker -L- is selected from: -L^{a}-, -L^{a}-L^{e}-, -L^{a}-L^{b}-L^{e}-, and -L^{a}-L^{d}-L^{e}-;
-L^{a}- represents -(CH₂)ₒ-, -(CH₂-CH₂-O)ₒ-C₂H₄-, or -(CH₂-CH₂-O)ₒ-CH₂ ;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
**-L^{e-}** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
and o, q, p1 and p2 are independently of each other an integer selected from 1, 2, 3, 4, 5, and 6.

It is also preferred that -W- represents 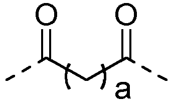 and a is an integer selected from 2, 3, 4, 5 and 6 is especially preferred.

Hence, a conjugate of general formula (IV), wherein
the linker -L- is selected from: -L^{a}-, -L^{a}-L^{e}-, -L^{a}-L^{b}-L^{e}-, and -L^{a}-L^{d}-L^{e}-;
-L^{a}- represents -(CH₂)ₒ-, -(CH₂-CH₂-O)ₒ-C₂H₄-, or -(CH₂-CH₂-O)ₒ-CH₂ ;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
o, q, p1 and p2 are independently of each other an integer selected from 1, 2, 3, 4, 5, and 6;
-W- represents 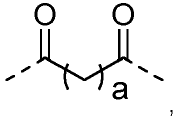 and a is an integer selected from 2, 3, 4, 5 and 6 is especially preferred.

Preferred is also conjugate of general formula (IVa)

**[H-Uₓ₊₂-Uₓ₊₁-Uₓ-T-O-L-N H-W]ₘ-CP** **(IVa)**

wherein m is comprised between about 2 and about 18;
-W- is selected from: 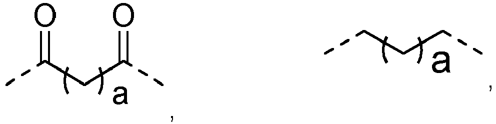 and 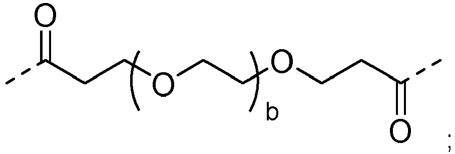
a represents an integer from 1 to 10;
b represents an integer from 1 to 4;
**CP** represents a carrier protein, and
Uₓ, Uₓ₊₁, Uₓ₊₂, Uₓ₊₃, x, n and L have the meanings defined herein.

As well known to the skilled person, "m" in structure **IVa** corresponds to the average load of saccharide units per unit of carrier protein as determined by MALDI-TOF MS method using the molecular weight of carrier protein as reference. By varying the reaction conditions for the coupling of the saccharide according to the present invention to the carrier protein any conjugate of structure **IVa** with m being comprised between about 2 and about 18 can be obtained.

Preferably, the linker -L- is selected from: -L^{a}-, -L^{a}-L^{e}-, -L^{a}-L^{b}-L^{e}-, and -L^{a}-L^{d}-L^{e}-;
-L^{a}- represents -(CH₂)ₒ-, -(CH₂-CH₂-O)ₒ-C₂H₄-, or -(CH₂-CH₂-O)ₒ-CH₂ ;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
**-L^{e-}** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
and o, q, p1 and p2 are independently of each other an integer selected from 1, 2, 3, 4, 5, and 6.

It is also preferred that -W- represents 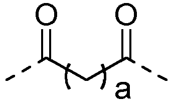 and a is an integer selected from 2, 3, 4, 5 and 6 is especially preferred.

Hence, a conjugate of general formula **(IV),** wherein
the linker -L- is selected from: -L^{a}-, -L^{a}-L^{e}-, -L^{a}-L^{b}-L^{e}-, and -L^{a}-L^{d}-L^{e}-;
-L^{a}- represents -(CH₂)ₒ-, -(CH₂-CH₂-O)ₒ-C₂H₄-, or -(CH₂-CH₂-O)ₒ-CH₂ ;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
**-L^{e-}** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
o, q, p1 and p2 are independently of each other an integer selected from 1, 2, 3, 4, 5, and 6;
-W- represents 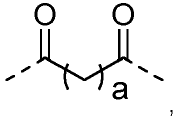 and a is an integer selected from 2, 3, 4, 5 and 6 is especially preferred.

The cysteine residue on the carrier protein can be converted to the corresponding dehydroalanine that can be further reacted with a suitable interconnecting molecule to provide modified carrier protein having on their surface the functional group X of the interconnecting molecule.

It is especially preferred that the inventive saccharides described herein are conjugated to the non-toxic mutated diphtheria toxin CRM₁₉₇ presenting as a functionality a primary amine functionality of a lysine residue.

CRM₁₉₇ like wild-type diphtheria toxin is a single polypeptide chain of 535 amino acids (58 kD) consisting of two subunits linked by disulfide bridges having a single amino acid substitution of glutamic acid for glycine. It is utilized as a carrier protein in a number of approved conjugate vaccines for diseases such as Prevnar^{®}.

Thus, in a preferred embodiment of the present invention the carrier protein presents on its surface primary amino functionalities of lysine residues that are able to react with the functional group Y of the interconnecting molecule to provide modified carrier protein having on their surface said functional group X of the interconnecting molecule, which is able to react with the terminal amino group of the linker functionalizing the inventive saccharides.

Said functional group X of the interconnecting molecules is selected from the group comprising or consisting of maleimide; α-iodoacetyl; α-bromoacetyl; and N-hydroxysuccinimide ester (NHS), aldehyde, imidoester, carboxylic acid, alkyl sulfonate, sulfonyl chloride, epoxide, anhydride, carbonate (see **Figure 3****).**

Even more preferred is a conjugate of general formula **(V),**

**[H-Uₓ₊₂-Uₓ₊₁-Uₓ-T-O-L-NH-W]ₘ-CRM₁₉₇** **(V)**

wherein m is comprised between about 2 and about 18;
-W- is selected from: 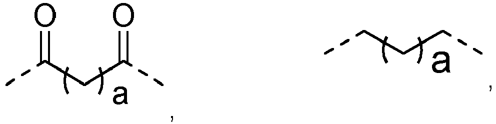 and 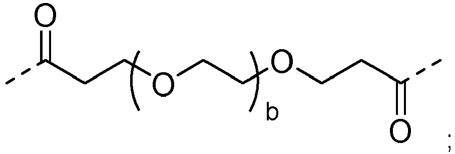
a represents an integer from 1 to 10;
b represents an integer from 1 to 4, and
Uₓ, Uₓ₊₁, Uₓ₊₂, Uₓ₊₃, x, n and L have the meanings defined herein.

Also preferred is a conjugate of general formula **(V),** wherein x represents 1 or 3. Thus, a conjugate of general formula **(V-a),** or **(V-b),** 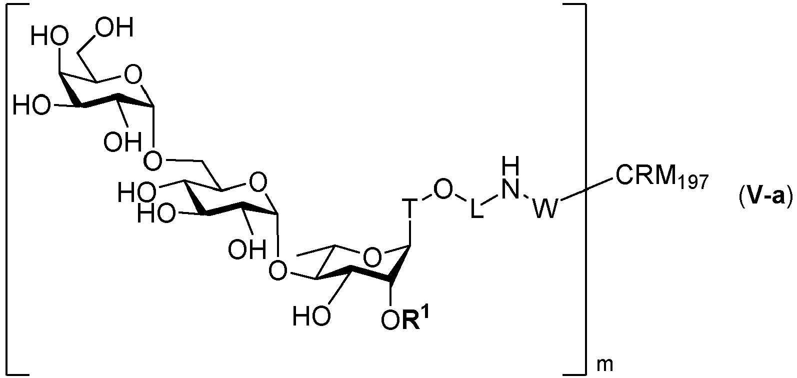 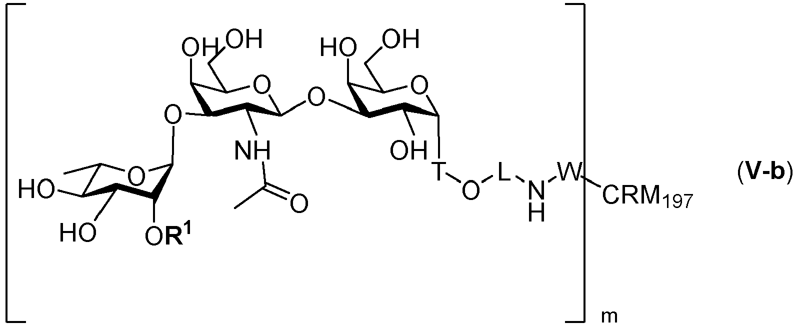
wherein m is comprised between about 2 and about 18;
-W- is selected from: 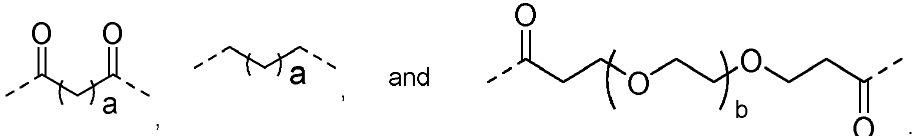
a represents an integer from 1 to 10;
b represents an integer from 1 to 4;
T, L, and R¹ have the meaning defined herein.

Also preferred is a conjugate of general formula **(V),** wherein **T** represents a bond or -Uₓ₊₃-, wherein -Uₓ₊₃- is either -U₄- or -U₆-. Thus, a conjugate of general formula (**VI-a**), (**VI-b**), (**VI-c**), or (**VI-d**), 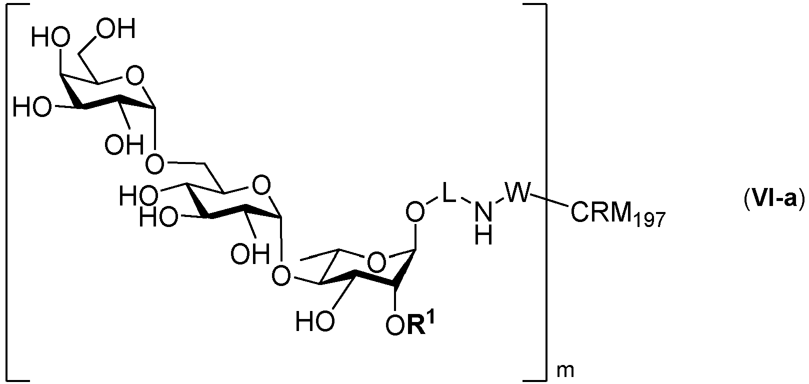 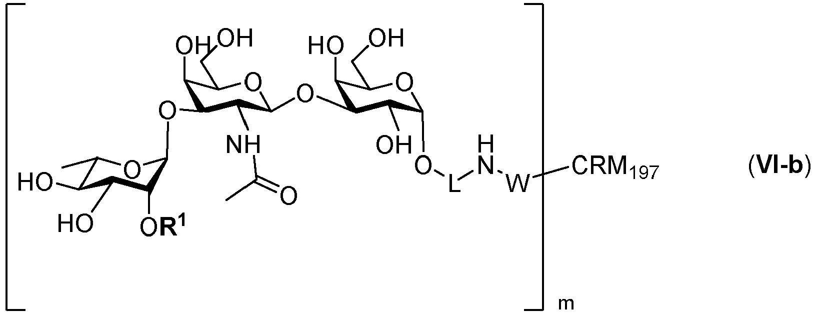 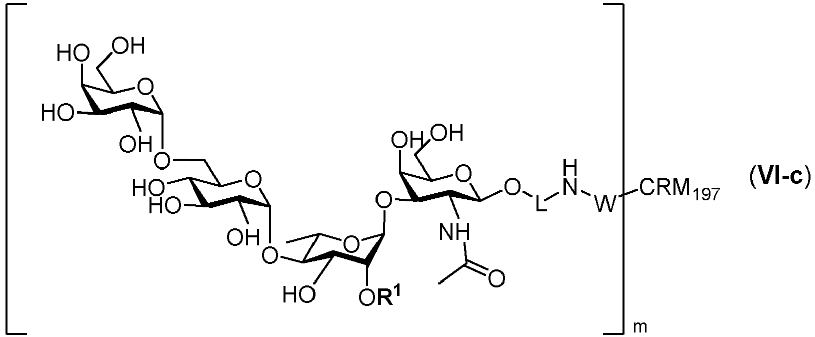 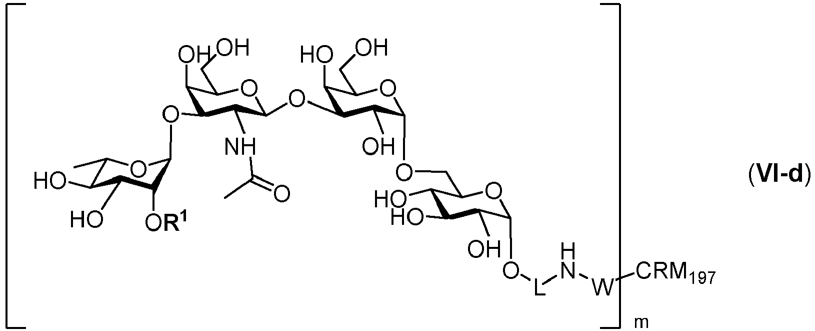
wherein m is comprised between about 2 and about 18;
-W- is selected from: 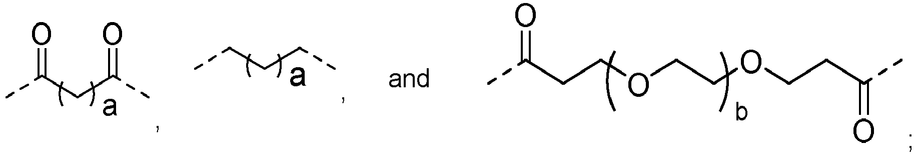
a represents an integer from 1 to 10;
b represents an integer from 1 to 4;
T, L, and R¹ have the meaning defined herein, is also preferred.

In another embodiment, the immunogenic carrier is a *P*. *gingivalis* outer membrane protein. The conjugation of *P. gingivalis* outer membrane protein with a saccharide of the present invention may enhance the immunogenicity of the saccharide as both immunogenic components address the same pathogen.

Thus, preferred is also conjugate of general formula (**IV**), wherein IM represents a *P. gingivalis* outer membrane protein, and m, -W-, Uₓ, Uₓ₊₁, Uₓ₊₂, Uₓ₊₃, x, n and L have the meanings defined herein.

Particularly preferred is, when the *P. gingivalis* outer membrane protein is selected from long fimbriae, RagA, RagB, RgpA, RgpB, and Kgp.

Also preferred is a conjugate of general formula (**VII-a**) or (**VII-b**), wherein PGOMP represents a *P*. *gingivalis* outer membrane protein as defined herein 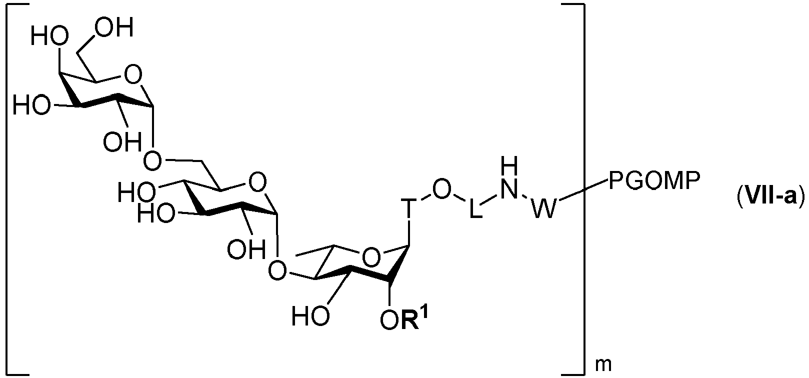 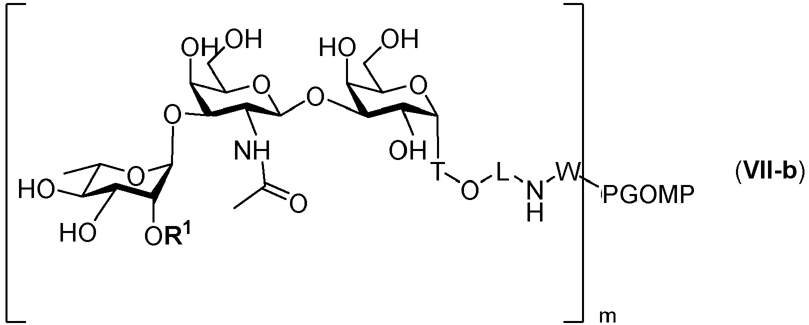
wherein m is comprised between about 2 and about 18;
-W- is selected from: 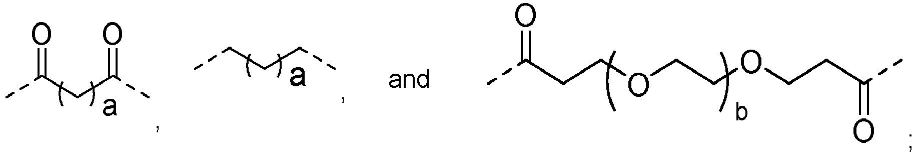
a represents an integer from 1 to 10;
b represents an integer from 1 to 4;
T, L, and R¹ have the meaning defined herein.

Also preferred is a conjugate of general of general formula (**VIII-a**), (**VIII-b**), (**VIII-c**), or (**VIII-d**), wherein PGOMP represents a *P. gingivalis* outer membrane protein as defined herein 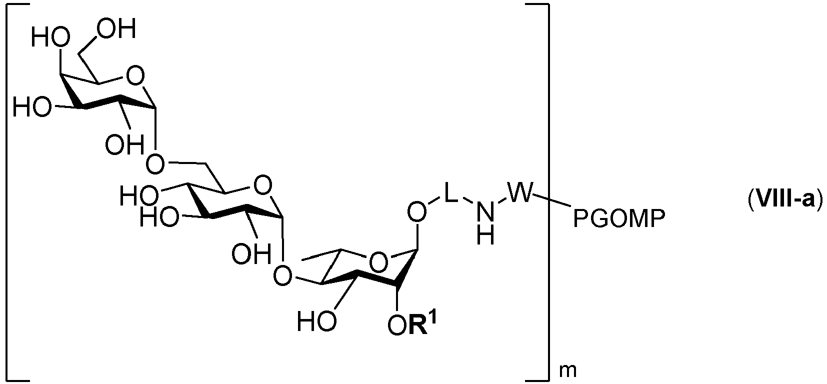 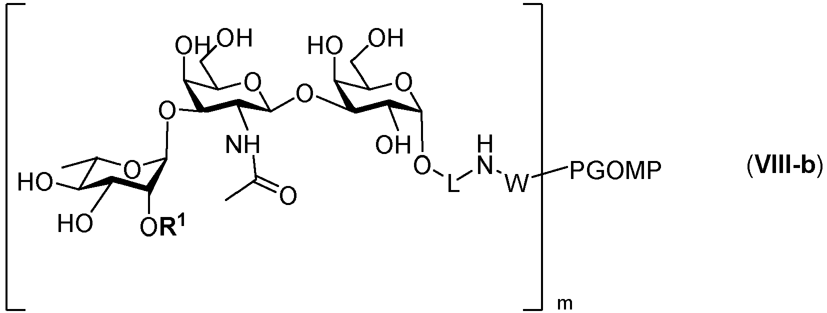 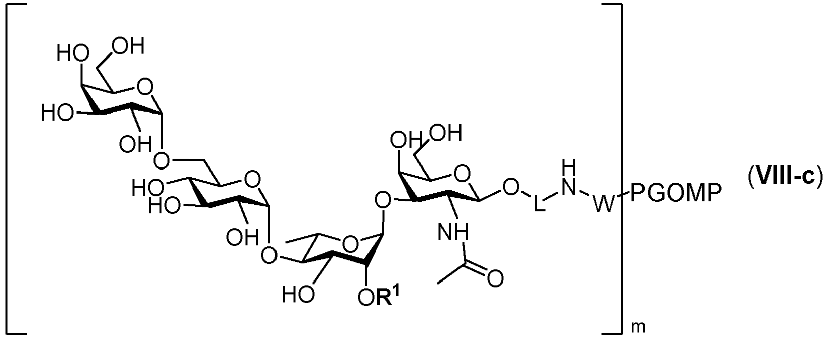 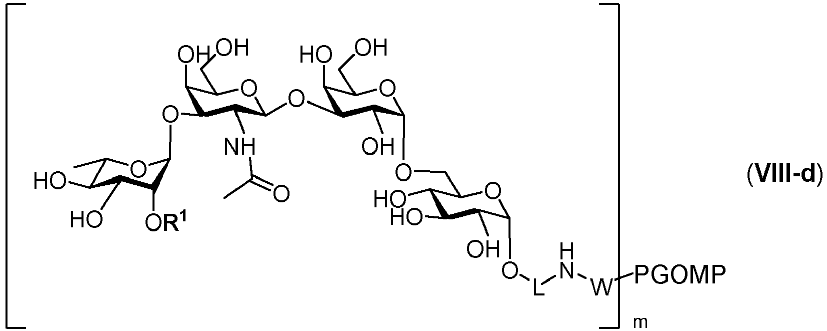
wherein m is comprised between about 2 and about 18;
-W- is selected from: 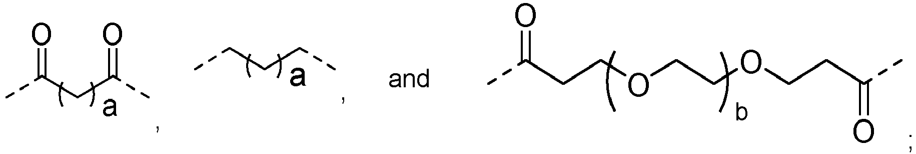
a represents an integer from 1 to 10;
b represents an integer from 1 to 4;
T, L, and R¹ have the meaning defined herein, is also preferred.

Preferably, in general formulae (**IV**), (**IVa**), (**V**), (**V-a**), (**V-b**) (**VI-a**), (**VI-b**), (**VI-c**), **(VI-d), (VII-a), (VII-b)** (**VIII-a**), (**VIII-b**), (**VIII-c**), and (**VI-d**) the linker -L- is selected from: -L^{a}-, -L^{a}-L^{e}-, -L^{a}-L^{b}-L^{e}-, and -L^{a}-L^{d}-L^{e}-;
-L^{a}- represents -(CH₂)ₒ-, -(CH₂-CH₂-O)ₒ-C₂H₄-, or -(CH₂-CH₂-O)ₒ-CH₂;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂- ;
-L^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ;
o, q, p1 and p2 are independently of each other an integer selected from 1, 2, 3, 4, 5, and 6;
-W- represents 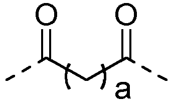 and a is an integer selected from 2, 3, 4, 5 and 6.

Especially preferred is a conjugate of general formula **(IV), (IVa), (V), (V-a), (V-b), (VI-a), (VI-b), (VI-c), (VI-d), (VII-a),** (**VII-b**) (**VIII-a**), (**VIII-b**), (**VIII-c**), or **(VI-d),** wherein the linker -L- represents -(CH₂)ₒ- ,
o is an integer selected from 2, 3, 4, 5, 6, 7 and 8;
-W- represents 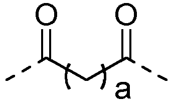 and a is an integer selected from 2, 3, 4, 5 and 6.

It is also preferred that R¹- represents -H in general formulae **(IV), (V), (IVa), (V-a), (V-b) (VI-a), (VI-b), (VI-c), (VI-d), (VII-a),** (**VII-b**) (**VIII-a**), (**VIII-b**), (**VIII-c**), and **(VI-d).** Thus, particularly preferred is a conjugate of general formula **(IV), (V), (V-a), (V-b) (VI-a), (VI-b), (VI-c), (VI-d), (VII-a),** (**VII-b**) (**VIII-a**), (**VIII-b**), (**VIII-c**), or **(VI-d),** wherein
R¹- represents -H, the linker-L- represents -(CH₂)ₒ-,
o is an integer selected from 2, 3, 4, 5, 6, 7 and 8;
-W- represents 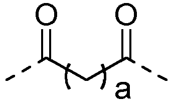 and a is an integer selected from 2, 3, 4, 5 and 6.

Preferably m is comprised between about 2 and about 18, more preferably between about 5 and about 15, even more preferably between about 8 and about 12.

In another embodiment, said immunogenic carrier is preferably a glycosphingolipid with immunomodulatory properties, and more preferably (2*S*,3*S*,4*R*)-1-(*α*-L-galactopyranosyl)-2-hexacosanoylaminooctadecane-3,4-diol. The term glycosphingolipid with immunomodulatory properties, as used herein, refers to a suitable glycosphingolipid capable of stimulating the immune system's response to a target antigen, but which does not in itself confer immunity as defined above.

Glycosphingolipids as used herein are compounds containing a carbohydrate moiety α-linked to a sphingolipid. Preferably, the carbohydrate moiety is a hexopyranose and most preferably is α-D-galactopyranose. For the person skilled in the art, sphingolipids are a class of lipids containing a C18 amino alcohol connected *via* an amide bond to a fatty acid. The C18 amino alcohol is preferably mono-, di- or polysubstituted with hydroxyl groups. Especially preferred, the C18 amino alcohol is phytosphingosine. The fatty acid is preferably a monocarboxylic acid having a saturated alkyl chain of a number of carbons ranging from 16 to 28 and more preferably from 18 to 26. Glycosphingolipids with immunomodulatory properties include, but they are not restricted to (2*S*,3*S*,4*R*)-1-(α-D-galactopyranosyl)-2-hexacosanoylaminooctadecane-3,4-diol, which can stimulate natural killer (NK) activity and cytokine production by natural killer T (NKT) cells and exhibits potent antitumor activity *in vivo* (Proc. Natl Acad. Sci. USA, 1998, 95, 5690).

The conjugates of the inventive saccharides with a glycosphingolipid with immunomodulatory properties have the advantage of being heat stable. To be suitable for conjugation, on the glycosphingolipid with immunomodulatory properties a functionality is introduced. Said functionality is prone to react directly with the terminal amino group of the linker of the inventive to provide conjugates of the saccharides or with the functional group Y of the interconnecting molecule to provide the modified glycosphingolipid with immunomodulatory properties.

Preferably, said functionality is introduced at the C6 of the carbohydrate moiety of the glycosphingolipid with immunomodulatory properties. Thus, the glycosphingolipid with immunomodulatory properties is functionalized with a functionality, which is prone of reacting with the terminal amino group of the saccharides or with the functional group Y of the interconnecting molecule. A functionality prone to react with an amino group includes, but it is not restricted to activated ester, isocyanate group, aldehyde, epoxide, imidoester, carboxylic acid, alkyl sulfonate and sulfonyl chloride. A functionality prone to react with the functional group Y of the interconnecting molecule so that to provide the modified glycosphingolipid with immunomodulatory properties presenting the functional group X of the interconnecting molecule includes, but it is not restricted to amine, alcohol, thiol, activated ester, isocyanate group, aldehyde, epoxide, vinyl, imidoester, carboxylic acid, alkyl sulfonate, sulfonyl chloride, vinyl group, alkynyl group and azido group.

Preferably, the functionality introduced at the C-6 position of the carbohydrate moiety of the glycosphingolipid with immunomodulatory properties is selected from the group comprising or containing an amine, a thiol, an alcohol, a carboxylic acid, a vinyl, maleimide, α-iodoacetyl, α-bromoacetyl, N-hydroxysuccinimide ester (NHS), 2-pyridyldithiols.

Said functional group X of the interconnecting molecules is selected from the group comprising or consisting of maleimide, α-iodoacetyl, α-bromoacetyl, *N-*hydroxysuccinimide ester (NHS), aldehyde, carboxylic acid, epoxyde, alkyl sulfonate, sulfonyl chloride, anhydride, carbonate.

Preferably, di(*N*-succinimidyl) adipate or bis(4-nitrophenyl) adipate is first reacted with a synthetic saccharide having a primary amino group. Activated saccharide is subsequently condensed with a glycosphingolipid, which is modified at C-6 position by an interconnecting molecule having a terminal amino functionality in order to afford the conjugate.

As used herein, the term "interconnecting molecule" refers to a bifunctional molecule containing functional group X and functional group Y, wherein functional group X is capable of reacting with the terminal amino group on the linker -L- and the functional group Y is capable of reacting with a functionality present on the immunogenic carrier.

As used herein, the term "interconnecting molecule" refers to a bifunctional molecule containing functional group X and functional group Y, wherein functional group X is capable of reacting with the terminal amino group on the linker -L- and the functional group Y is capable of reacting with a functionality present on the immunogenic carrier.

Thus, in another preferred embodiment the conjugate has the structure of general formula **(IX)**

**H-Uₓ₊₂-Uₓ₊₁-Uₓ-T-O-L-NH-W'-GSL** **(IX)**

-W'- represents -L²-NH-L³- ;
L³ represents -L^{3'}-L^{3"}-L^{3‴}- - or -L^{3'}-L^{3‴}- or -L^{3'}-; and
L^{3'}, L^{3"}, and L^{3‴} are independently of each other selected from: -CH₂-,-C₂H₄-, -C₃H₆-, -C₄H₈-, -C₅H₁₀-, -C₆H₁₂-, -C₇H₁₄-, -C₈H₁₆-, -C₉H₁₈-,-C₁₀H₂₀-, -CR²³R²⁴-, -CR²⁵R²⁶-, -CR²⁷R²⁸-, -CR²⁹R³⁰-, -CR³¹R³²-,-CR³³R³⁴-, -(CH₂-CH₂-O)ₒ-; -*o*-C₆H₄-, -*m*-C₆H₄-, -*p*-C₆H₄-, -CH₂-S-CH₂--CH₂-O-CH₂-, -S-, -O-;
L² is selected from: -C(O)-, -E-, -C(O)-NH-NH-C(O)- 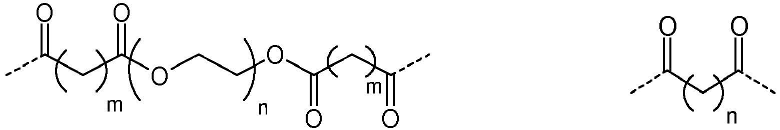 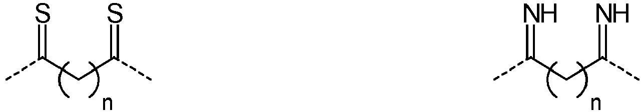 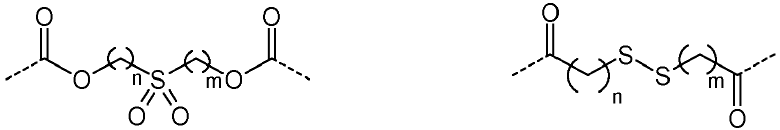 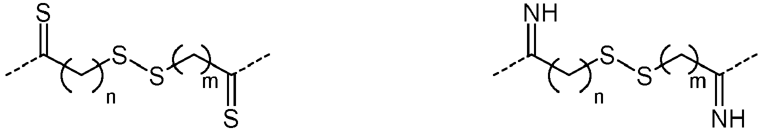 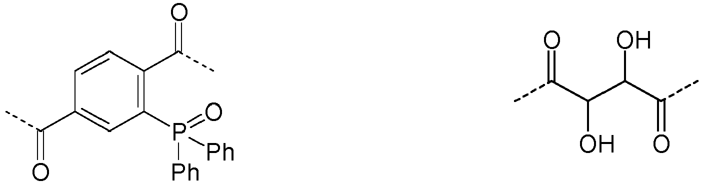 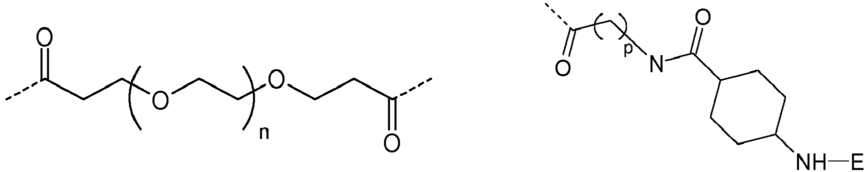 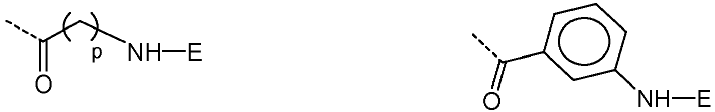 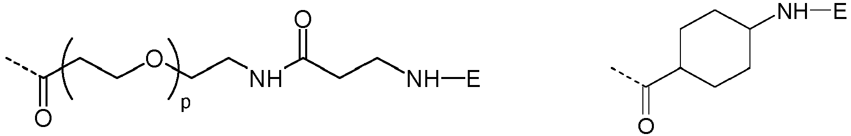 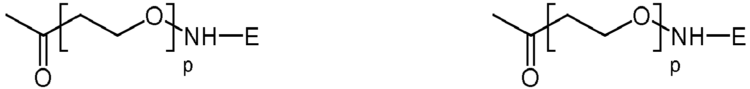
E is selected from 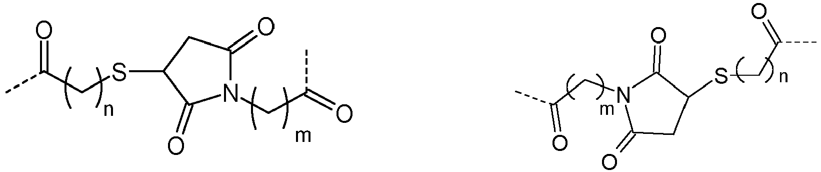 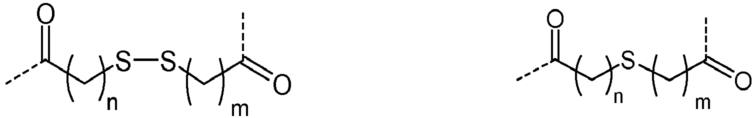 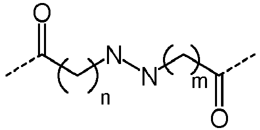
m, n, p represent independently of each other an integer from 0 to 30;
**GSL** represents a glycosphingolipid,
R²³ - R³⁴ are independently of each other selected from: -H, -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂H₅)₂; and
Uₓ, Uₓ₊₁, Uₓ₊₂, Uₓ₊₃, x, and L have the meanings defined herein.

In a preferred embodiment of the conjugate of general formula **(IX),** L² is selected from: 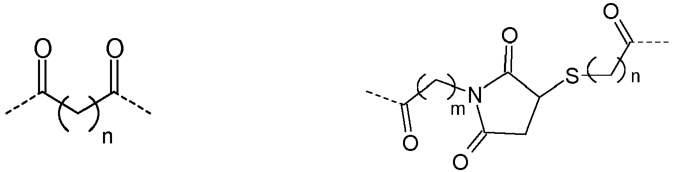 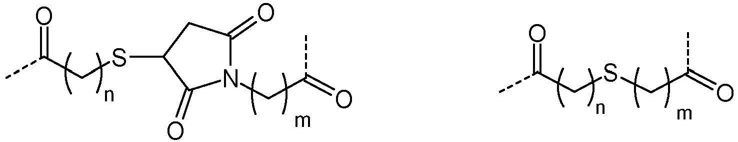 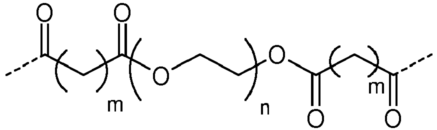 and m and n represent independently of each other an integer from 0 to 30.

In a further preferred embodiment the glycosphingolipid GSL has the following structure: 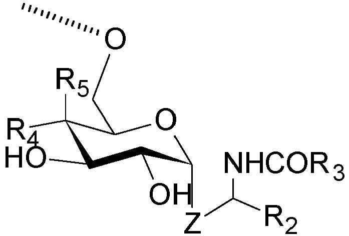
wherein R² is selected from 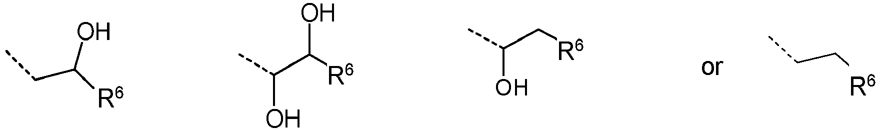
R³ is -(X¹)ₚ₁-(X²)ₚ₂-(X³)ₚ₃-X⁴;
R⁴and R⁵ are selected from -H and -OH and cannot be simultaneously the same;
R⁶ is -(Y¹)ₘ₁-(Y²)ₘ₂-(Y³)ₘ₃-Y⁴;
X¹, X², X³, Y¹, Y², and Y³ are independently of each other selected from:
   -CH₂-, -CH(OH)-, -CH(CH₃)-, -CH(C₂H₅)-, -CH(C₃H₇)-, -CH(C₄H₉)-, 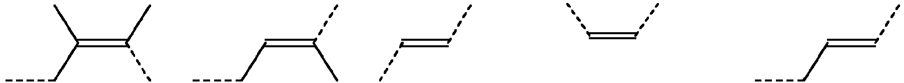 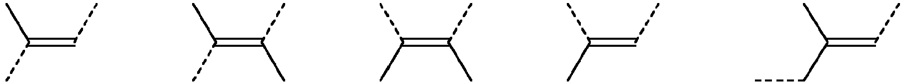 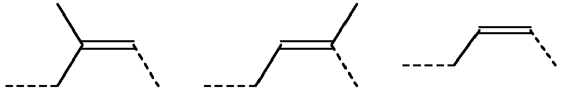
   X⁴ represents: -H, -iPr, -tBu, or -sBu, 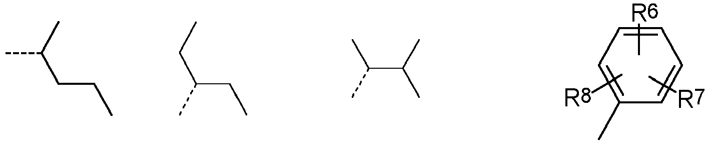 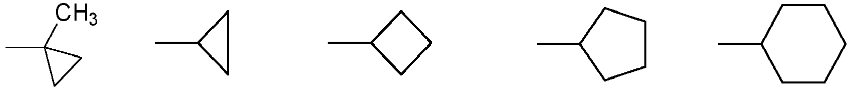 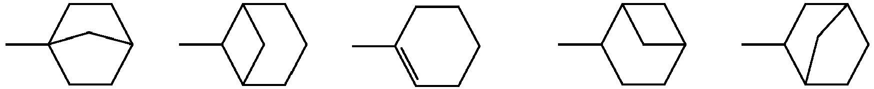 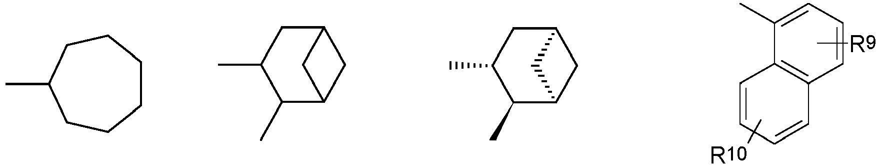 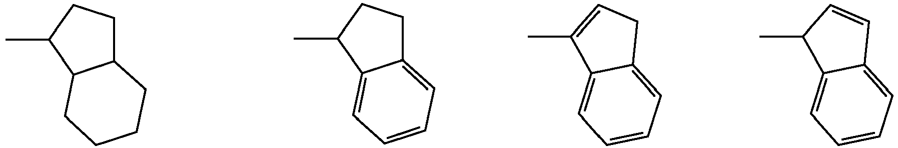 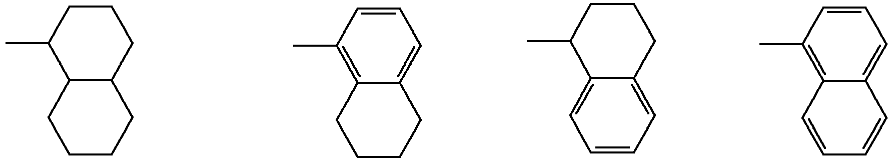
   Y⁴ is selected from: -H, -iPr, -tBu, -Ph, sBu, 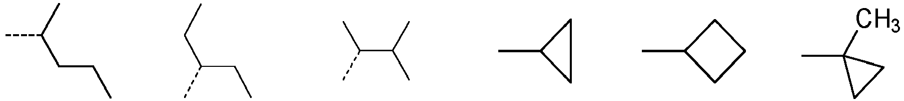 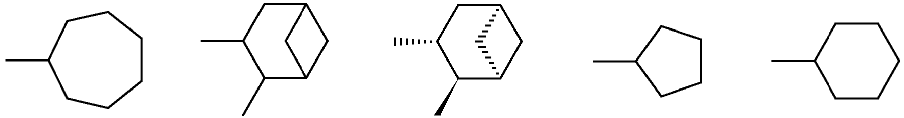 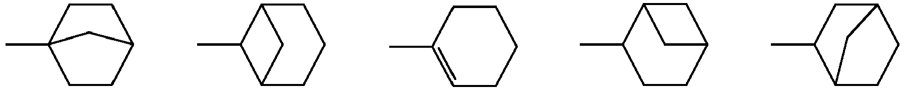 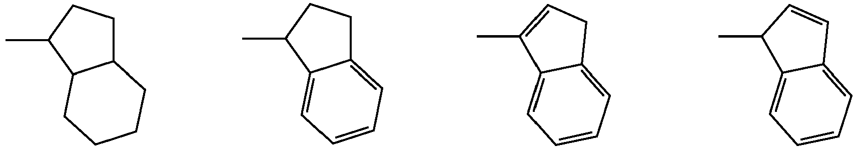 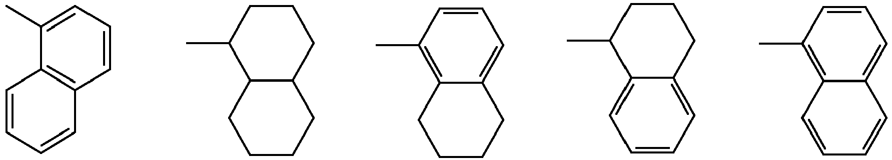
p1, p2, p3, m1, m2 and m3 represent independently of each other an integer from 0 to 30;

In a particularly preferred embodiment, the glycosphingolipid is (2*S*,3*S*,4*R*)-1-(α-L-galactopyranosyl)-2-hexacosanoylaminooctadecane-3,4-diol (α-galactosylceramide).

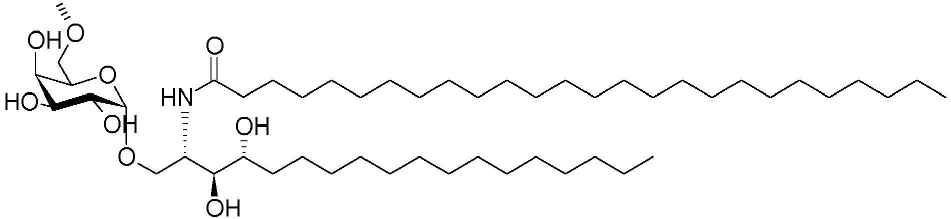

Also preferred is a conjugate of general of general formula (X-a), (X-b), (X-c), or (X-d), wherein **GSL** represents a glycosphingolipid as defined herein 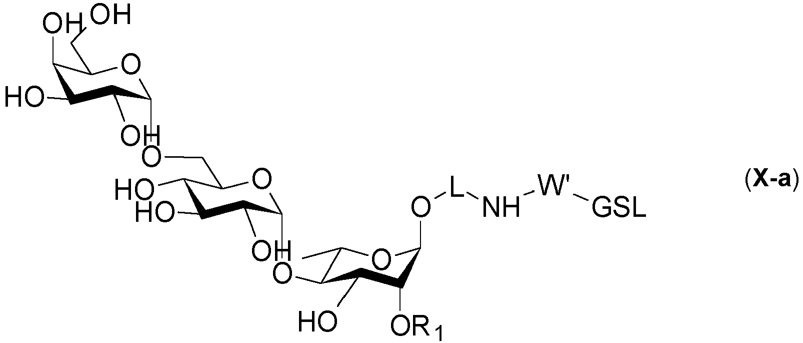 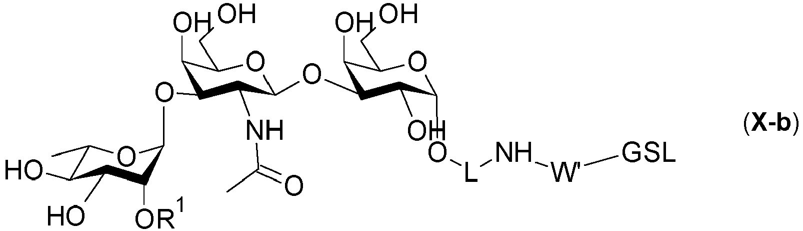 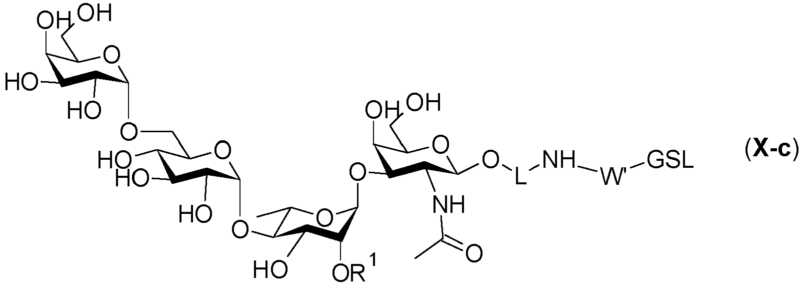 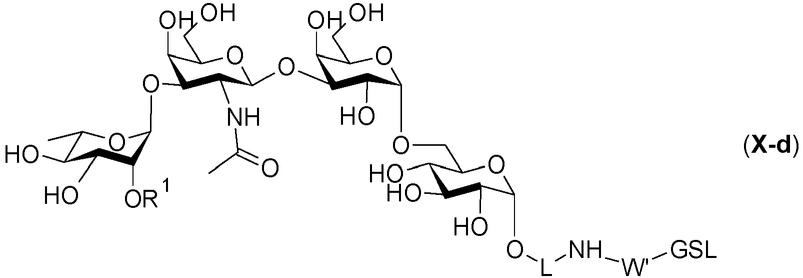 wherein R¹, W, L and **GSL** have the meanings as defined herein.

It was found that the saccharide of general formula (**I**), (**I-a**), (**I-b**), (**I-c**), (**I-d**), (**II-a**), (**II-b**), (**II-c**), (**II-d**), or (**III**) covalently linked or covalently bound to an immunogenic carrier through the nitrogen atom of the -O-L-NH₂ group or in other words the conjugate obtained by reacting a saccharide of general formula formula (**I**), (**I-a**), (**I-b),** (**I-c**), (**I-d**), (**II-a**), (**II-b**), (**II-c**), (**II-d**), or (**III**) with an immunogenic carrier, and especially a conjugate of general formula **(IV), (IVa), (V), (V-a), (V-b), (VI-a), (VI-b), (VI-c), (VI-d), (VII-a),** (**VII-b**) (**VIII-a**), (**VIII-b**), (**VIII-c**), **(VIII-d), (IX), (X-a), (X-b), (X-c),** or **(X-d),** elicits a protective immune response in a human and/or animal host, and therefore is useful in the prevention and/or treatment of a disease caused by *P. gingivalis* . Such disease includes periodontal disease, Alzheimer's disease, atherosclerosis, or rheumatoid arthritis.

### Pharmaceutical composition

A further aspect of the present invention relates to a pharmaceutical composition or a vaccine containing at least one synthetic saccharide and/or a pharmaceutically acceptable salt thereof according to the present invention and/or a conjugate comprising a saccharide according to the present invention covalently linked to an immunogenic carrier, preferably to CRM₁₉₇ carrier protein, through the nitrogen atom of the -O-L-NH₂ group together with at least one pharmaceutically acceptable adjuvant and/or excipient.

The term "adjuvant" as used herein refers to an immunological adjuvant i.e. a material used in a pharmaceutical composition or vaccine that modifies or augments the effects of said pharmaceutical composition or said vaccine by enhancing the immune response to a given antigen contained in the pharmaceutical composition or vaccine without being antigenically related to it. For the persons skilled in the art, classically recognized examples of immunological adjuvants include, but are not restricted to oil emulsions (e.g. Freund's adjuvant), saponins, aluminium or calcium salts (e.g. alum), non-ionic block polymer surfactants, and many others.

Pharmaceutical compositions or vaccines are preferably in aqueous form, particularly at the point of administration, but they can also be presented in non-aqueous liquid forms or in dried forms e.g. as gelatin capsules, or as lyophilisates, etc..

Pharmaceutical compositions or vaccines may include one or more preservatives, such as thiomersal or 2-phenoxyethanol. Mercury-free compositions are preferred, and preservative-free vaccines can be prepared.

Pharmaceutical compositions or vaccines may include a physiological salt, such as a sodium salt e.g. to control tonicity. Sodium chloride (NaCl) is typical and may be present at between 1 and 20 mg/ml. Other salts that may be present include potassium chloride, potassium dihydrogen phosphate, disodium phosphate dehydrate, magnesium chloride, calcium chloride, etc.

Pharmaceutical compositions or vaccines can have an osmolality of between 200 mOsm/kg and 400 mOsm/kg.

Pharmaceutical compositions or vaccines may include compounds (with or without an insoluble metal salt) in plain water (e.g. w.f.i.), but will usually include one or more buffers. Typical buffers include: a phosphate buffer; a Tris buffer; a borate buffer; a succinate buffer; a histidine buffer (particularly with an aluminium hydroxide adjuvant); or a citrate buffer. Buffer salts will typically be included in the 5-20 mM range.

Pharmaceutical compositions or vaccines typically have a pH between 5.0 and 9.5, e.g. between 6.0 and 8.0.

Pharmaceutical compositions or vaccines are preferably sterile and gluten free.

Pharmaceutical compositions or vaccines are suitable for administration to animal (and, in particular, human) patients, and thus include both human and veterinary uses. They may be used in a method of raising an immune response in a patient, comprising the step of administering the composition to the patient.

The pharmaceutical compositions or vaccines of the present invention may be administered before a subject is exposed to *Porphyromonas gingivalis* and/or after a subject is exposed to *Porphyromonas gingivalis.*

Pharmaceutical compositions or vaccines may be prepared in unit dose form. In some embodiments a unit dose may have a volume of between 0.1-1.0 mL e.g. about 0.5 mL.

Pharmaceutical compositions or vaccines of the invention may be prepared in various forms. For example, the pharmaceutical compositions or vaccines may be prepared as injectables, either as liquid solutions or suspensions. Solid forms suitable for solution in, or suspension in, liquid vehicles prior to injection can also be prepared (e.g. a lyophilized composition or a spray-freeze dried composition). The composition may be prepared for topical administration e.g. as an ointment, cream or powder. The composition may be prepared for oral administration e.g. as a tablet or capsule, as a spray, or as a syrup (optionally flavored). The composition may be prepared for pulmonary administration e.g. by an inhaler, using a fine powder or a spray. The composition may be prepared as a suppository. The composition may be prepared for nasal, aural or ocular administration e.g. as a spray or drops. Injectables for intramuscular administration are typical.

A therapeutically effective dosage of one conjugate according to the present invention or of one saccharide of general formula (**I**) refers to that amount of the compound that results in an at least a partial immunization against a disease.

Toxicity and therapeutic efficacy of such compounds can be determined by standard pharmaceutical, pharmacological, and toxicological procedures in cell cultures or experimental animals. The dose ratio between toxic and therapeutic effect is the therapeutic index. The actual amount of the composition administered will be dependent on the subject being treated, on the subject's weight, the severity of the affliction, the manner of administration and the judgement of the prescribing physician.

### Antibody

A further embodiment of the present invention relates to a monoclonal antibody having specificity for the saccharide of general formula (**I**) or a fragment thereof. Said antibody is able to prevent and treat diseases caused by *Porphyromonas gingivalis.* Thus, the monoclonal antibody is useful for passive immunization against *Porphyromonas gingivalis* infections by providing a fast immune response in any subject. The monoclonal antibody is particularly useful for passive immunization against *Porphyromonas gingivalis* infections in a subject having a deficient immune response and who does not respond to active immunization.

The term fragment of a saccharide of general formula (**I**) refers to constituting subunits of a saccharide of general formula (**I**). For example, if the saccharide of general formula (**I**) is a hexasaccharide, then a fragment thereof includes penta-, tetra-, tri-, di- and monosaccharides, which are the constituting units of the hexasaccharide.

The term "antibody" as used herein encompasses polyclonal and monoclonal antibody preparations, as well as preparations including hybrid antibodies, F(ab')₂ fragments, F(ab) molecules, single domain antibodies and functional fragments thereof, which exhibit immunological binding properties of the parent antibody molecule. The antibody disclosed herein may be a monoclonal antibody.

Preferably, the monoclonal antibody has been raised against a conjugate according to the present invention.

Also disclosed herein is a vaccine composition comprising a monoclonal antibody having specificity for the saccharide of general formula (**I**) or a fragment thereof, and a pharmaceutically acceptable carrier.

The vaccine composition typically includes one or more pharmaceutically acceptable carriers (e.g., sterile liquids, such as water and oils, including those of petroleum, animal, vegetable or synthetic origin, such as peanut oil, soybean oil, mineral oil, sesame oil and the like). Water is a more typical carrier when the pharmaceutical composition is administered intravenously. Saline solutions and aqueous dextrose and glycerol solutions can also be employed as liquid carriers, particularly for injectable solutions. Suitable pharmaceutical excipients include, for example, starch, glucose, lactose, sucrose, gelatine, malt, rice, flour, chalk, silica gel, sodium stearate, glycerol monostearate, talc, sodium chloride, dried skimmilk, glycerol, propylene glycol, water, ethanol, and the like. The vaccine composition, if desired, can also contain minor amounts of wetting or emulsifying agents, or pH buffering agents. These compositions can take the form of solutions, suspensions, emulsion, tablets, pills, capsules, powders, sustained-release formulations and the like.

For prophylactic treatment against *Porphyromonas gingivalis* infection, the monoclonal antibody or the vaccine composition can be administered prior to exposure of a subject to the bacteria so that the resulting immune response can inhibit or reduce the severity of the bacterial infection such that the bacteria can be eliminated from said subject.

Also, the monoclonal antibody or the vaccine composition can be administered post infection or after presumed infection, exposure or manifestation of clinical symptoms. In an aspect thereof, the monoclonal antibody or the vaccine composition can be administered in a time period up to 8 hours post infection. Alternatively, the monoclonal antibody combination is administered in a time period up to 24 hours post infection. In a further alternative, the monoclonal antibody combination is administered in a time period up to 48 hours post infection.

The monoclonal antibody or the vaccine composition may be administered, including as a single dose or in multiple sequential doses, up to 8 hours post infection (8hpi), 12hpi, 18hpi, 24hpi, 36hpi, 48hpi, 72hpi, 1 day post infection, 2 days post infection, 3 days post infection, 4 days post infection, 5 days post infection, 6 days post infection 7 days post infection, a week post infection, 10 days post infection, 2 weeks post infection, 3 weeks post infection, 4 weeks post infection, a month post infection, months post infection.

Various delivery systems are known and can be used to administer the monoclonal antibody. Methods of introduction include but are not limited to intradermal, intramuscular, intraperitoneal, intravenous, subcutaneous, intranasal, epidural, intrarectal, and oral routes. The therapeutic agent can be administered, for example by infusion or bolus injection, by absorption through epithelial or mucocutaneous linings (e.g., oral mucosa, rectal and intestinal mucosa, and the like).

### Immunological assay

A further embodiment of the present invention is directed to a saccharide according to the present invention for use as marker in immunological assays for detection of antibodies against *Porphyromonas gingivalis.* Such assays comprise, for instance, microarray and ELISA.

Hence, the inventive saccharide can be used for diagnosis of diseases caused by *Porphyromonas gingivalis.* An assay conducted for diagnostic purposes according to the invention may be an immune assay like a solid-phase enzyme immunoassay (EIA), an enzyme linked immunosorbent assay (ELISA), especially an "indirect" ELISA or a radioimmune assay (RIA). Preferably, the saccharide according to the present invention is covalently linked on the solid support through an interconnecting molecule. Thus, the saccharide according to the present invention can be covalently linked on the solid support directly or indirectly through the nitrogen atom of the -O-L-NH₂ group. The solid support is preferably selected from the group comprising or consisting of: a glass slide, a microtitre plate, test tubes, microspheres, nanoparticle or beads.

It is particularly preferred that the solid support is a glass slide or a microtitre plate. A microtitre plate or microplate or microwell plate is a flat plate with multiple "wells" used as small test tubes. Typically, a microtitre plate having 6, 24, 96, 384 or even 1536 sample wells can be used. Microplates are produced from many different materials, like polycarbonate for microtitre plate used for PCR. The most common is polystyrene as used for most optical detection microplates. It can be colored white by the addition of titanium dioxide for optical absorbance or luminescence detection or black by the addition of carbon for fluorescent biological assays.

**Description of the figures**
- **Figure 1:**: shows a repeating unit of the O-antigen of *Porphyromonas gingivalis* LPS.
- **Figure 2:**: Commercially available interconnecting molecules .
- **Figure 3:**: Examples of functional group X of the interconnecting molecule.

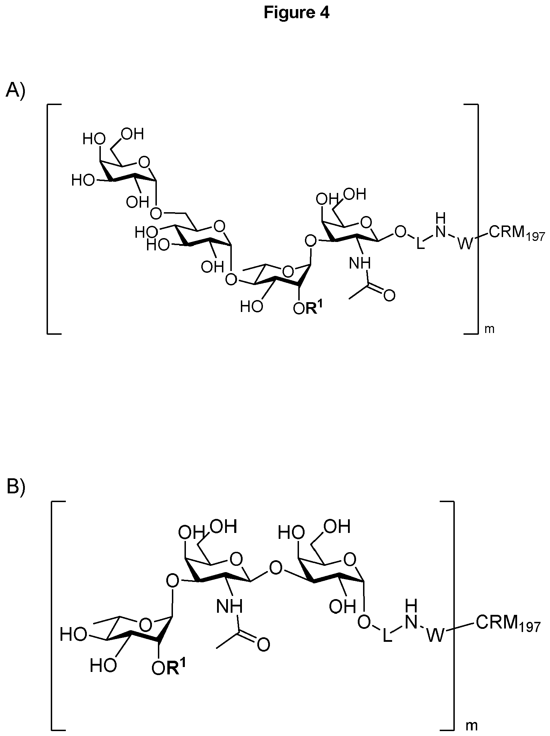

- **Figure 4:**: Shows two CRM₁₉₇ conjugates of the present invention.
- **Figure 5:**: Characterization of conjugates **CRM₁₉₇-tetrasaccharide 1** and **CRM₁₉₇-trisaccharide 2. (A)** MALDI-TOF-MS spectra of glycoconjugate **1. (B)** MALDI-TOF-MS spectra of glycoconjugate **2. (C)** 12% SDS-PAGE of 1 µg CRM₁₉₇, glycoconjugate **1** and glycoconjugate **2.** The PageRuler Prestained Protein Ladder was used as a molecular weight marker. The average carbohydrate to protein loading was 4.5 for glycoconjugate **1** and 7.7 for glycoconjugate **2.**
- **Figure 6:**: Glycan microarray analysis of antibody binding in sera of mice vaccinated with glycoconjugate **1,** glycoconjugate **2** and PBS-CRM₁₉₇. Mean Fluorescence Intensity of **(A):** IgG antibody **(B):** IgM and **(C):** IgA **(D):** exemplary glycan array screens, one serum per group, day 56, Values represent mean ± SEM.
- **Figure 7:**: Glycan microarray analysis of antibody binding in saliva of mice vaccinated with glycoconjugate **1,** glycoconjugate **2** and PBS-CRM₁₉₇. Mean Fluorescence Intensity of **(A):** IgG antibody **(B):** IgM and **(C):** IgA **(D):** exemplary glycan array screens, pooled saliva, day 70, Values represent mean ± SEM.
- **Figure 8:**: Glycan microarray analysis of hybridoma. Mean Fluorescence Intensity of IgG antibody binding to tetrasaccharide **1.** Values represent mean ± SEM. Differences were tested for significance to secondary antibody only using one sample t test with (****) p<0.0001, (**) p<0.01.
- **Figure 9:**: Bacterial ELISA with *P. gingivalis* W50, **1:** sera of mice immunized with **1**-CRM₁₉₇, **2:** sera of mice immunized with **2**-CRM₁₉₇, CRM: mice immunized with CRM₁₉₇, **1**-hybridoma: antibodies generated against **1** with the hybridoma technology, Values represent mean ± SEM. Differences were tested for significance to secondary antibody only using one sample t test with N=3, *: p<0.0332.
- **Figure 10:**: Determination of human IgG and IgA antibodies binding to synthesized LPS-fragments LPS1-11 and positive control 5.06. **(A)** Fluorescence signals indicating IgG antibody binding in saliva of one exemplary chronic periodontitis patient, **(B)** fluorescence signals indicating IgA antibody binding in saliva of one exemplary healthy, and **(C)** associated printing pattern. **D-G)** Mean fluorescence intensity (MFI) indicating IgG or IgA titer of bound human IgG or IgA antibodies in saliva or serum to glycan library.
- **Figure 11:**: Mean fluorescence intensity (MFI) indicating IgG titer of bound human IgG antibodies in saliva to glycan library with labled outlayers.
- **Figure 12:**: Determination of human antibodies in saliva binding to terminal α(1→3)-rhamnose glycans. P-T-01 and P-H-01 are masked. **P ≤ .01, ns - not significant, two-sided unpaired t test.
- **Figure 13:**: Determination of human antibodies in saliva binding to terminal α(1→3)-rhamnose glycans. P-T-01 and P-H-01 are masked. **P ≤ .01, ns - not significant, two-sided unpaired t test.

The following examples are included to demonstrate preferred embodiments of the invention. It should be appreciated by those skilled in the art that the techniques disclosed in the examples, which follow represent techniques discovered by the inventor to function well in the practice of the invention, and thus can be considered to constitute preferred modes for its practice. However, those skilled in the art should, in light of the present disclosure, appreciate that many changes can be made in the specific embodiments, which are disclosed and still obtain a like or similar result.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention.

### Examples

### A. Chemical synthesis

### General Information

All **chemicals** were reagent grade and used as supplied unless otherwise noted. All **solvents** for chemical reactions were commercially purchased in p.a. quality. If stated, they were dried in a Solvent Dispensing System (J.C. Meyer). For HPLC and MS spectrometry, solvents with corresponding quality were used. Water was used from a Milli Q-station from Millipore. The automated syntheses were performed on a home-built synthesizer developed at the Max Planck Institute of Colloids and Interfaces.

Reaction completion, identity, and purity of all compounds were determined by low resolution mass spectrometry **(ESI-LRMS)** or analytical thin-layer chromatography **(TLC).** TLC was performed on Merck silica gel 60 F254 plates (0.25 mm). Compounds were visualized by UV irradiation (254 nm) or stained (5% sulfuric acid in ethanol or Hanessian's Stain: 235 mL of distilled water, 12 g of ammonium molybdate, 0.5 g of ceric ammonium molybdate, and 15 mL sulfuric acid). **Flash column chromatography** was performed on Kieselgel 60 with 230-400 mesh (Sigma-Aldrich, St. Louis, USA). Analysis and purification by normal and reverse phase **HPLC** and ESI-LRMS was performed by using an Agilent 1200 series. ¹H, ¹³C, COSY and HSQC **NMR spectra** were recorded in parts per million (o) relative to the resonance of the solvent on a Varian 400-MR (400 MHz), Varian 600-MR (600 MHz), or Bruker Biospin AVANCE700 (700 MHz) spectrometer. Assignments were supported by COSY and HSQC experiments. High resolution mass spectra **(HRMS)** were obtained using 6210 ESI-TOF mass spectrometer (Agilent) and **MALDI-TOF** autoflex^{™} (Bruker) instruments.

**Abbreviations**
- Ac: Acetyl
- AcOH: Acetic acid
- Ac₂O: Acetic anhydride
- BAIB: Bisacetyliodobenzene
- Bn: Benzyl
- *^{t}*BuOH: *t*-Butanol
- Bz: Benzoyl
- CAN: Cericammonium nitrate
- Cbz: Benzyloxycarbonyl
- Cu(OAc)₂: Copper(II) acetate
- DBU: 1,8-Diazabicyclo[5.4.0]undec-7-ene
- DCC: *N,N'*-Dicyclohexylcarbodiimide
- DCM: Dichloromethane
- DMAP: *N,N*-Dimethylaminopyridine
- DMF: *N,N*'-Dimethylformamide
- ESI: Electrosprayionization
- Et₃N: Triethylamine
- Et: Ethyl
- EtOAc: Ethyl acetate
- FmocCl: 9-Fluorenylmethylchloroformate
- g: Grams
- h: Hours
- HRMS: High resolution mass spectrometry
- Lev: Levulinyl
- min: Minute
- mL: Millilitre
- Me: Methyl
- Mel: Methyl iodide
- MeOH: Methanol
- MP: *p*-Methoxy phenyl
- MS: Molecular sieves
- NaHCOs: Sodium bicarbonate
- NaOH: Sodium hydroxide
- NaOMe: Sodium methoxide
- NIS: *N*-Iodo succinimide
- NMR: Nuclear magnetic resonance
- Pd/C: Palladium on charcoal
- Ph: Phenyl
- Pico: Picoloyl
- CPS: Capsular polysaccharide
- Py: Pyridine
- RT: Room temperature
- TCA: Trichloroacetamide
- TEMPO: 2,2,6,6-Tetramethylpiperidinyloxy
- TfOH: Trifluromethanesulfonic acid
- TMSOTf: Trimethylsilyltrifluromethanesulfonate
- THF: Tetrahydrofuran
- Tol: *p*-Tolyl

### Materials and Measurements

Solvents used for dissolving all building blocks and making of various solutions were taken from Solvent Dispensing System (J.C. Meyer). Wash solvents were HPLC grade. Prior to automated synthesis, the building blocks were weighed and co-evaporated three times with anhydrous toluene and dried for at least one hour under high vacuum prior to use. All solutions were freshly prepared in oven-dried, argon-flushed glassware and kept under argon during the automation process. Isolated product yields were calculated on the basis of resin loading. Functionalized resin **R2** was synthesized as previously reported *(*Green Chem. 2006, 8, 861-867) and resin loading (0.40 mmol/g) was determined following a published protocol (Chem. Rev. 1996, 96, 683-720). Resin was placed in the reaction vessel and was swollen in dichloromethane for 20 min at room temperature before starting the first module. During this time, all reagent lines involved in the synthesis were washed and primed.

Galactosamine building block **5.02** was purchased from GlycoUniverse GmbH & CO KGaA.

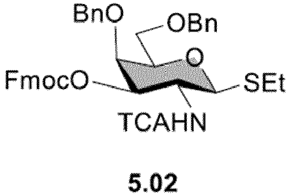

### Preparation of Stock Solutions

**Building Block Solution:** Thioglycoside building block was dissolved in 1 mL (per cycle) of anhydrous CH₂Cl₂.

**Activator Solution/Acidic Wash Solution:** TMSOTf (0.9 mL, 0.62 mmol) was added to 80 mL of anhydrous CH₂Cl₂.

**Pre-Capping Solution:** Pyridine (10 mL) was added to 90 mL of DMF.

**Capping Solution:** Methanesulfonic acid (1.2 mL, 18.5 mmol) and acetic anhydride (6 mL, 63.5 mmol) were added to 50 mL of anhydrous CH₂Cl₂.

**Lev Deprotection Solution:** N₂H₄ . HOAc (725 mg, 7.87 mmol) was dissolved in 50 mL of a 4:1:0.25 mixture of pyridine/acetic acid/water.

**Fmoc Deprotection Solution 1:** Piperidine (20 mL) was added to 80 mL anhydrous DMF.

**Fmoc Deprotection Solution 2:** Et3N (20 mL) was added to 80 mL anhydrous DMF. **CIAc Deprotection Solution:** Thiourea (2.5 g, 32.84 mmol) was dissolved in 55 mL of a 10:1 mixture of 2-methoxyethanol/pyridine.

### Modules for Automated Synthesis

**Initiation:** The resin loaded in the reaction vessel is washed with DMF, THF, and CH₂Cl₂ (3 x 3 mL for 15 s, respectively). The resin is then swollen in 2 mL CH₂Cl₂ for 20 minutes while the temperature of the reaction vessel is cooled to the lowest temperature required throughout the synthesis.

**Module I** - **Acidic Washing:** Once the temperature of the reaction vessel has adjusted to the desired temperature of the subsequent glycosylation by the cooling device, 1 mL of the **Acidic Wash Solution** is delivered to the reaction vessel through the precooling device (set at -20 °C). After three minutes, the solution is drained. Finally, the resin is washed with 3 mL CH₂Cl₂ (bubbling = 15 s) and drained. **Module IIa** - **Glycosylation** (for thioglycosides): Upon draining the CH₂Cl₂ in the reaction vessel, 1 mL of **Building Block Solution 1** containing the appropriate building block is delivered from the building block storing component to the reaction vessel. After the temperature reaches the desired temperature (T₁), **Activator Solution 1** (1 mL) is delivered to the reaction vessel from the respective activator storing component to the reaction vessel The glycosylation mixture is incubated for the selected duration (t₁) at the desired T₁, the reaction temperature is linearly ramped to T₂ (rate = 4 °C/min). Once T₂ is reached, it is maintained, and the reaction mixture is incubated for an additional time (t₂). Once the incubation time is finished, the reaction mixture is drained and the resin is washed with CH₂C1₂(1 x 2 mL for 15 s), then dioxane (1 x 2 mL for 15 s), and finally CH₂Cl₂(2 x 2 mL for 15 s).

**Module Illa** - **Capping:** The resin is washed with DMF (2 x 3 mL for 15 s). Then **Pre-capping Solution** (2 mL) is delivered, and the reaction temperature is adjusted to and maintained at 50 °C
for one minute (max power = 5 W). The resin is then washed with CH2Cl2 (3 x 2 mL for 15). Upon washing, **Capping Solution** (4 mL) is delivered and the temperature is adjusted and maintained 25 °C). The resin and the reagents are incubated for 8 min. The solution is then drained from the reactor vessel and the resin is washed with CH2Cl2 (3 x 3 mL for 15 s).

**Module IVa** - **Fmoc Deprotection 1:** The resin is first washed with DMF (3 x 3 mL for 15 s), and then **Fmoc Deprotection Solution 1** (2 mL) is delivered to the reaction vessel. The temperature of the reagents inside the reactor vessel is then adjusted to and maintained at 60 °C. After 1 min the reaction solution is drained and the resin is washed with DMF (3 x 3 mL for 15 s) and CH2Cl2 (5 x 3 mL for 15 s). After this module the resin is ready for the next glycosylation cycle.

**Module IVb** - **Lev Deprotection:** The resin is washed with CH2Cl2 (3 x 2 mL for 15 s), and then **Lev Deprotection Solution** (2 mL) is delivered to the reaction vessel. The temperature of the reagents inside the reactor vessel is then adjusted to and maintained at 25 °C. After 5 min, the reaction solution is drained from the reactor vessel and the resin is washed with CH2Cl2 (3 x 2 mL for 15 s). Then, of fresh **Lev Deprotection Solution** (2 mL) is delivered and the process is repeated twice more. Then, the resin is washed with DMF, THF, and CH2Cl2 (3 x 3 mL for 15 s, respectively). After this module the resin is ready for the next glycosylation cycle. **Module IVc** - **Fmoc Deprotection 2:** The resin is first washed with DMF (3 x 3 mL for 15 s), and then **Fmoc Deprotection Solution 2** (2 mL) is delivered to the reaction vessel. The temperature of the reagents inside the reactor vessel is then adjusted to and maintained at 60 °C. After 5 min the reaction solution is drained and the resin is washed with DMF (3 x 2 mL for 15 s). Then, fresh **Fmoc Deprotection Solution 2** (2 mL) is delivered and the process is repeated twice more. Then, the resin is washed with DMF (3 x 3 mL) and CH2Cl2 (3 x 3 mL) for 15 s each time. After this module the resin is ready for the next glycosylation cycle.

**Module IVe** - **CIAc Deprotection:** The resin is first washed with CH2Cl2 (3 x 2 mL for 15 s) then **CIAc Deprotection Solution** (2 mL) was delivered to the reaction vessel. The temperature of the reagents inside the reactor vessel is then adjusted to and maintained at 90 °C. After 22 min, the reaction solution is drained from the reactor vessel. The resin is washed with DMF (3 x 2 mL for 15 s). Then fresh **CIAc Deprotection Solution 2** (2 mL) is delivered, and the process is repeated twice more. Then, the resin is washed with DMF (3 x 3 mL for 15 s) and CH2Cl2 (5 x 3 mL for 15 s). After this module the resin is ready for the next glycosylation cycle.

### Post-automated Synthesis Manipulations, Analysis and Purification Cleavage from Solid Support (Method A-1): Protected Oligosaccharides

After automated synthesis, the resin was removed from the reaction vessel, suspended in CH₂Cl₂ (20 mL), and photocleaved in a continuous-flow photoreactor. A Vapourtec E-Series easy-MedCHem, equipped with a UV-150 Photochemical reactor having a UV-150 Medium- Pressure Mercury Lamp (arc length 27.9 cm, 450 W) surrounded by a long-pass UV filter (Pyrex, 50% transmittance at 305 nm) was used. A Pump 11 Elite Series (Harvard Apparatus syringe pump at a flow rate of 0.8 mL/min was used to pump the mixture through a FEP tubing (i.d. 3.0 inch, volume: 12 mL) at 20 °C. The reactor was washed with 20 mL CH₂Cl₂ at a flow rate of 2.0 mL/min. The output solution was filtered to remove the resin and the solvent was evaporated *in vacuo.* Crude was then analyzed by MALDI.

### Deprotection of Oligosaccharides

AGA-synthesized and photocleaved product was subjected to methanolysis and hydrogenolysis. The hydrogenolysis product was purified by RP-HPLC and lyophilized on a Christ Alpha 2-4 LD plus freeze dryer to afford the final deprotected compound.

▪ **Methanolysis (Method C):** To a solution of protected oligosaccharide in MeOH:CH₂Cl₂ (2 mL, 1:1), sodium methoxide (0.5 M solution in MeOH, 2.2 equiv. per ester group) was added. The mixture was stirred at room temperature for 2 h. Then Amberlite IR-120 (H⁺ form) was added to quench. After neutralization, the reaction mixture was filtered, and the solvent was removed in vacuo. The crude compound was used for hydrogenolysis without further purification.

**Hydrogenolysis (Method D):** The crude compound obtained after methanolysis was dissolved in 4 mL of EtOAc:t-BuOH:H₂O (2:1:1). Pd/C (10%) was added to the solution and the suspension was stirred in a H2 bomb with 60 psi pressure over night. The insoluble material was removed by a CHROMAFIL ^{®}Xtra, RC 0.45 syringe filter. The solid was washed once with t-BuOH and several times with water. The filtrate was collected and concentrated in vacuo.

### Analytical NP-HPLC of Crude Material (Method B-2)

Analytical NP-HPLC was conducted on an Agilent 1200 Series system. A YMC-Diol-300-NP column (150 mm x 4.60 mm I.D.) was used at a flow rate of 1.00 mL/min with hexane/EtOAc as eluent (20% EtOAc in hexane for 5 min, 20 → 55% EtOAc in hexane over 35 min, 55 → 100% EtOAc in hexane over 35 min, 100% EtOAc for 10 min).

### Analytical/preparative RP-HPLC of Crude Material (Method E-1)

Crude products were dissolved in water and analyzed/purified using analytical/preparative HPLC. A Thermo-Scientific Hypercarb column (150 mm x 4.60 mm I.D.) was used for analytical RP-HPLC with a flow rate of 0.70 mL/min with water (0.1% HCO₂H)/acetonitrile as eluents (100% H₂O (0.1% HCO₂H) for 5 min, 0 → 30% acetonitrile in H₂O (0.1% HCO₂H) over 30 min, 30 → 100% acetonitrile in H₂O (0.1% HCO₂H) over 5 min, 100% acetonitrile for 5 min).

### Example A.1: Preparation of Rhamnose Building Blocks 5.04a and 5.04b

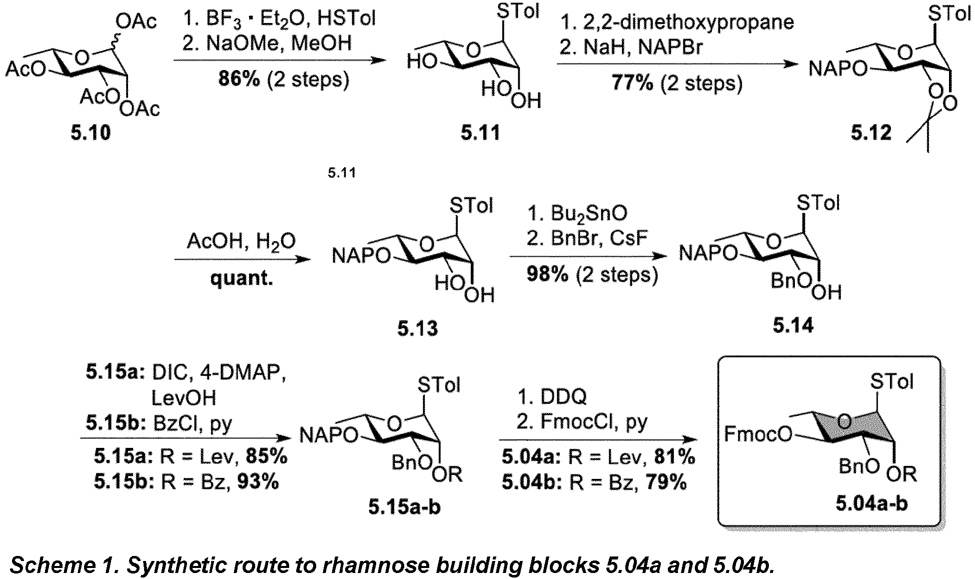

### 4-Methylphenyl 2,3-O-(1-methylethylidene)-4-O-(2-naphthalenylmethyl)-1 - thio-α-L-rhamnopyranoside (5.12)

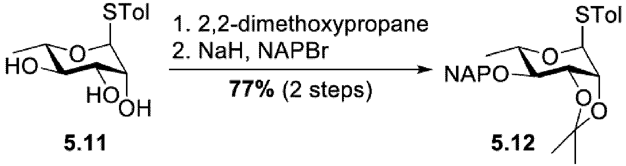

Rhamnopyranose **5.11** *(*Trends Microb. 2003, 11, 554-561) (4.13 g, 15.3 mmol, 1.0 equiv.) was dissolved in anhydr. acetone (60 mL). 2,2,-Dimethoxypropane (7.5 mL, 61.2 mmol, 4.0 equiv.) was added followed by para-toluenesulfonic acid (582 mg, 3.1 mmol, 0.2 equiv.). The reaction mixture was stirred for 6 h at room temperature. Then, it was quenched by the addition of sat. aq. NaHCOs, filtered and concentrated under reduced pressure. The residue was extracted with CH₂Cl₂ (2 x 100 mL), dried over Na₂SO₄, filtered and concentrated. 2,3-Isopropylidene product (3.7 g, 11.9 mmol, 78%) was obtained as a colorless solid after purification by column chromatography (Hex:AcOEt 3:1). R*_{f}* = 0.48 (Hex:AcOEt, 3:1). The residue (3.7 g, 11.9 mmol, 1.0 equiv.) was dissolved in dry *N,N*-dimethylformamide (40 mL). The stirred solution was cooled to 0 °C and sodium hydride (1.1 g, 27.4 mmol; 60% dispersion in mineral oil, 2.3 eq.) was added in small portions. After 30 min, NAPBr (4.0 g, 17.9 mmol, 1.5 equiv.) was added. The reaction mixture was allowed to warm up to room temperature and was stirred over night. Methanol (10 mL) was added, the reaction mixture was stirred for 10 min and afterwards diluted with ethyl acetate (100 mL). The organic layer was washed with water (2 x 100 mL). The aqueous phase was extracted with ethyl acetate (2 x 100 mL). The combined organic phase was washed with water (100 mL), dried over Na₂SO₄ and concentrated. Product **5.12** (4.0 g, 8.9 mmol, **75%)** was obtained as a colorless oil after purification by column chromatography (Hex:AcOEt 9:1 to 3:1).

**R*_{f}* =** 0.8 (Hex:AcOEt, 1:1).

**¹H NMR** (400 MHz, CDCl₃) δ 7.88 - 7.83 (m, 4H), 7.56 - 7.46 (m, 3H), 7.42 - 7.37 (m, 2H), 7.17 - 7.12 (m, 2H), 5.71 (s, 1H), 4.98 (dd, *J* = 108.5, 11.8 Hz, 2H), 4.41 - 4.39 (m, 1H), 4.23 (dd, *J* = 9.8, 6.2 Hz, 1H), 3.42 - 3.35 (m, 1H), 1.55 (s, 3H), 1.43 (s, 3H), 1.30 (d, *J* = 6.2 Hz, 3H) ppm.

¹³C **NMR** (101 MHz, CDCl₃) δ 137.9, 135.8, 133.3, 133.1, 132.6, 129.9, 129.8, 128.2, 128.0, 127.8, 126.9, 126.2, 126.0, 109.5, 84.3, 81.5, 78.6, 76.8, 73.2, 66.2, 28.2, 26.6, 21.2, 17.9 ppm.

**HRMS (QToF):** Calcd for C₂₇H₃₀O₄SNa [M + Na]⁺473.1757; found 473.1743.

### 4-Methylphenyl 4-O-(2-naphthalenylmethyl)-1-thio-α-L-rhamnopyranoside (5.13)

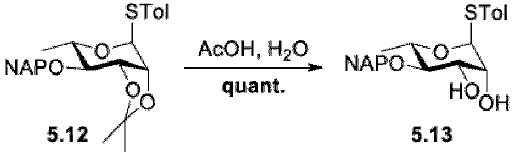

2,3-Isopropylidene **5.12** (4.0 g, 8.8 mmol, 1.0 equiv.) was dissolved in acetic acid/water (9:1, 50 mL) solution and reacted for 18 h at 50 °C. The mixture was concentrated and purified by column chromatography (Hex:AcOEt 3:1 to 1:1) to obtain title compound **5.13** (3.6 g, 1.7 mmol, 99%) as a colorless syrup.

**R*_{f}*** = 0.27 (Hex:AcOEt, 1:1).

**¹H NMR** (400 MHz, CDCl₃) δ 7.90 - 7.76 (m, 4H), 7.53 - 7.46 (m, 3H), 7.40 - 7.31 (m, 2H), 7.12 (dd, *J* = 8.2, 4.1 Hz, 2H), 5.41 (d, *J* = 1.7 Hz, 1H), 4.92 (d, *J* = 3.2 Hz, 2H), 4.32 - 4.23 (m, 1H), 4.19 (dd, J= 3.4, 1.6 Hz, 1H), 3.97 (dd, *J* = 9.1, 3.4 Hz, 1H), 3.47 (t, *J* = 9.2 Hz, 1H), 2.33 (s, 3H), 1.39 (d, *J* = 6.2 Hz, 3H) ppm.

**¹³C NMR** (101 MHz, CDCl₃) δ 137.9, 135.7, 133.4, 133.2, 132.3, 130.3, 130.0, 129.9, 128.7,128.1, 127.9, 126.9, 126.4, 126.3, 125.9, 87.9, 82.0, 75.3, 72.7, 72.0, 68.7, 21.3, 18.1 ppm.

**HRMS (QToF):** Calcd for C₂₄H₂₆O₄SNa [M + Na]⁺433.1444; found 433.1432.

### 4-Methylphenyl 3-O-benzyl-4-O-(2-naphthalenylmethyl)-1-thio-α-L-rhamnopyranoside (5.14)

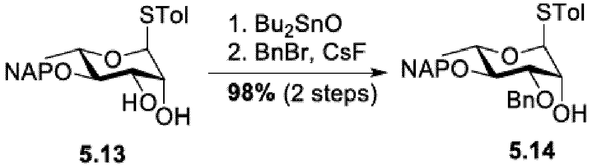

Diol **5.13** (7.0 g, 17.1 mmol, 1.0 equiv.) was dissolved in MeOH (100 mL) and Bu₂SnO (8.5 g, 34.2 mmol, 2.0 equiv.) was added. The reaction mixture was then heated up to 65 °C and stirred at the same temperature overnight. The reaction mixture was co-evaporated with toluene and dried under high vacuum. The crude was dissolved in DMF (100 mL) and benzyl bromide (2.45 mL, 20.5 mmol, 1.2 equiv.), and CsF (3.4 g, 22.2 mmol, 1.3 equiv.) were added to the stirred reaction mixture at room temperature under argon atmosphere. The reaction was stirred at room temperature for 5 h and then diluted with EtOAc, passed through a short plug of silica gel and
dried under vacuum. The title compound **5.14** (8.4 g, 16.8 mmol, 98%) was obtained as a colorless oil after purification by column chromatography (Hex:AcOEt 9:1 to 3:1).

**R*_{f}*** = 0.41 (Hex:AcOEt, 3:1).

**¹H NMR** (400 MHz, CDCl₃) δ 7.86 - 7.76 (m, 4H), 7.53 - 7.43 (m, 3H), 7.41 - 7.32 (m, 7H), 7.12 (d, *J* = 8.2 Hz, 2H), 5.47 (d, *J* = 1.8 Hz, 1H), 4.94 (dd, *J* = 91.4, 11.2 Hz, 2H), 4.74 (s, 2H), 4.31 - 4.20 (m, 2H), 3.91 (dd, *J* = 9.0, 3.2 Hz, 1H), 3.59 (t, *J* = 9.3 Hz, 1H), 2.33 (s, 3H), 1.34 (d, *J* = 6.2 Hz, 3H) ppm.

**¹³C NMR** (101 MHz, CDCl₃) δ 137.8, 137.7, 135.9, 133.4, 133.1, 132.2, 130.3, 130.0, 128.8, 128.3, 128.2, 128.1, 128.1, 127.8, 126.8, 126.2, 126.1, 126.0, 87.5, 80.3, 80.2, 75.6, 72.3, 70.2, 68.8, 21.3, 18.0 ppm.

**HRMS (QToF):** Calcd for C₃₁H₃₂O₄SNa [M + Na]⁺ 523.1913; found 523.1924.

### 4-Methylphenyl 3-O-benzyl-2-O-levulinyl-4-O-fluorenylmethoxycarbonyl-1-thio-α-L-rhamnopyranoside (5.15a)

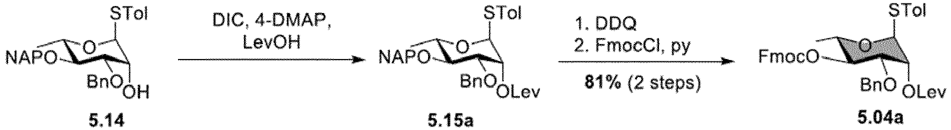

To a solution of **5.14** (800 mg, 1.6 mmol, 1.0 equiv.) and LevOH (0.33 mL, 3.2 mmol, 2.0 equiv.) in anhydrous CH₂Cl₂ (15 mL) was added DIC (0.75 mL, 4.8 mmol, 3.0 equiv.) and 4- DMAP (39 mg, 0.32 mmol, 0.2 equiv.) at 0 °C. The reaction was stirred at room temperature overnight. The reaction mixture was filtered through Celite, the filtrate was washed with aqueous NaHCOs and the aqueous phase was extracted with CH₂Cl₂. The combined organic phase was dried over Na₂SO₄, filtered and concentrated. Sugar **5.15a** (810 mg, 1.35 mmol, 85%) was obtained as a colorless syrup after purification by column chromatography (Hex:AcOEt 3:1). **R*_{f}*** = 0.35 (Hex: EtOAc 3:1). To a well stirred emulsion of sugar **5.15a** (810 mg, 1.3 mmol, 1.0 equiv.) in CH₂Cl₂/water (7:1, 24 mL), was added DDQ (337 mg, 1.5 mmol, 1.1 equiv.) and the suspension was stirred at room temperature for 1.5 h protected from light. The mixture was diluted with CH₂Cl₂, washed with 10% Na₂S₂O₃ and saturated aqueous NaHCOs solution. The organic layer was dried over Na₂SO₄, filtered, concentrated and the residue was purified by column chromatography (Hex:AcOEt 9:1 to 3:1) to obtain **3-OH-sugar** (620 mg, 1.4 mmol, quant.) as a colorless solid. **R*_{f}*** = 0.35 (Hex:AcOEt 3:1). To a solution of **3-OH-sugar** (620 g, 1.4 mmol, 1.0 equiv.) and anhydrous pyridine (0.55 mL, 6.7 mmol, 5.0 equiv.) in anhydrous CH₂Cl₂(15 mL) at 0 °C was added FmocCl (524 mg, 2.0 mmol, 1.5 equiv.) followed by 4-DMAP (8 mg, 0.1 mmol, 0.05 equiv.) was added and the reaction mixture was stirred for two hours at 0 °C. Aqueous citric acid solution (10 mL) was added and the mixture was allowed to warm up to room temperature. The aqueous phase was extracted with CH₂Cl₂ and the combined organic phase was dried over Na₂SO₄, filtered and concentrated. The title compound **5.04a** (840 mg, 1.2 mmol, 91%) was obtained as a white solid after purification by column chromatography (Hex:AcOEt = 19:1 to 3:1).

**R*_{f}*** = 0.25 (Hex:AcOEt 3:1).

**[α]_{D}** -39.32 cm⁻¹ (*c* 1, CHCl₃).

**IR** (film): 3067, 2872, 1749, 1723, 1603, 1495, 1451, 1386, 1246, 1152, 1102, 986, 848, 784, 738, 698 cm⁻¹.

**HRMS** (QToF): Calcd for C₄₀H₄₀O₈SNa [M + Na]⁺703.2336; found 703.2354.

**¹H NMR (400 MHz, CDCl₃):** δ 7.79 (d, *J* = 7.5 Hz, 2H), 7.67 - 7.57 (m, 2H), 7.46 - 7.36 (m, 2H), 7.35 - 7.30 (m, 4H), 7.26 - 7.20 (m, 5H), 7.12 (d, *J* = 7.9 Hz, 2H), 5.57 (dd, *J* = 3.3, 1.7 Hz, 1H), 5.35 (d, *J* = 1.6 Hz, 1H), 4.88 (t, *J* = 9.8 Hz, 1H), 4.65 (d, *J* = 11.9 Hz, 1H), 4.52 - 4.40 (m, 3H), 4.35 (dd, *J* = 9.8, 6.2 Hz, 1H), 4.26 (t, *J* = 7.2 Hz, 1H), 3.88 (dd, *J* = 9.7, 3.2 Hz, 1H), 2.68 (ddd, *J* = 10.8, 5.1, 1.3 Hz, 4H), 2.33 (s, 3H), 2.15 (s, 3H), 1.26 (d, *J* = 6.2 Hz, 3H) ppm.

**¹³C NMR (101 MHz, CDCl₃):** δ 206.44, 171.98, 154.99, 143.51, 143.31, 141.45, 138.30, 137.41, 132.49, 130.09, 129.59, 128.50, 128.07, 127.99, 127.34, 125.23, 120.25, 86.32, 74.97, 71.56, 70.41, 70.13, 67.50, 46.94, 38.10, 29.93, 28.24, 21.29, 17.43 ppm.

### 4-Methylphenyl 2-O-benzoyl 3-O-benzyl-4-O-fluorenylmethoxycarbonyl-1-thio-α-L-rhamnopyranoside (5.04b)

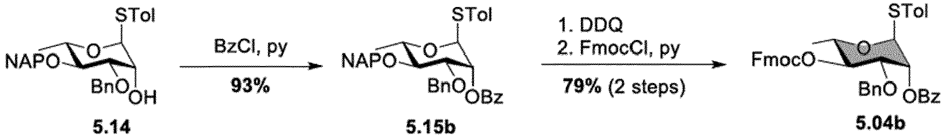

To a solution of **5.14** (5.0 g, 10.0 mmol, 1.0 equiv.) in anhydrous pyridine (10 mL), BzCl (3.48 mL, 30.0 mmol, 3.0 equiv.) was added at 0 °C under an argon atmosphere. The mixture was allowed to warm up to room temperature and stirred for 16 h. The reaction mixture was diluted with water and extracted with ethyl acetate. The combined organic phase was washed with aqueous citric acid solution (10% v/v) and brine, dried over Na₂SO₄, filtered and concentrated. The benzoylated **5.15b** (5.6 g, 9.3 mmol, 93%) was obtained as a colorless syrup after purification by column chromatography (Hex/EA 9:1). R*_{f}* = 0.41 (Hex: EtOAc 9:1). To a well stirred emulsion of **5.15b** (5.5 g, 9.1 mmol, 1.0 equiv.) in CH₂Cl₂/water (7:1, 48 mL), was added DDQ (1.3 g, 10.0 mmol, 1.1 equiv.) and the suspension was stirred at room temperature for 1.5 h protected from light. The mixture was diluted with CH₂Cl₂, washed with 10% aqueous Na₂S₂O₃ and saturated aqueous NaHCOs solution. The organic layer was dried over Na₂SO₄, filtered, concentrated and the residue was purified by column chromatography (Hex/EA 9:1 to 3:1) to obtain the **4-OH-sugar** (3.8 g, 6.5 mmol, 71%) as a colorless solid. R*_{f}* = 0.41 (Hex:EA 3:1). To a solution of **4-OH-sugar** (3.8 g, 8.2 mmol, 1.0 equiv.) and anhydrous pyridine (3.31 mL, 40.9 mmol, 5.0 equiv.) in anhydrous CH₂Cl₂ (60 mL) at 0 °C was added FmocCl (3.2 g, 1.5 mmol, 1.5 equiv.) followed by 4-DMAP (50 mg, 0.4 mmol, 0.05 equiv.) was added and the reaction mixture was stirred for two hours at 0 °C. Aqueous citric acid solution (30 mL) was added and the mixture was allowed to warm up to room temperature. The aqueous phase was extracted with CH₂Cl₂ and the combined organic phase was dried over Na₂SO₄, filtered and concentrated. The title compound **5.04b** (4.9 g, 7.1 mmol, 87%) was obtained as a white solid after purification by column chromatography (Hex/EtOAc = 19:1 to 3:1).

R*_{f}* = 0.69 (Hex:EA 3:1).

**[α]_{D}** -54.58 cm⁻¹ (c 1, CHCl₃).

**IR** (film): 3067, 2924, 1752, 1721, 1585, 1494, 1451, 1385, 1244, 1176, 1090, 988, 848, 758, 740, 709 cm⁻¹.

**HRMS** (QToF): Calcd for C₄₂H₃₃O₇SNa [M+ Na]⁺709.2230; found 709.2250.

**¹H NMR (600 MHz, CDCl₃):** δ 8.10 - 8.05 (m, 2H), 7.79 (d, J = 7.5 Hz, 2H), 7.66 - 7.53 (m, 3H), 7.49 - 7.39 (m, 4H), 7.38 - 7.25 (m, 6H), 7.22 (q, J = 2.5 Hz, 3H), 7.13 (d, J = 8.2 Hz, 2H), 5.80 (dd, J = 3.3, 1.7 Hz, 1H), 5.50 (d, J = 1.8 Hz, 1H), 5.06 (t, J = 9.8 Hz, 1H), 4.65 (dd, J = 94.5, 12.1 Hz, 2H), 4.47 (d, J = 7.3 Hz, 2H), 4.42 (dd, J = 9.8, 6.2 Hz, 1H), 4.26 (t, J = 7.2 Hz, 1H), 4.01 (dd, J = 9.7, 3.3 Hz, 1H), 2.33 (s, 3H), 1.32 (d, J = 6.3 Hz, 3H) ppm.

**¹³C NMR (151 MHz, CDCl₃):** δ 165.8, 155.0, 143.5, 143.3, 141.5, 141.4, 138.3, 137.4, 133.5, 132.5, 130.1, 130.1, 129.7, 129.6, 128.6, 128.5, 128.1, 127.9, 127.3, 125.2, 125.2, 120.2, 86.6, 77.4, 75.0, 71.6, 70.9, 70.1, 67.7, 47.0, 21.3, 17.6 ppm.

### Example A.2: Preparation of Galactose Building Block 5.03

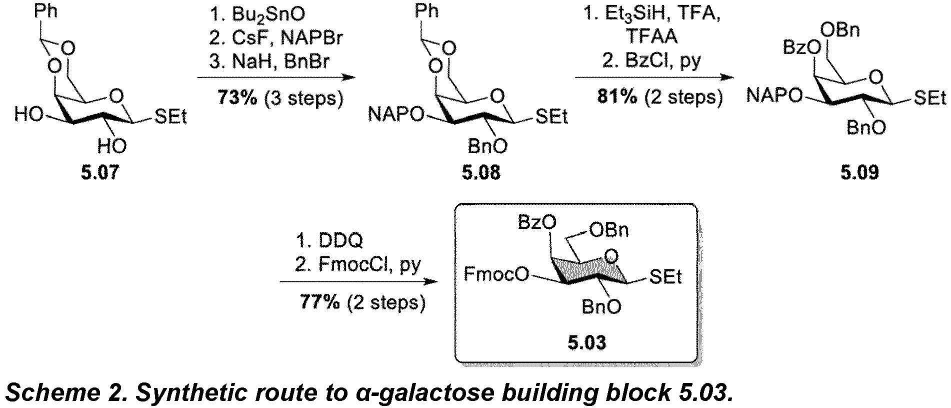

### Ethyl 2-O-benzyl-3-O-(2-naphthalenylmethyl)-4,6-O-[(S)-phenylmethylene]-1-thio-β-D- galactopyranoside (5.08)

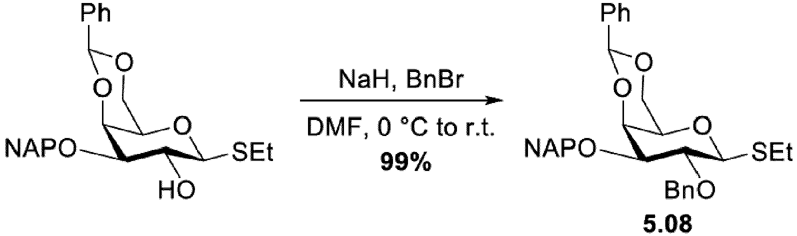

**OH-galactose** (7.4 g, 16.4 mmol, 1.0 equiv.) was dissolved in anhydrous *DMF* (75 mL). The stirred solution was cooled to 0 °C and sodium hydride (1.5 g, 24.6 mmol; 60% dispersion in mineral oil, 2.3 equiv.) was added in small portions. After 30 min, benzyl bromide (1.3 mL, 37.7 mmol, 1.5 equiv.) was added dropwise. The reaction mixture was allowed to warm up to room temperature and was stirred overnight. Methanol (10 mL) was added, the reaction mixture was stirred for 10 min and afterwards diluted with ethyl acetate (100 mL). The organic layer was washed with water (2 x 100 mL). The aqueous phase was extracted with ethyl acetate (2 x 100 mL). The combined organic phase was washed with water (100 mL), dried over Na₂SO₄ and concentrated. Product **5.08** (8.8 g, 16.2 mmol, **99%**) was obtained as a colorless solid after purification by column chromatography (SiO₂, Hex/EtOAc = 3:1).

**R*_{f}*** = 0.22 (Hex/EtOAc 3:1).

**¹H NMR** (700 MHz, CDCl₃) δ 7.87 - 7.78 (m, 3H), 7.74 - 7.69 (m, 1H), 7.57 - 7.29 (m, 13H), 5.48 (s, 1H), 4.96 - 4.87 (m, 4H), 4.44 (d, *J* = 9.7 Hz, 1H), 4.30 (dd, *J* = 12.3, 1.8 Hz, 1H), 4.17 (d, *J* = 3.7 Hz, 1H), 3.96 - 3.90 (m, 2H), 3.65 (dd, *J* = 9.2, 3.5 Hz, 1H), 3.35 - 3.32 (m, 1H), 2.90 - 2.73 (m, 2H), 1.34 (t, *J* = 7.5 Hz, 3H) ppm.

**¹³C NMR** (176 MHz, CDCl₃) δ 138.6, 138.1, 135.9, 133.4, 133.2, 129.2, 128.5, 128.5, 128.4, 128.3, 128.0, 127.9, 127.8, 126.7, 126.7, 126.3, 126.1, 126.0, 101.7, 84.6, 81.1, 77.1, 75.9, 74.2, 72.1, 69.9, 69.5, 24.0, 15.2 ppm.

**HRMS** (QToF): Calcd for C₃₃H₃₄O₅SNa [M + Na]⁺ 565.2019; found 565.2018.

### Ethyl 4-O-benzoyl-2,6-bis-O-benzyl-3-O-(2-naphthalenylmethyl)-1-thio-β-D-galactopyranoside (5.09)

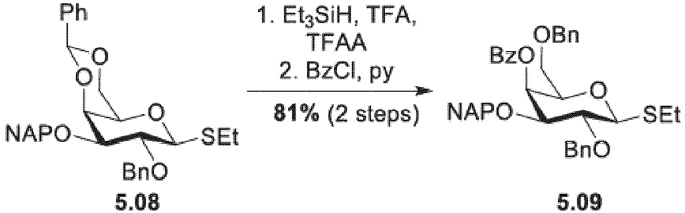

**Compound 5.08** (2.0 g, 3.7 mmol, 1.0 equiv.) was co-evaporated with anhydrous toluene (2 x 3 mL), and dissolved in anhydrous CH₂Cl₂ (20 mL). Triethylsilane (3.5 mL, 22.1 mmol, 6.0 equiv.) and trifluoroacetic anhydride (0.52 mL, 3.7 mmol, 1.0 equiv.) were added and the solution was cooled to 0 °C. Trifluoroacetic acid (1.7 mL, 22.1 mmol, 6.0 equiv.) was added dropwise. The mixture was allowed to warm up to room temperature and was stirred for 5 h. The solution was diluted with CH₂Cl₂ and quenched with saturated aqueous NaHCOs (40 mL). The aqueous phase was extracted with CH₂Cl₂ (2 x 60 mL) and the combined organic phase was washed with water (60 mL), dried over Na₂SO₄, filtered and concentrated. **4-OH-galactose** (1.8 g, 3.3 mmol, **90%**) was obtained as a colorless syrup after purification by column chromatography (SiO₂, Hex/EtOAc = 3:1 to 1:1). **R*_{f}*** = 0.39 (Hex/EtOAc 1:1). **4-OH-galactose** (1.4 g, 2.6 mmol, 1.0 equiv.) was dissolved in pyridine (20 mL) and the solution was cooled to 0 °C. Benzoyl chloride (0.89 mL, 7.7 mmol, 3.0 equiv.) was added under an argon atmosphere. The reaction mixture was allowed to warm up to room temperature and was stirred for 16 h. The reaction was quenched by addition of water and the aqueous layer was extracted with ethyl acetate. The combined organic phase was washed with 1.0 M HCl and brine, dried over Na₂SO₄, filtered and concentrated. The title compound **5.09** (1.2 g, 1.8 mmol, 72%) was obtained as a colorless oil after purification by column chromatography (Hex/EtOAc = 9:1 to 3:1).

R*_{f}* = 0.31 (Hex:EA 3:1).

**HRMS (QToF):** Calcd for C₄₀H₄₀O₆SNa [M + Na]⁺671.2438; found 671.2488.

**¹H NMR (400 MHz, CDCl₃):** δ 8.16 - 7.98 (m, 3H), 7.81 - 7.68 (m, 3H), 7.65 - 7.56 (m, 2H), 7.52 - 7.13 (m, 14H), 5.94 (dd, *J* = 3.3, 1.0 Hz, 1H), 5.02 (d, *J* = 11.6 Hz, 1H), 4.89 - 4.66 (m, 3H), 4.57 - 4.36 (m, 3H), 3.84 (d, *J* = 1.2 Hz, 1H), 3.77 (d, *J =* 3.3 Hz, 1H), 3.75 - 3.61 (m, 2H), 3.58 (d, *J* = 7.1 Hz, 1H), 2.78 - 2.66 (m, 2H), 1.34 (t, *J* = 7.5 Hz, 3H) ppm.

**¹³C NMR (101 MHz, CDCl₃):** δ 165.9, 138.2, 137.7, 135.4, 133.8, 133.3, 133.1, 130.3, 130.2, 130.0, 128.6, 128.5, 128.5, 128.2, 128.1, 127.9, 127.0, 126.3, 126.0, 125.9, 99.7, 85.6, 81.1, 77.9, 77.4, 76.2, 76.0, 73.9, 71.9, 68.4, 67.6, 25.1, 15.2 ppm.

### Ethyl 4-O-benzoyl-2,6-bis-O-benzyl-3-O-fluorenylmethoxycarbonyl-1-thio-β-D-galactopyranoside (5.03)

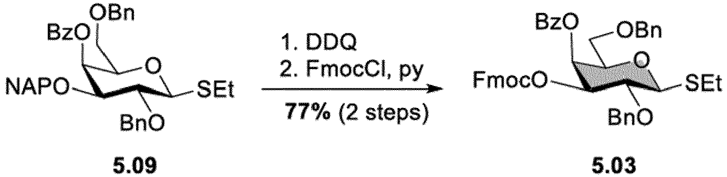

To a well stirred emulsion of **5.09** (1.8 g, 2.8 mmol, 1.0 equiv.) in CH₂Cl₂/water (7:1, 24 mL), was added DDQ (692 mg, 3.1 mmol, 1.1 equiv.) and the suspension was stirred at room temperature for 1.5 h protected from light. The mixture was diluted with CH₂Cl₂, washed with aqueous 10% Na₂S₂O₃ and saturated aqueous NaHCOs solution. The organic layer was dried over Na₂SO₄, filtered, concentrated and the residue was purified by column chromatography (Hex/EA 9:1 to 3:1) to obtain the title compound (1.1 g, 2.2 mmol, 78%) as a colorless solid. R*_{f}* = 0.35 (Hex:EA 3:1). To a solution of **4-OH-sugar** (1.1 g, 2.2 mmol, 1.0 equiv.) and anhydrous pyridine (0.87 mL, 10.8 mmol, 5.0 equiv.) in anhydrous CH₂Cl₂ (15 mL) at 0 °C was added FmocCl (838 mg, 3.2 mmol, 1.5 equiv.) followed by 4-DMAP (13 mg, 0.11 mmol, 0.05 equiv.) and the reaction mixture was stirred for two hours at 0 °C. Aqueous citric acid solution (10 mL) was added and the mixture was allowed to warm up to room temperature. The aqueous phase was extracted with CH₂Cl₂ and the combined organic phase was dried over Na₂SO₄, filtered and concentrated. The title compound (1.2, 1.6 mmol, 76%) was obtained as a white solid after purification by column chromatography (Hex/EtOAc = 19:1 to 3:1).

R*_{f}* = 0.5 (Hex:EA 3:1).

**[a]D** 14.29 cm⁻¹ (c 1, CHCl₃).

**IR** (film): 3066, 2927, 2869, 1750, 1726, 1603, 1497, 1451, 1388, 1274, 1246, 1096, 1070, 1026, 984, 907, 758, 737, 697 cm⁻¹.

**HRMS** (QToF): Calcd for C₄₄H₄₂O₈SNa [M + Na]⁺ 753.2493; found 753.2518.

**¹H NMR (400 MHz, CDCl₃):** δ 8.10 - 8.03 (m, 2H), 7.76 (ddt, *J* = 7.7, 1.9, 0.9 Hz, 2H), 7.63 (d, *J* = 7.4 Hz, 1H), 7.60 - 7.52 (m, 2H), 7.49 (t, *J* = 7.8 Hz, 2H), 7.44 - 7.31 (m, 4H), 7.31 - 7.12 (m, 10H), 5.90 (dd, *J* = 3.4, 1.1 Hz, 1H), 5.00 (dd, *J* = 9.6, 3.4 Hz, 1H), 4.88 (d, *J* = 10.5 Hz, 1H), 4.71 (d, *J* = 10.5 Hz, 1H), 4.66 - 4.56 (m, 2H), 4.39 (d, *J* = 12.0 Hz, 1H), 4.31 (t, *J* = 7.4 Hz, 1H), 4.19 (dd, *J* = 10.3, 8.3 Hz, 1H), 3.98 (td, *J* = 6.4, 1.1 Hz, 1H), 3.81 (t, *J* = 9.7 Hz, 1H), 3.59 (ddd, *J* = 33.9, 9.6, 6.4 Hz, 2H), 2.93 - 2.74 (m, 2H), 1.37 (t, *J* = 7.4 Hz, 3H) ppm.

**¹³C NMR (101 MHz, CDCl₃):** δ 165.77, 154.36, 143.97, 143.20, 141.42, 141.29, 137.75, 137.57, 133.52, 130.26, 129.56, 128.62, 128.47, 128.28, 127.99, 127.97, 127.94, 127.87, 127.85, 127.31, 127.23, 125.67, 125.34, 120.10, 85.56, 78.91, 76.29, 75.97, 75.90, 73.69, 70.48, 68.54, 68.03, 46.78, 25.37, 15.21 ppm.

### Example A.3: Preparation of Glucose Building Block 5.05

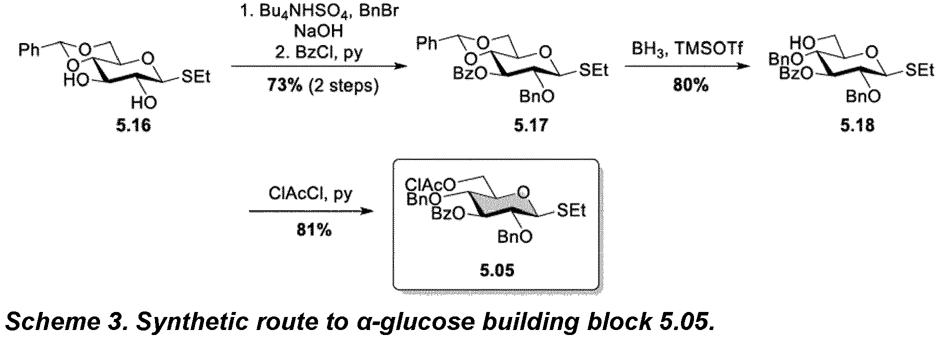

### Ethyl 2-O-benzyl-4,6-O-[(S)-phenylmethylene]-1-thio-β-D-glucopyranoside (5.16a)

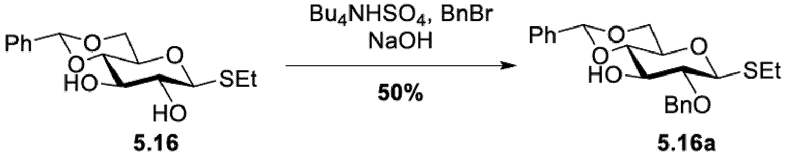

Tetrabutylammonium hydrogen sulfate (2.2 g, 6.4 mmol, 0.5 equiv.), benzyl bromide (1.68 mL, 14.1 mmol, 1.1 equiv., filtered using a short aluminium oxide column before use) and 5% aqueous NaOH (33 mL) was added to a stirred solution of ethyl 4,6-*O-*benzylidene-1-thio-β- D-glucopyranoside (4.0 g, 12.8 mmol, 1.0 equiv.) in CH₂Cl₂ (300 mL). The mixture was heated to 45 °C and was stirred for 8 h. The mixture was allowed to cool down to room temperature and the organic phase was separated from the aqueous phase. The aqueous phase was extracted with CH₂Cl₂ (2 x 100 mL) and the combined organic phase was washed with brine (100 mL), dried over Na₂SO₄, filtered and concentrated. **3-OH-galactose 5.16a** (2.6 g, 6.5 mmol, **50%**) was obtained as a colorless solid after purification by column chromatography (SiO₂, Toluene/EtOAc = 95:5 to 9:1). All spectra are in accordance with literature spectra.

**R*_{f}*** = 0.42 (Toluene/EtOAc 9:1).

**[α]_{D}** 24.1 cm⁻¹ (c 1, CHCl₃).

**IR** (film): 3066, 2962, 2884, 1761, 1722, 1602, 1453, 1406, 1335, 1266, 1248, 1199, 1135, 1061, 1027, 998, 916, 805, 753, 700 cm⁻¹.

**HRMS** (QToF): Calcd for C₃₁H₃₃ClO₇SNa [M + Na]⁺ 607.1528; found 607.1539.

**¹H NMR (400 MHz, CDCl₃):** δ 7.54 - 7.28 (m, 10H), 5.52 (s, 1H), 4.87 (dd, *J =* 68.7, 10.8 Hz, 2H), 4.57 (d, *J* = 9.8 Hz, 1H), 4.35 (dd, *J =* 10.4, 4.9 Hz, 1H), 3.89 (t, *J* = 8.8 Hz, 1H), 3.76 (t, *J* = 10.2 Hz, 1H), 3.56 (d, *J* = 9.3 Hz, 1H), 3.45 (d, *J* = 5.0 Hz, 1H), 3.38 (dd, *J* = 9.8, 8.3 Hz, 1H), 2.87 - 2.68 (m, 2H), 1.34 (t, *J* = 7.5 Hz, 3H) ppm.

### Ethyl 3-O-benzoyl-2,4-bis-O-benzyl-1-thio-β-D-glucopyranoside (5.18)

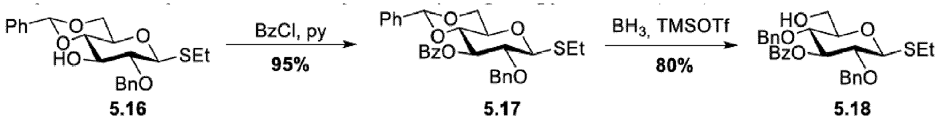

**OH-galactose 5.16a** (2.6 g, 6.5 mmol, 1.0 equiv.) was dissolved in pyridine (30 mL) and the solution was cooled to 0 °C. Benzoyl chloride (2.26 mL, 19.4 mmol, 3.0 equiv.) was added under an argon atmosphere. The reaction mixture was allowed to warm up to room temperature and was stirred for 16 h. The reaction was quenched by addition of water and the aqueous layer was extracted with ethyl acetate (3 x 100 mL). The combined organic phase was washed with 1.0 M HCl and brine, dried over Na₂SO₄, filtered and concentrated. Compound **5.17** (3.1 g, 6.1 mmol, 95%) was obtained as a colorless oil after purification by column chromatography (Hex/EtOAc = 9:1 to 3:1). **R*_{f}*** = 0.29 (Hex/EtOAc 9:1). Compound **5.17** (1.0 g, 2.0 mmol, 1.0 equiv.) was co-evaporated by anhydrous toluene and dried for 2 h at high vacuum. Then, it was dissolved in anhydrous CH₂Cl₂ (25 mL) and the mixture was stirred over activated molecular sieves (3 Å-AW) for 30 minutes at room temperature. The mixture was cooled to 0 °C and BH₃ (1 M solution in THF, 9.9 mL, 9.9 mmol, 5.0 equiv.) and TMSOTf (53 µL, 0.29 mmol, 0.15 equiv.) were added dropwise. The mixture was allowed to warm up to room temperature and stirred under argon atmosphere for 4 h. Et₃N (1 mL) was added followed by MeOH until the evolution of H₂ ceased. The mixture was concentrated and co-evaporated with MeOH (3 x 30 mL). **6-OH-sugar 5.18** (805 mg, 1.6 mmol, 80%) was obtained as a white solid after purification by column chromatography (Hex:AcOEt 3:1).

**R*_{f}*** = 0.18 (Hex: AcOEt 3:1).

**HRMS (QToF):** Calcd for C₂₉H₃₂O₆SNa [M + Na]⁺ 531.1812; found 531.1830.

**¹H NMR (600 MHz, CDCl₃):** δ 8.02 - 7.97 (m, 2H), 7.61 - 7.55 (m, 1H), 7.48 - 7.41 (m, 2H), 7.20 - 7.07 (m, 10H), 5.58 (t, *J* = 9.2 Hz, 1H), 4.79 (d, *J* = 10.7 Hz, 1H), 4.62 (d, *J* = 9.7 Hz, 1H), 4.55 (s, 2H), 4.54 (d, *J* = 10.7 Hz, 1H), 3.92 (dd, *J* = 12.2, 2.6 Hz, 1H), 3.80 - 3.72 (m, 2H), 3.54 - 3.47 (m, 2H), 2.84 - 2.73 (m, 2H), 1.33 (t, *J* = 7.5 Hz, 3H) ppm.

**¹³C NMR (151 MHz, CDCl₃):** δ 165.6, 137.4, 133.3, 130.1, 129.9, 128.6, 128.5, 128.4, 128.1, 127.9, 85.4, 79.5, 79.2, 78.0, 75.7, 75.1, 74.8, 62.0, 25.6, 15.3 ppm.

### Ethyl 3-O-benzoyl-2,4-bis-O-benzyl-6-O-(2-chloroacetyl)-1-thio-β-D-glucopyranoside (5.05)

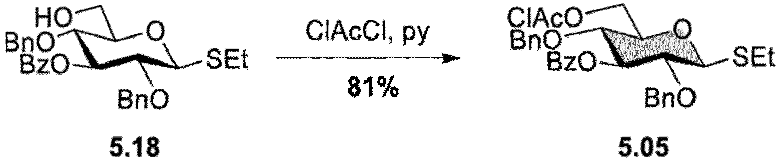

To a solution of **6-OH-sugar 5.18** (1.5 g, 2.9 mmol, 1.0 equiv.) and anhydrous pyridine (1.19 mL, 14.8 mmol, 5.0 equiv.) in anhydrous CH₂Cl₂ (20 mL) at -20 °C was added chloroacetyl chloride (0.47 mL, 5.9 mmol, 2.0 equiv.). The reaction was stirred for 10 min, aqueous citric acid solution (2 mL) was added and the mixture was allowed to warm up to room temperature. Water was added and the aqueous phase was extracted with CH₂Cl₂ (3 x 50 mL). The combined organic phase was dried over Na₂SO₄, filtered and concentrated. The **title compound 5.05** (1.4 g, 2.4 mmol, 81%) was obtained as a light-yellow solid after purification by column chromatography (Hex:AcOEt 9:1 to 3:1).

**R*_{f}*** = 0.44 (Hex: AcOEt 3:1).

**[a]D** 24.1 cm⁻¹ (c 1, CHCl₃).

**IR (film):** 3066, 2962, 2884, 1761, 1722, 1602, 1453, 1406, 1335, 1266, 1248, 1199, 1135, 1061, 1027, 998, 916, 805, 753, 700 cm⁻¹.

**HRMS (QToF):** Calcd for C₃₁H₃₃ClO₇SNa [M + Na]⁺ 607.1528; found 607.1539.

**¹H NMR (600 MHz, CDCl₃):** δ 8.05 - 8.00 (m, 2H), 7.62 - 7.56 (m, 1H), 7.46 (t, *J* = 7.8 Hz, 2H), 7.20 (dd, *J* = 4.9, 1.9 Hz, 3H), 7.12 (dq, *J* = 5.5, 3.0 Hz, 7H), 5.59 (t, *J* = 8.8 Hz, 1H), 4.79 (d, *J* = 10.7 Hz, 1H), 4.64 - 4.51 (m, 3H), 4.48 - 4.43 (m, 2H), 4.29 (dd, *J* = 11.9, 4.6 Hz, 1H), 4.03 (q, *J* = 14.8 Hz, 2H), 3.68 (t, *J* = 6.4 Hz, 2H), 3.53 (t, *J* = 9.4 Hz, 1H), 2.77 (dtt, *J* = 20.1, 12.6, 7.4 Hz, 2H), 1.33 (t, *J* = 7.4 Hz, 3H) ppm.

**¹³C NMR (151 MHz, CDCl₃):** δ 167.1, 165.6, 137.3, 137.0, 133.4, 129.9, 128.7, 128.6, 128.5, 128.5, 128.4, 128.3, 127.9, 85.4, 79.3, 78.1, 76.5, 75.7, 75.1, 74.7, 64.8, 40.9, 25.6, 15.2 ppm.

### Example A.4: Automated Glycan Assembly of P. gingivalis LPS fragments

### 5-Amino-pentyl α-D-glucopyranosyl-(1→4)-α-L-rhamnopyranosyl-(1→3)-2-acetamido-2- deoxy-β-D-galactopyranosyl-(1→3)-α-D-galactopyranoside (3)

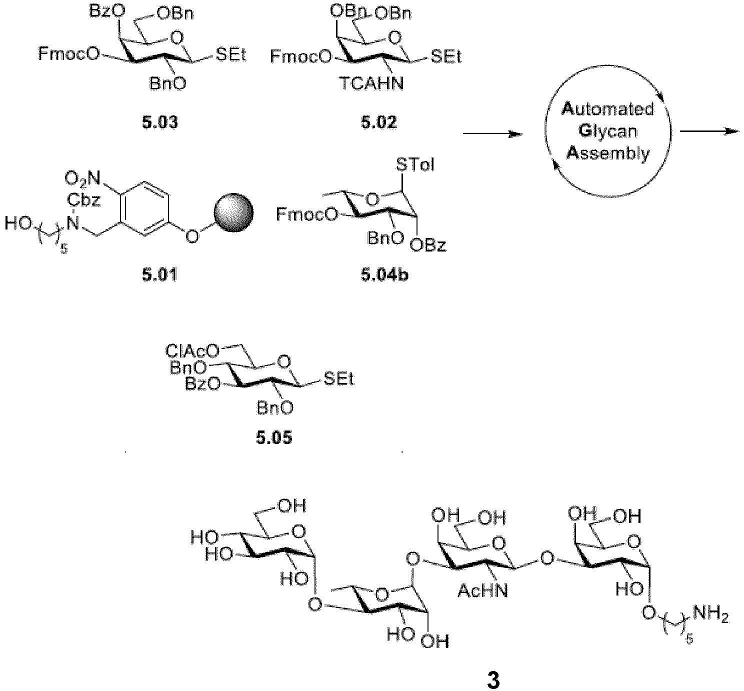

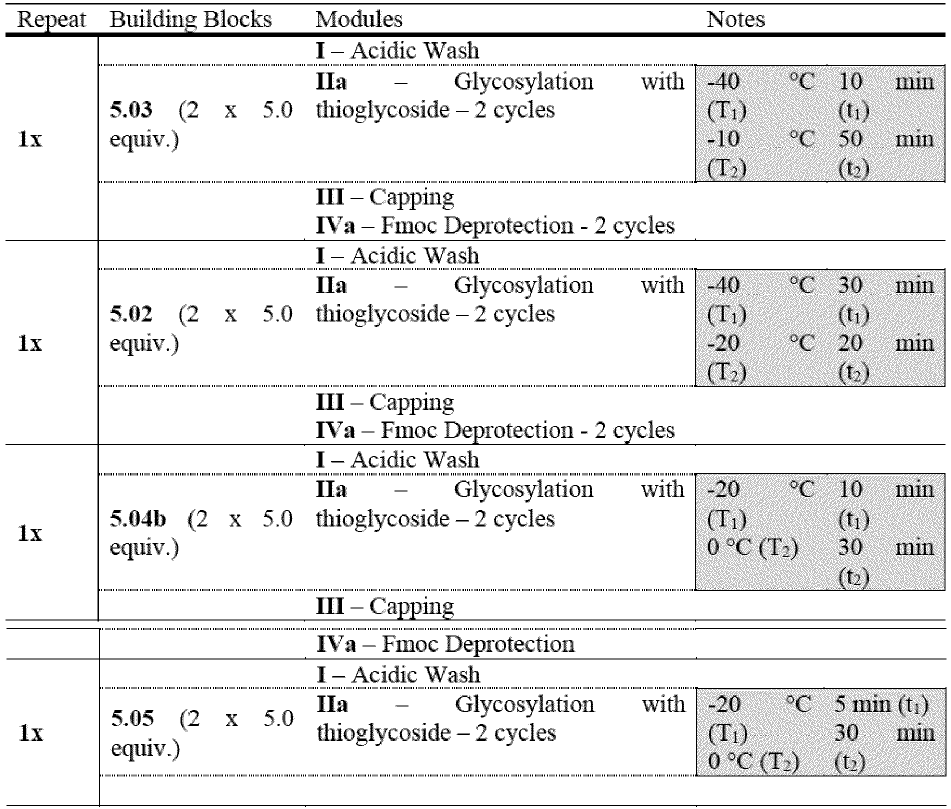

Protected **3** (22 mg, 0.011 mmol, crude yield: 81%) was obtained as a colorless oil after photocleavage from solid support following **Method A-1.** Deprotection of **3** following **Method C** and **D** and purification by reverse-phase HPLC (**Method E-1,** t_{R} = 18.8 min) afforded deprotected compound **3** (1.1 mg, 0.002 mmol, 11%) as a white solid after lyophylization.

**¹H NMR (700 MHz, D₂O):** δ 4.97 - 4.92 (m, 1H), 4.85 - 4.80 (m, 1H), 4.79 (s, 1H), 4.63 (d, J = 8.2 Hz, 1H), 4.10 (s, 1H), 4.01 - 3.55 (m, 19H), 3.49 - 3.42 (m, 2H), 3.43 - 3.35 (m, 2H), 2.94 - 2.89 (m, 2H), 1.95 (s, 3H), 1.64 - 1.57 (m, 4H), 1.40 - 1.34 (m, 2H), 1.27 (d, J = 6.2 Hz, 3H) ppm.

**¹³C NMR (176 MHz, D₂O):** δ 174.9, 102.5, 102.0, 99.7, 98.4, 81.0, 79.0, 75.0, 72.7, 70.7, 70.5, 69.3, 68.7, 68.4, 67.8, 67.3, 61.2, 60.9, 60.1, 51.8, 39.4, 28.1, 26.6, 22.4, 22.2, 16.9 ppm.

**HRMS (QToF):** Calcd for C₃₁H₅₇N₂O₂₀ [M + H]⁺ 777.3499; found 777.3508.

### 5-Aminopentyl-α-D-galactopyranosyl-(1→6)-α-D-glucopyranosyl-(1→4)-α-L-rhamnopyranosyl-(1→3)-2-acetamido-2-deoxy-B-D-galactopyranoside (1)

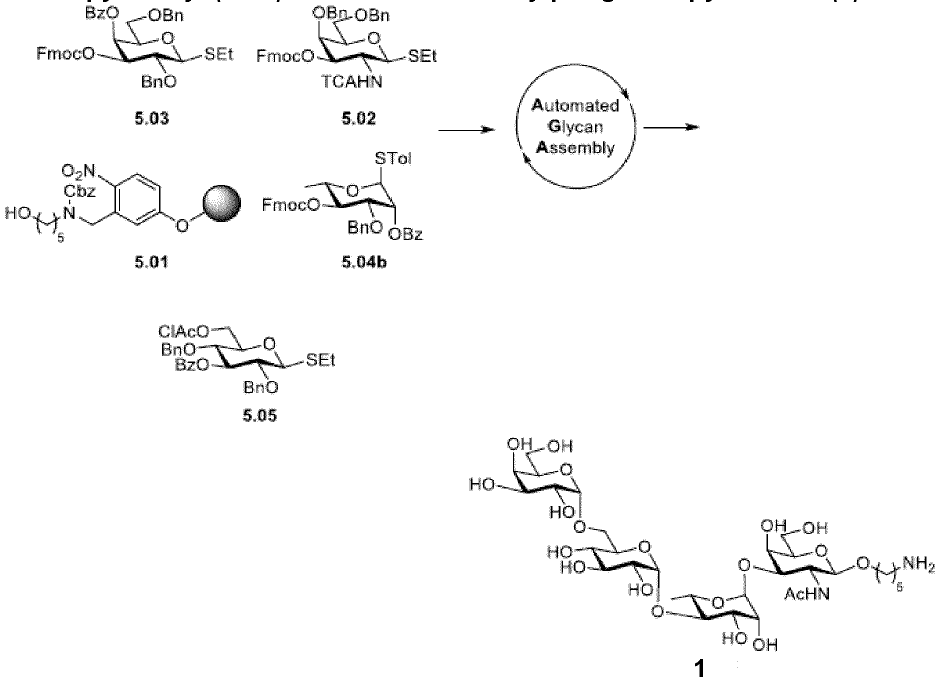

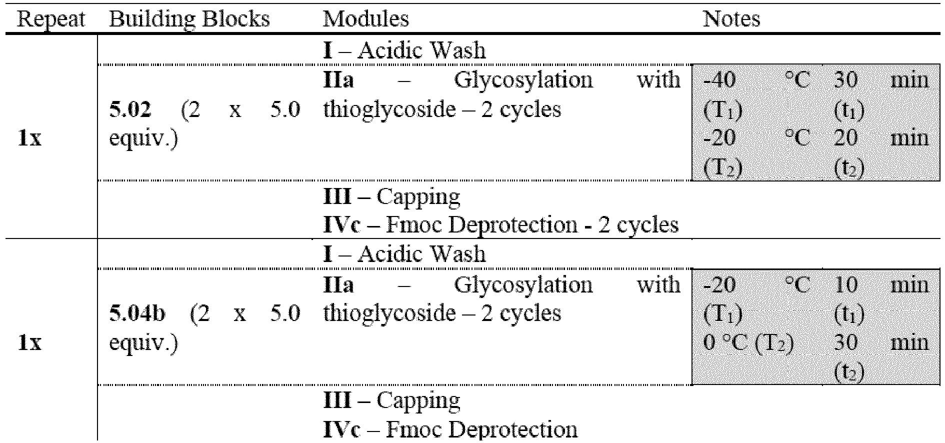

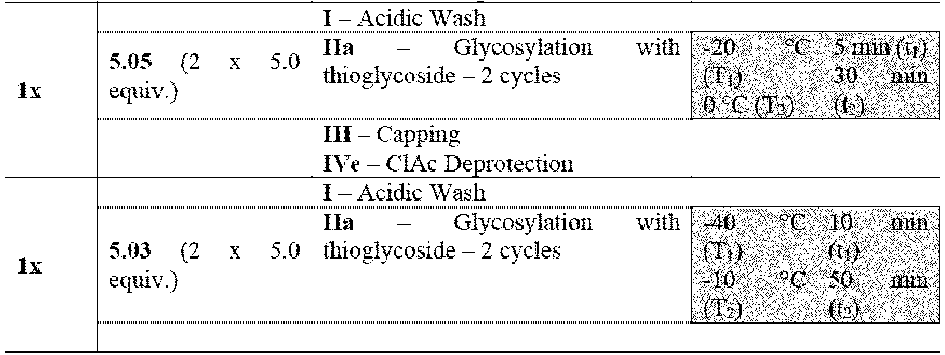

Protected **1** (26 mg, 0.012 mmol, crude yield: 86%) was obtained as a colorless oil after photocleavage from solid support following **Method A-1.** Deprotection of **1** following **Method C** and **D** and purification by reverse-phase HPLC **(Method E-1,** t_{R} = 18.9 min) afforded deprotected compound **1** (1.5 mg, 0.002 mmol, 15%) as a white solid after lyophylization.

**¹H NMR (700 MHz, D₂O):** δ 5.00 (d, J = 4.0 Hz, 1H), 4.94 (d, J = 3.8 Hz, 1H), 4.83 (s, 1H), 4.47 (d, J = 8.6 Hz, 1H), 4.19 - 4.15 (m, 1H), 3.99 - 3.40 (m, 23H), 2.97 - 2.92 (m, 2H), 2.00 (s, 3H), 1.67 - 1.60 (m, 2H), 1.59 - 1.54 (m, 2H), 1.40 - 1.33 (m, 2H), 1.31 (d, J = 6.3 Hz, 3H) ppm.

**¹³C NMR (176 MHz, D₂O):** δ 174.4, 102.0, 101.0, 99.7, 98.1, 80.9, 78.9, 75.0, 72.8, 71.4, 70.9, 70.6, 70.4, 69.9, 69.4, 69.1, 68.7, 68.4, 68.3, 67.5, 65.4, 61.0, 60.8, 51.4, 39.2, 28.0, 26.3, 22.1, 22.0, 16.7 ppm.

**HRMS (QToF):** Calcd for C₃₁H₅₇N₂O₂₀ [M + H]⁺ 777.3499; found 777.3489.

### 5-Aminopentyl-2-acetamido-2-deoxy-β-D-galactopyranosyl-(1→3)-α-D-galactopyranosyl-(1→6)-α-D-glucopyranosyl-(1→4)-α-L-rhamnopyranoside (4)

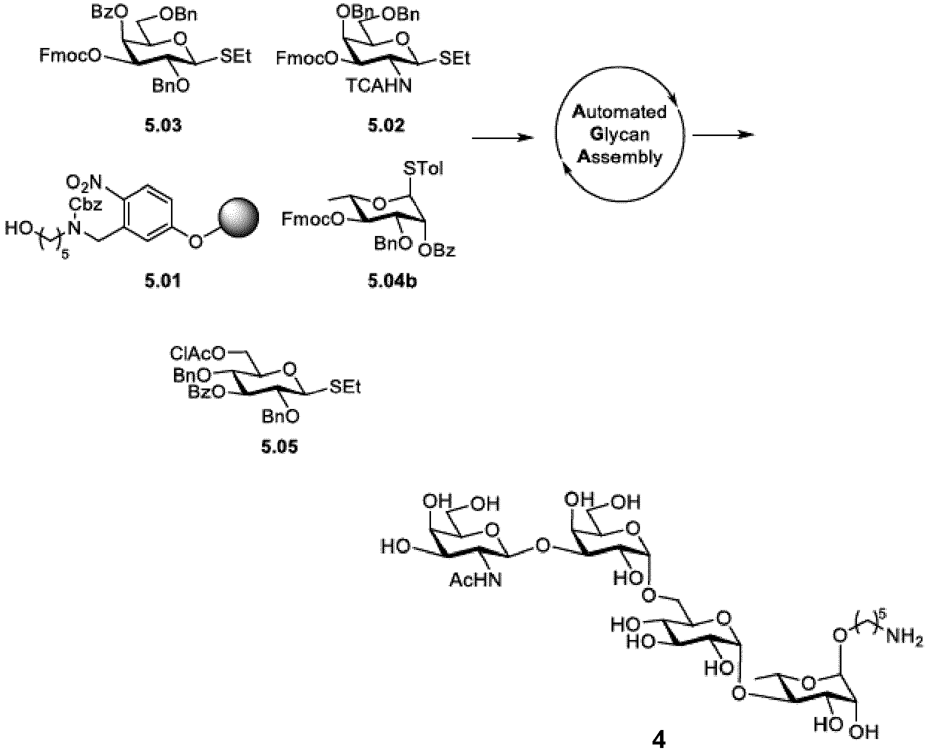

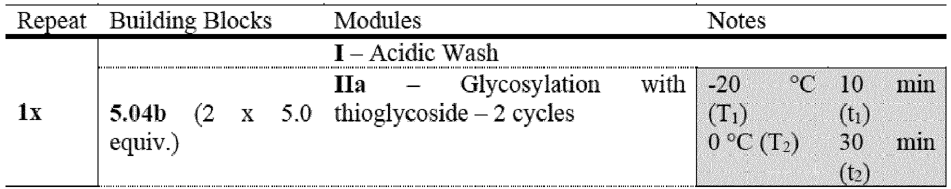

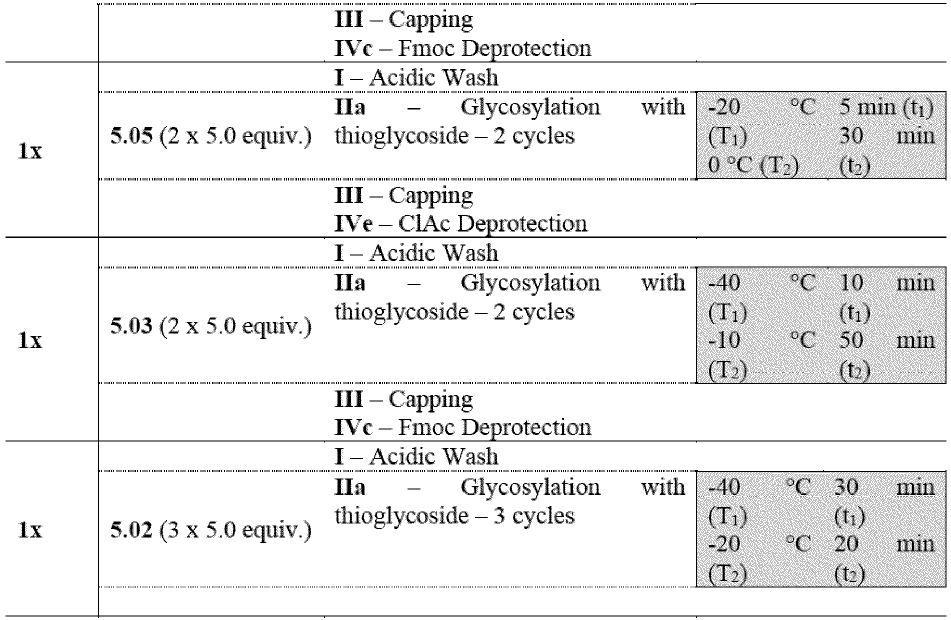

Protected **4** (27 mg, 0.012 mmol, crude yield: 92%) was obtained as a colorless oil after photocleavage from solid support following **Method A-1.** Deprotection of **4** following **Method C** and **D** and purification by reverse-phase HPLC **(Method E-1,** t_{R} = 19.4 min) afforded deprotected compound **4** (1.8 mg, 0.002 mmol, 18%) as a white solid after lyophylization.

**¹H NMR (700 MHz, D₂O):** δ 5.01 (d, J = 3.9 Hz, 1H), 4.91 (d, J = 3.9 Hz, 1H), 4.76 - 4.74 (m, 1H), 4.60 (d, J = 8.5 Hz, 1H), 4.19 - 4.14 (m, 2H), 3.98 (dd, J = 11.3, 3.5 Hz, 1H), 3.94 - 3.60 (m, 18H), 3.57 - 3.48 (m, 3H), 3.44 (t, J = 9.4 Hz, 1H), 2.96 (t, J = 7.7 Hz, 2H), 1.99 (s, 2H), 1.68 - 1.56 (m, 4H), 1.41 (d, J = 7.9 Hz, 2H), 1.33 (d, J = 6.3 Hz, 3H) ppm.

**¹³C NMR (176 MHz, D₂O):** δ 175.1, 103.2, 99.7, 99.4, 98.1, 81.1, 79.1, 74.9, 72.9, 71.3, 70.7, 70.5, 70.4, 69.2, 69.1, 69.0, 67.6, 67.5, 67.4, 65.1, 60.9, 52.6, 39.2, 28.0, 26.5, 22.3, 22.2, 16.6 ppm.

**HRMS (QToF):** Calcd for C₃₁H₅₇N₂O₂₀ [M + H]⁺ 777.3499; found 777.3501.

### 5-Aminopentyl-α-L-rhamnopyranosyl-(1→3)-2-acetamido-2-deoxy-β-D-galactopyranosyl-(1→3)-α-D-galactopyranosyl-(1→6)-α-D-glucopyranoside (5)

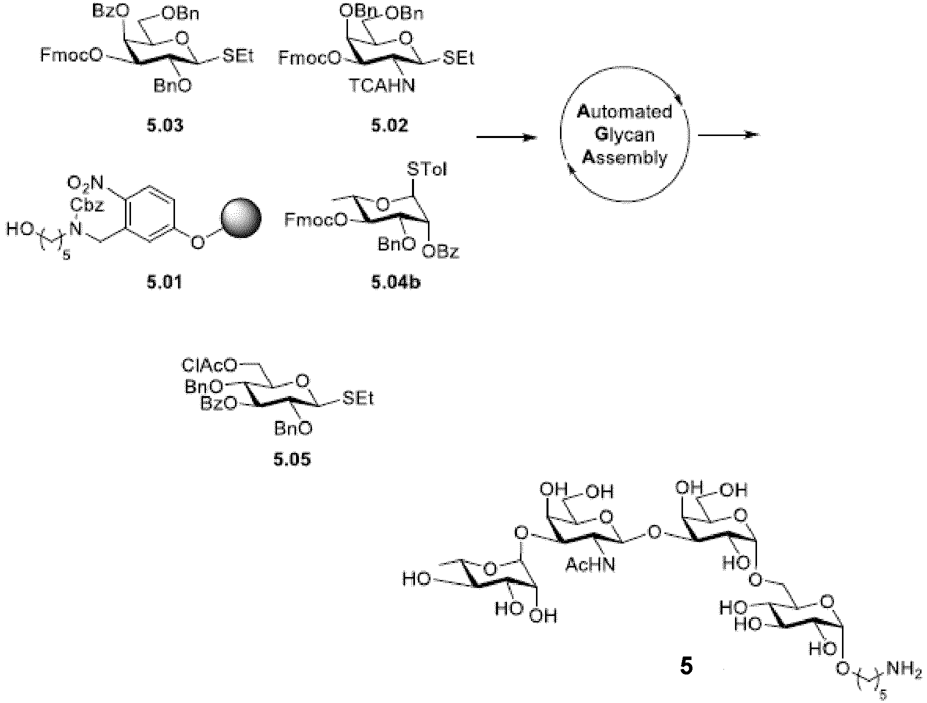

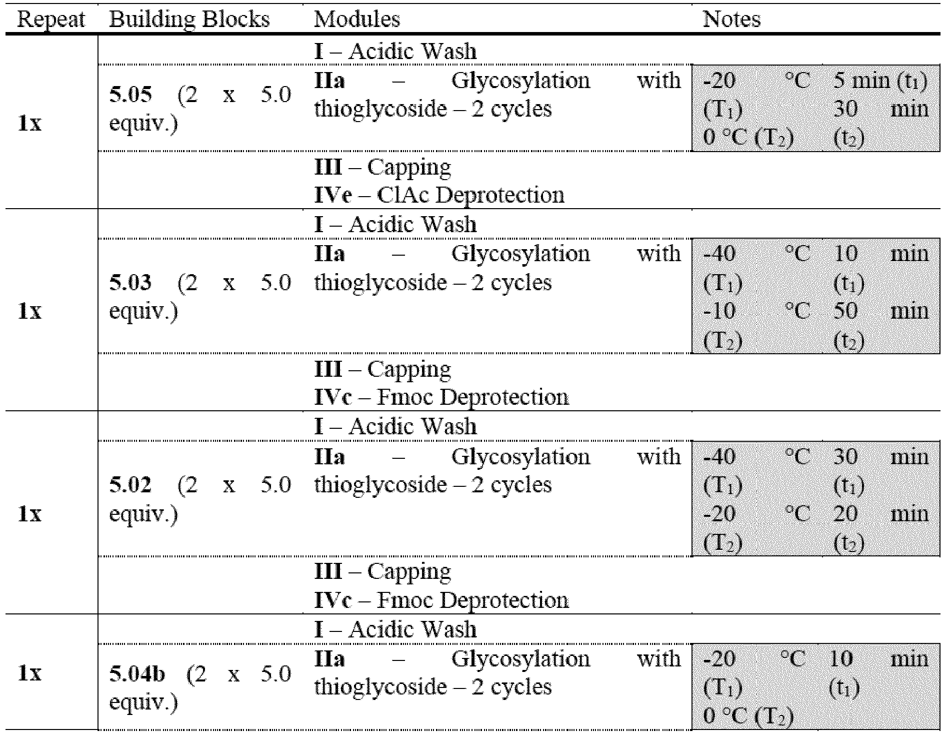

Protected **5** (17 mg, 0.012 mmol, crude yield: 57%) was obtained as a colorless oil after photocleavage from solid support following **Method A-1.** Deprotection of **5** following **Method C** and **D** and purification by reverse-phase HPLC **(Method E-1,** t_{R} = 19.4 min) afforded deprotected compound **5** (0.8 mg, 0.001 mmol, 8%) as a white solid after lyophylization.

**¹H NMR (700 MHz, D₂O):** δ 4.88 (d, *J* = 3.8 Hz, 1H), 4.86 (d, *J* = 4.1 Hz, 1H), 4.64 (d, *J* = 8.5 Hz, 1H), 4.15 (s, 1H), 4.02 - 3.83 (m, 6H), 3.82 - 3.59 (m, 14H), 3.52 - 3.44 (m, 3H), 3.36 (t, *J* = 9.7 Hz, 1H), 2.93 (t, *J* = 7.6 Hz, 2H), 1.97 (s, 3H), 1.63 (p, *J* = 7.4 Hz, 4H), 1.40 (dt, *J* = 16.0, 8.4 Hz, 2H), 1.20 (d, *J* = 6.3 Hz, 3H) ppm.

**¹³C NMR (176 MHz, D₂O):** δ 174.8, 102.6, 102.3, 98.2, 98.1, 73.3, 70.0, 69.1, 67.9, 60.9, 51.8, 46.5, 39.4, 38.7, 28.0, 26.6, 22.5, 16.6 ppm.

**HRMS (QToF):** Calcd for C₃₁H₅₇N₂O₂₀ [M + H]⁺ 777.3499; found 777.3502.

### 5-Aminopentyl α-D-galactopyranoside (11)

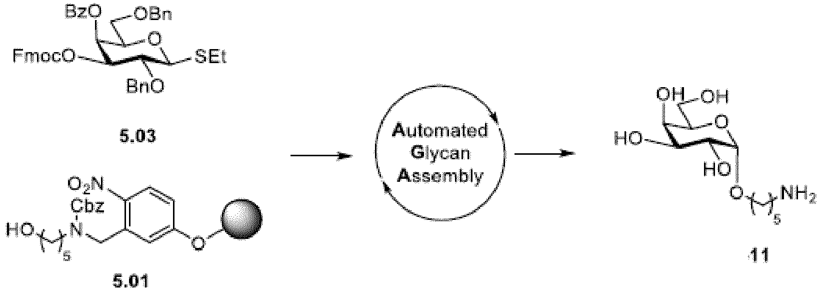

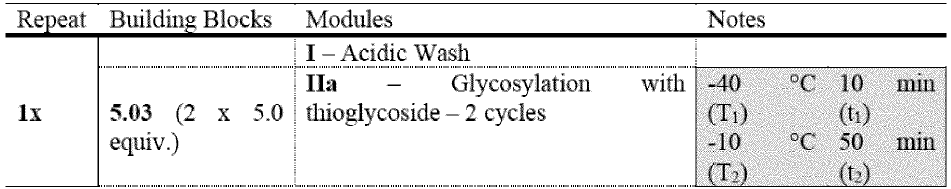

Protected **11** (8 mg, 0.012 mmol, crude yield: 87%) was obtained as a colorless oil after photocleavage from solid support following **Method A-1.** Deprotection of **11** following **Method** C and **D** and purification by reverse-phase HPLC **(Method E-1,** t_{R} = 13.5 min) afforded deprotected compound **11** (1.2 mg, 0.004 mmol, 18%) as a white solid after lyophylization.

**¹H NMR (700 MHz, D₂O):** δ 4.95 (d, *J* = 3.4 Hz, 1H), 3.98 (d, *J* = 3.2 Hz, 1H), 3.93 (t, *J* = 6.2 Hz, 1H), 3.87 - 3.81 (m, 2H), 3.78 - 3.72 (m, 3H), 3.54 (dt, *J* = 10.0, 6.2 Hz, 1H), 3.01 (t, *J* = 7.6 Hz, 2H), 1.75 - 1.62 (m, 4H), 1.53 - 1.42 (m, 2H) ppm.

**¹³C NMR (176 MHz, D₂O):** δ 98.2, 70.9, 69.5, 69.3, 68.3, 67.8, 61.3, 39.4, 28.0, 26.5, 22.4 ppm.

**HRMS (QToF):** Calcd for C₁₁H₂₄NO₆ [M + H]⁺ 266.1598; found 266.1726.

### 5-Amino-pentyl α-D-glucopyranoside (8)

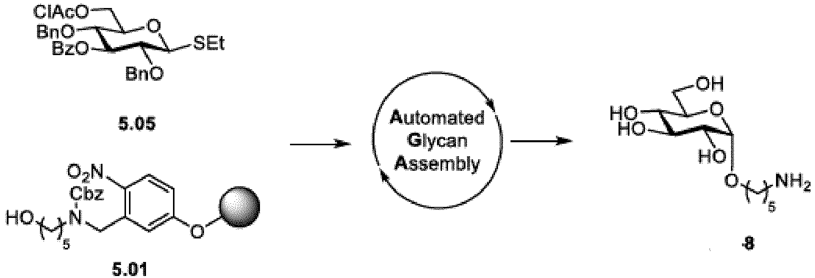

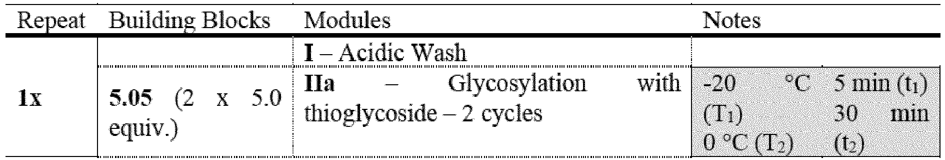

Protected **8** (5 mg, 0.006 mmol, crude yield: 46%) was obtained as a colorless oil after photocleavage from solid support following **Method A-1.** Deprotection of **8** following **Method C** and **D** and purification by reverse-phase HPLC **(Method E-1,** t_{R} = 12.8 min) afforded deprotected compound **8** (0.9 mg, 0.003 mmol, 25%) as a white solid after lyophylization.

**¹H NMR (700 MHz, D₂O):** δ 4.92 (d, *J* = 3.8 Hz, 1H), 3.86 (dd, *J* = 12.3, 2.4 Hz, 1H), 3.78 - 3.65 (m, 4H), 3.59 - 3.52 (m, 2H), 3.41 (dd, *J* = 10.1, 9.1 Hz, 1H), 3.02 (t, *J* = 7.6 Hz, 2H), 1.72 - 1.63 (m, 4H), 1.54 - 1.41 (m, 2H) ppm.

**¹³C NMR (176 MHz, D₂O):** δ 97.9, 73.0, 71.7, 71.1, 69.5, 67.6, 60.5, 39.2, 28.0, 26.4, 22.4 ppm.

**HRMS (QToF):** Calcd for C₁₁H₂₄NO₆ [M + H]⁺ 266.1598; found 266.1750.

### 5-Aminopentyl-2-acetamido-2-deoxy-β-D-galactopyranosyl-(1→3)-α-D-galactopyranoside (6)

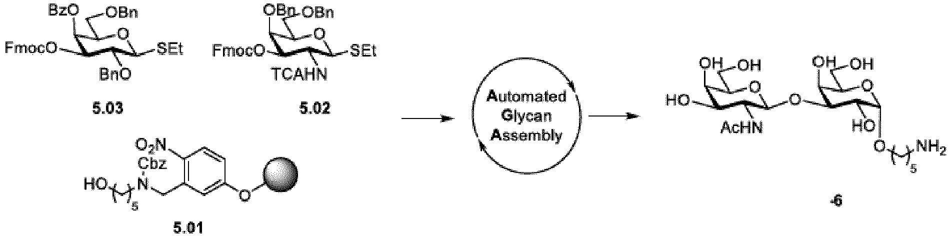

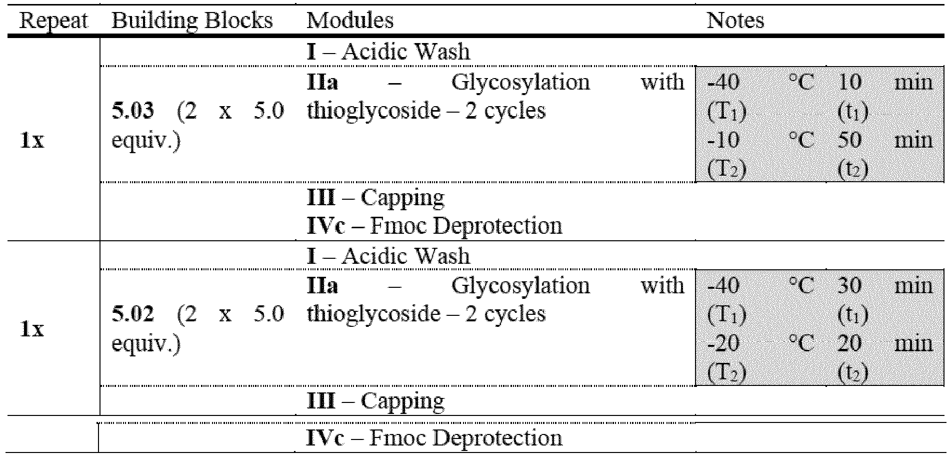

Protected **6** (11 mg, 0.009 mmol, crude yield: 67%) was obtained as a colorless oil after photocleavage from solid support following **Method A-1.** Deprotection of 6 following **Method C** and **D** and purification by reverse-phase HPLC **(Method E-1,** t_{R} = 15.6 min) afforded deprotected compound **6** (1.1 mg, 0.002 mmol, 17%) as a white solid after lyophylization.

**¹H NMR (700 MHz, D₂O):** δ 4.92 (d, *J* = 3.8 Hz, 1H), 4.65 (d, *J* = 8.4 Hz, 1H), 4.20 (d, *J* = 3.2 Hz, 1H), 3.97 - 3.86 (m, 5H), 3.83 - 3.71 (m, 6H), 3.70 - 3.65 (m, 1H), 3.54 (dt, *J* = 9.9, 6.2 Hz, 1H), 3.01 (t, *J* = 7.6 Hz, 2H), 2.04 (s, 3H), 1.74 - 1.63 (m, 4H), 1.53 - 1.42 (m, 2H) ppm.

**¹³C NMR (176 MHz, D₂O):** δ 175.2, 103.1, 98.4, 79.0, 75.0, 70.8, 70.5, 69.3, 67.8, 67.3, 61.2, 61.0, 52.7, 39.4, 28.1, 26.5, 22.4, 22.3 ppm.

**HRMS (QToF):** Calcd for C₁₉H₃₇N₂O₁₁ [M + H]⁺ 469.2392; found 469.2402.

### 5-Aminopentyl-α-L-rhamnopyranosyl-(1→3)-2-acetamido-2-deoxy-β-D-galactopyranosyl-(1→3)-α-D-galactopyranoside (2)

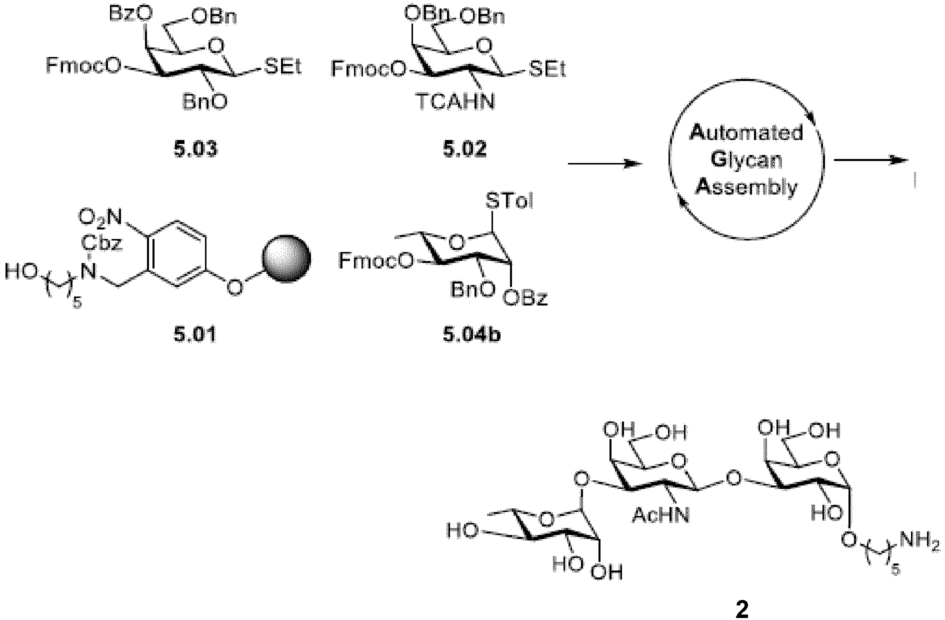

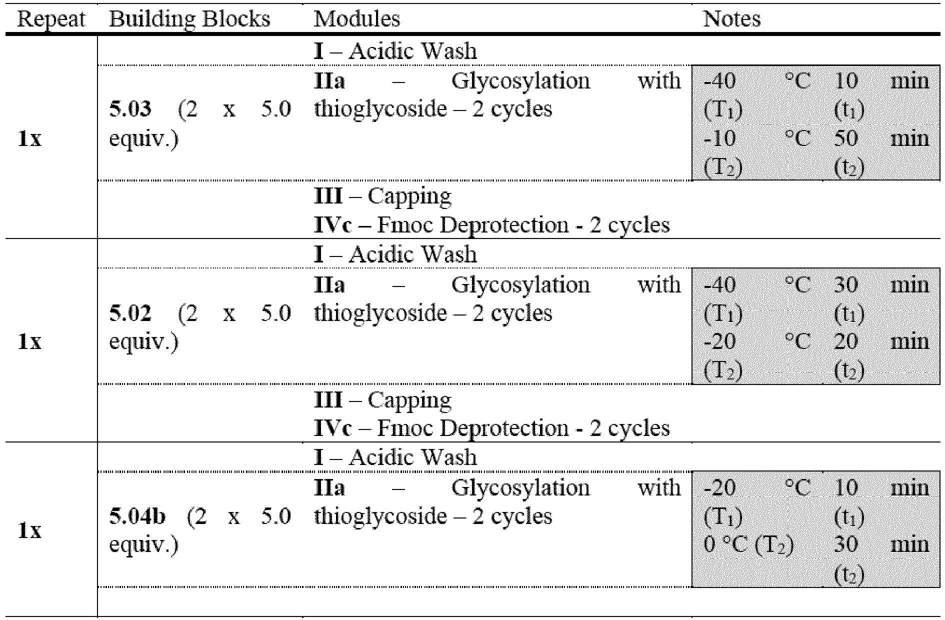

Protected **2** (16 mg, 0.011 mmol, crude yield: 79%) was obtained as a colorless oil after photocleavage from solid support following **Method A-1.** Deprotection of **2** following **Method C** and **D** and purification by reverse-phase HPLC **(Method E-1,** t_{R} = 17.4 min) afforded deprotected compound **2** (1.2 mg, 0.002 mmol, 15%) as a white solid after lyophylization.

**¹H NMR (700 MHz, D₂O):** δ 4.83 (d, *J* = 3.9 Hz, 1H), 4.80 (s, 1H), 4.65 (d, *J* = 8.5 Hz, 1H), 4.11 (d, *J* = 3.4 Hz, 1H), 3.96 (dd, *J* = 11.0, 8.5 Hz, 1H), 3.89 - 3.56 (m, 14H), 3.46 (dt, *J* = 10.1, 6.3 Hz, 1H), 3.36 (t, *J* = 9.7 Hz, 1H), 2.93 (t, *J* = 7.6 Hz, 2H), 1.97 (s, 3H), 1.61 (dt, J = 12.3, 6.1 Hz, 4H), 1.38 (q, *J* = 8.7 Hz, 2H), 1.20 (d, *J* = 6.2 Hz, 3H) ppm.

**¹³C NMR (176 MHz, D₂O):** δ 174.9, 102.5, 102.3, 98.4, 79.0, 78.6, 75.0, 71.9, 70.5, 70.4, 70.0, 69.3, 67.8, 67.6, 67.3, 61.2, 60.9, 51.8, 39.4, 28.1, 26.5, 22.4, 22.2, 16.6 ppm.

**HRMS (QToF):** Calcd for C₂₅H₄₇N₂O₁₅ [M + H]⁺ 615.2971; found 615.2974.

### 5-Aminopentyl-β-D-galactopyranosyl-(1→3)-α-D-galactopyranosyl-(1→6)-α-D-glucopyranoside (7)

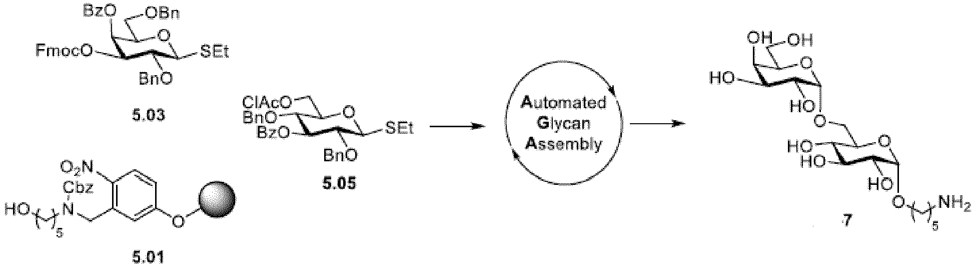

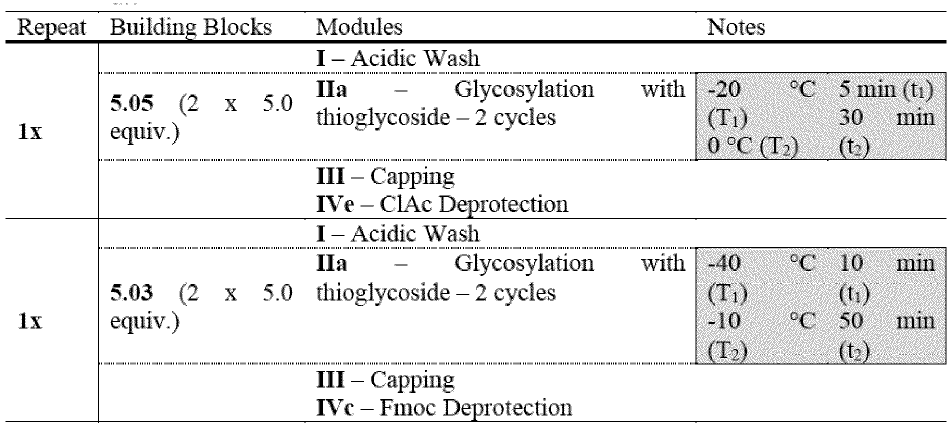

Protected **7** (12 mg, 0.010 mmol, crude yield: 76%) was obtained as a colorless oil after photocleavage from solid support following **Method A-1.** Deprotection of **7** following **Method C** and **D** and purification by reverse-phase HPLC **(Method E-1,** t_{R} = 17.4 min) afforded deprotected compound **7** (0.56 mg, 0.013 mmol, 10%) as a white solid after lyophylization.

**¹H NMR (700 MHz, D₂O):** δ 4.93 (d, *J* = 3.7 Hz, 1H), 4.88 (d, *J* = 3.8 Hz, 1H), 3.96 - 3.91 (m, 3H), 3.85 - 3.76 (m, 3H), 3.73 - 3.66 (m, 4H), 3.64 (t, *J* = 9.4 Hz, 1H), 3.55 - 3.44 (m, 3H), 2.96 (t, *J* = 7.6 Hz, 2H), 1.69 - 1.60 (m, 4H), 1.48 - 1.36 (m, 2H).

**¹³C NMR (176 MHz, D₂O):** δ 98.0, 97.9, 73.3, 71.1, 70.9, 70.2, 69.3, 69.1, 68.3, 67.8, 65.5, 61.0, 39.3, 28.0, 26.5, 22.4.

**HRMS (QToF):** Calcd for C₁₇H₃₄NO₁₁ [M + H]⁺ 428.2126; found 428.2142.

### 5-Aminopentyl-a-(1→2)-L-trirhamnopyranoside (5.06)

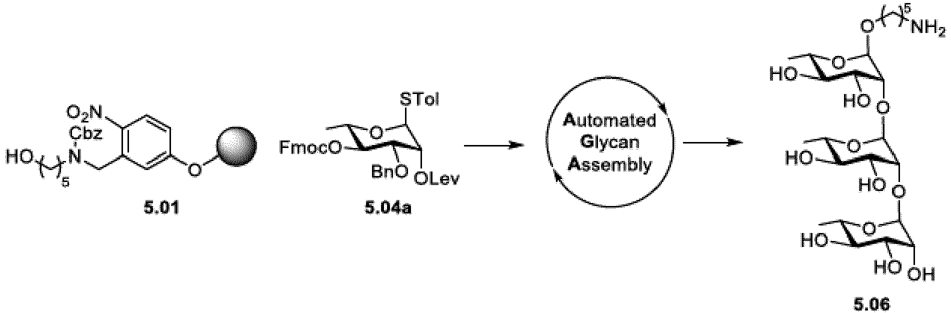

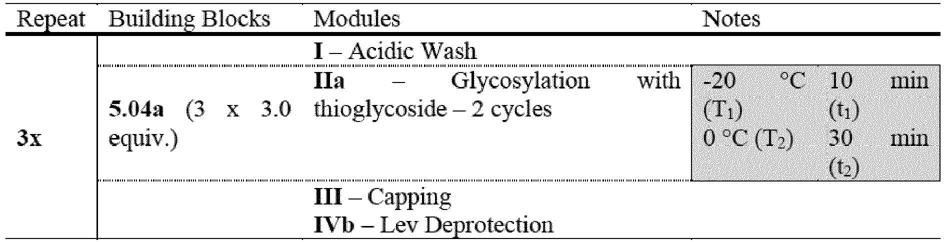

Protected **5.06** (35 mg, 0.022 mmol, crude yield: 80%) was obtained as a colorless oil after photocleavage from solid support following **Method A-1.** Deprotection of **5.06** following **Method C** and **D** and purification by reverse-phase HPLC **(Method E-1,** t_{R} = 17.4 min) afforded deprotected compound **5.06** (6 mg, 0.011 mmol, 41%) as a white solid after lyophylization.

**¹H NMR (700 MHz, D₂O):** δ 5.12 (d, *J* = 1.8 Hz, 1H), 4.98 (d, *J* = 1.8 Hz, 1H), 4.88 (d, *J* = 1.7 Hz, 1H), 4.09 (ddd, *J* = 11.5, 3.4, 1.7 Hz, 2H), 3.95 - 3.88 (m, 2H), 3.86 (dd, J = 9.8, 3.4 Hz, 1H), 3.82 - 3.67 (m, 5H), 3.56 (dt, *J* = 10.0, 6.1 Hz, 1H), 3.53 - 3.40 (m, 3H), 3.01 (t, *J* = 7.6 Hz, 2H), 1.76 - 1.61 (m, 4H), 1.53 - 1.41 (m, 2H), 1.31 (d, *J* = 6.3 Hz, 3H), 1.30 (d, *J* = 6.2 Hz, 3H), 1.28 (d, *J* = 6.2 Hz, 3H) ppm.

**¹³C NMR (176 MHz, D₂O):** δ 102.2, 100.9, 98.4, 72.1, 72.1, 72.0, 70.2, 70.1, 70.0, 69.8, 69.3, 69.1, 68.8, 67.7, 39.4, 28.0, 26.6, 22.4, 16.6 ppm.

**HRMS (QToF):** Calcd for C₂₃H₄₄NO₁₃ [M + H]⁺ 542.2807; found 542.2811.

### Example A.5: Synthesis and characterization of conjugates

### General procedure synthesis

The saccharide (**1**: 0.7 mg; **2**: 0.8 mg) was dissolved in 200 µL DMSO, 25 µL Pyridine and 10 µL Triethylamine were added. 6.5 equivalent of (4-nitrophenyl) adipate linker in DMSO were added to the saccharide solution. After 3 h of stirring, the reaction mixture was frozen in liquid nitrogen and subsequently lyophilized to give a crude white solid. The residue was washed with chloroform (5x 100 µL) and DCM (3x 1 µL) to remove excess of bis (4- nitrophenyl) adipate. It was checked by TLC that all excess linker was washed away and no glycan dissolved. 350 µL of water were added to an 10k Amicon filter and centrifuged at 10000 rpm for 8 min to wet the filter. Then CRM₁₉₇ was transferred into the Amicon 10k filter (180 µL, 1 mg) and the tube was washed with 300 µL of water and also added to the Amicon filter. After centrifugation at 10000 rpm for 8 min the CRM₁₉₇ vial was washed with 350 µL water and transferred to the filter. After centrifugation the CRM₁₉₇ vial was washed with 350 µL of 0.1 M phosphate buffer pH 8. The filter was removed after centrifugation, turned upside down into a clean vial and centrifuged for 2 min at 1,000 rpm, giving a colorless filtrate containing CRM₁₉₇. The filtrate (~70 µL) was transferred into the vial containing the sugar PNP ester and the tube was washed with phosphate buffer pH 8 (2 x 30 µL) and added to reaction solution. The reaction was stirred slowly for 24 h. The solution was transferred onto an Amicon 10K filter and the reaction vial was washed with 300 µL of the reaction buffer. The solution was washed three times with 400 µL water. The solution was then washed with 400 µL PBS. Finally, the filter was putted upside down into a clean vial and centrifuged at 1,000 rpm for 2 min. The filtrate was diluted with 350 µL PBS and stored at -20°C.

### Characterization of glycoconjugates1-CRM₁₉₇ and 2-CRM₁₉₇

The glycoconjugates were characterized by polyacrylamide gel electrophoresis and MALDI- TOF-MS. An alkaline separating gel (375 mM Tris/HCI pH 8.8, 12% (w/v) of a 29:1 acrylamide/N,N'- methylenebisacrylamide mixture) and an acidic stacking gel (100 mM Tris/HCl pH 6.8, 4.5% (w/v) of a 29:1 acrylamide/N,N'-methylenebisacrylamide mixture), polymerized by the addition of TEMED and 10% (w/v) ammonium peroxodisulfate, were used. 1 µg of glycoconjugate and CRM197 samples were dissolved in SDS-PAGE loading buffer (250 mM Tris (pH 6.8), 5 % (v/v) beta-mercaptoethanol, 10 % (w/v) SDS, 0.4% bromophenol blue, 50 % (v/v) glycerol) and were loaded. 4µL of PageRuler Plus Prestained Protein Ladder 10 to 250 kDa (Thermo Scientific) was used. The samples were run at 80 V and 25mA for 15 min and then at 120 V and 25 mA for 90 min and stained with 0.5 % (w/v) Coomassie Brilliant Blue R-250 for 30 min.

Mass spectra were acquired with an Autoflex Speed MALDI-TOF system (Bruker Daltonics; Bremen, Germany). Samples were spotted using the dried droplet technique with 2,5- dihydroxyacetophenone (DHAP) as matrix on MTP 384 ground steel target plates (Bruker Daltonics). The mass spectrometer was operated in linear positive mode. Mass spectra were acquired over an m/z range from 30,000 to 210,000 and data was analyzed with the FlexAnalysis software provided with the instrument.

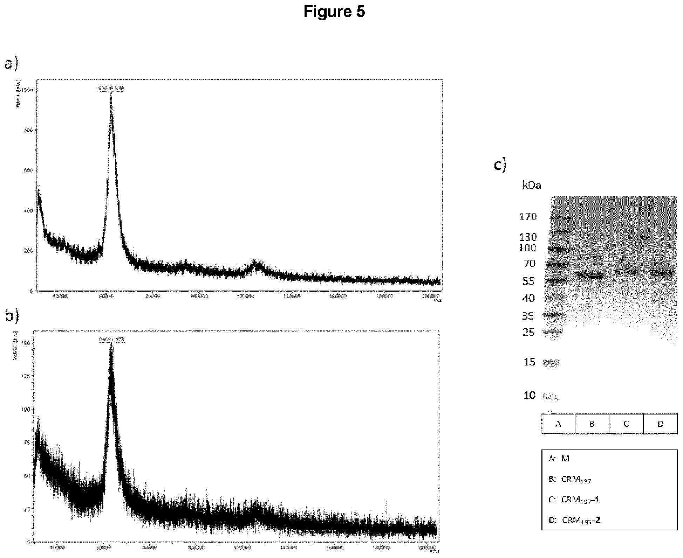

Conjugation was checked by MALDI-TOF-MS and SDS-PAGE (Figure 5). The results showed that **1** and **2** were successfully conjugated to CRM₁₉₇. The average loading was 4.5 **1** units and 7.7 **2** units per protein monomer.

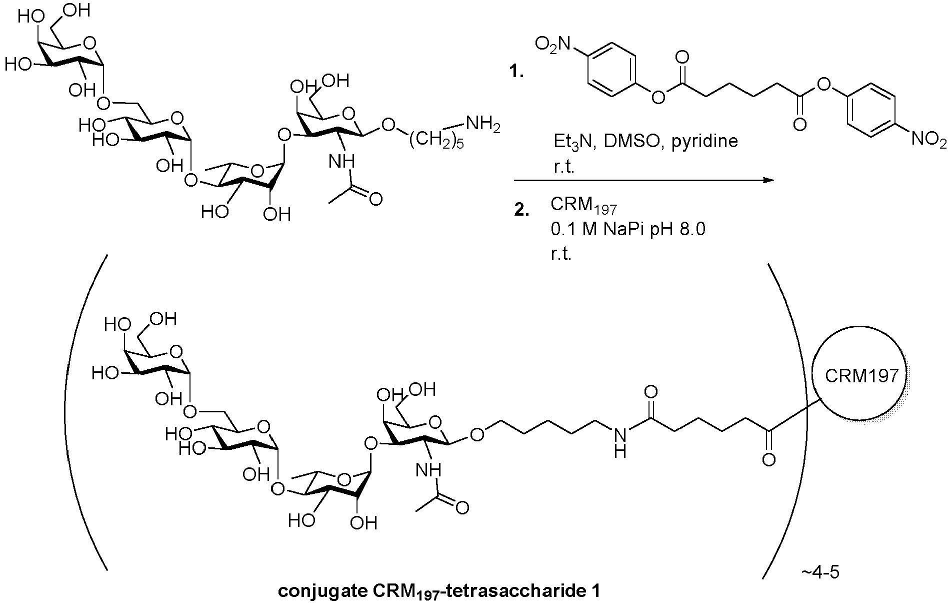

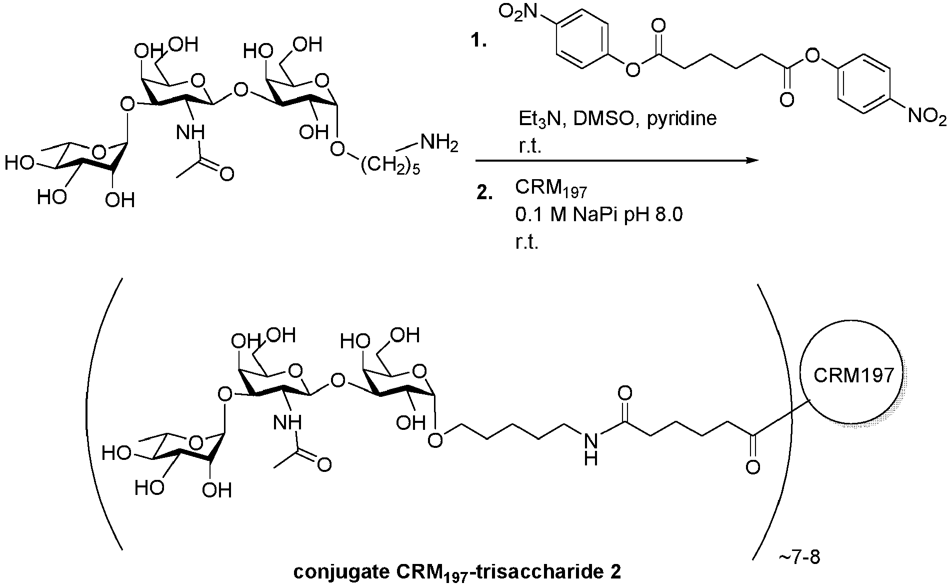

### B. Biological Evaluation

### Example B.1: Mice immunization and generation of polyclonal sera

### Immunization

The immunizations were approved by Landesamt für Gesundheit und Soziales Berlin, Germany (Approval ID: G 0329/18). 8 weeks old C57BL/6NRj mice (Janvier, Le Genest-Saint_Isle, France) were housed at Bundesinstitut für Risikobewertung, Berlin, Germany in groups of five animals in individually ventilated cages (Tecniplast, Hohenpeißenberg, Germany) under specific pathogen-free conditions. Housing and experiments were in accordance with institutional guidelines and with the regulations of the Federation of European Laboratory Animal Science Associations (FELASA). Mice were immunized subcutaneously (s.c.) with 1µg of glycoconjugate per injection. The glycoconjugates, together with Alum adjuvant, were diluted in sterile PBS to a final volume of 100 µL per dose. The animals were immunized on day 0 (primary immunization) and boosted on days 14, 28, and final boosting on day 196. Blood (max. 80µL) and saliva were collected every two weeks. The mice were fixed with on hand and a swap was used to collect the saliva and was afterwards centrifuged at 400 g for 5 min. After coagulation at room temperature, serum was separated by centrifugation at 2,000 g for 10 min. The sera and saliva were frozen at -20°C until further usage.

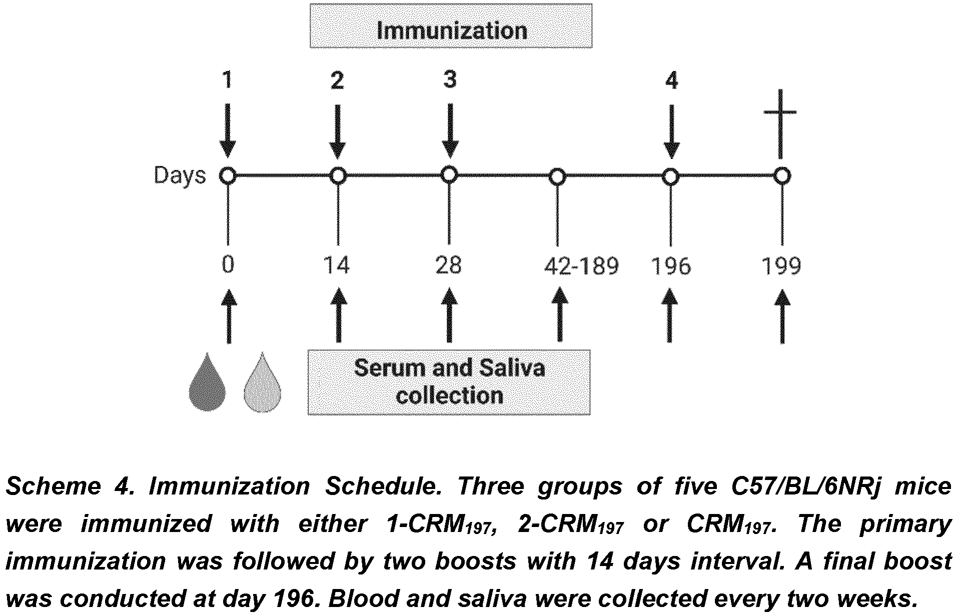

### Hybridoma Generation

The spleens were taken out of the mice and mashed with 5 mL serum-free RPMI w/o additions. The supernatant was collected in a 50 mL Falcon and filled up with serum-free RPMI w/o additions. The tube was centrifuged at 300 g for 10 min at RT and the pellet was washed with 50 mL serum-free RPMI w/o additions and resuspended in 15 mL serum-free RPMI w/o additions. X63-Ag8 myeloma cells were washed two times in 50 mL serum-free RPMI w/o additions and then transferred to the spleen cells. After centrifugation the pellet was gently disrupted by tapping the bottom of the tube and placed in a 37°C water bath and kept in there during the fusion. 1.5 mL pre-warmed 50% PEG 1500 were gradually added to the pellet over a period of 1 min, while continually stirring the cells gently with the pipette tip. The cells were stirred for 1 min and while gently swirling the tube, pre-warmed serum-free RPMI w/o additions medium was slowly added: 1 ml over 60 s, 3 ml over 60 s and 16 ml over 120 s. The cells were immediately centrifuged at 300 g for 10 min in an uncooled centrifuge and incubated for 5 min at RT. The supernatant was removed and the cells gently resuspended in 200 mL selection medium (RPMI 500mL, 2 mM L-Glutamine, 0.05 mg/mL Gentamycin, 1% Penicillin/Streptomycin, 10% FCS, 1% Non-Essential Amino Acids, 1xHAT (Hypoxanthin/Aminopterin/Thymidin), 1x BM-Condimed H1 supplement). 200 µL/well of cells were plated onto 96-well flat-bottom plates and incubated at 37°C and 5% CO₂ Binding to synthetic glycans was checked via Glycan microarray analysis after 10 days. Subclonings were performed until the clones were monoclonal. Three hybridoma (F5, F12 and G12) showed significant binding to 1. The cells were expanded and slowly adapted to ISF- 1 w/o FCS. The supernatant containing the antibody was harvested and sterile filtered 10 days after the last splitting, when the cells were about 100% dead. The antibodies were purified using a protein A/G column.

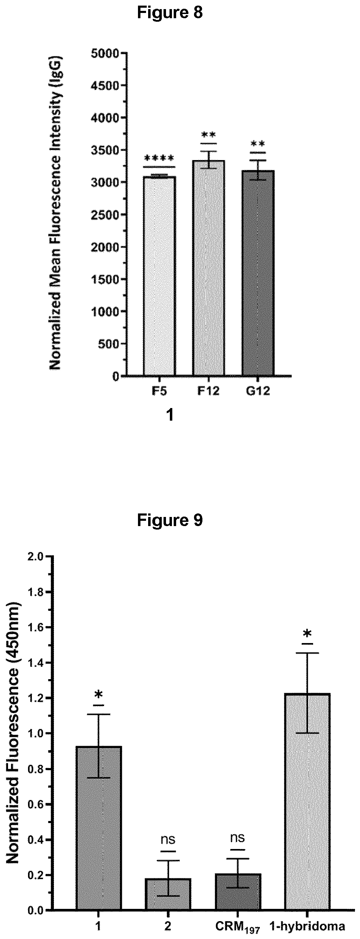

The isolated B-cells of the immunized mice were used for monoclonal antibody production via hybridoma technique. Binding of the antibodies to the synthetic glycan was shown by glycan array. Three clones (F5, F12 and G12) showed a significant binding to **1** (Figure 8).

### Glycan Microarray Experiments (mice sera, mice saliva and hybridoma)

Synthetic polysaccharides containing an amine linker as well as protein controls were immobilized on glass microarray slides. The slides were then quenched, blocked with 1% BSA-PBS, and stored at 4°C until use. A FlexWell 64 grid was attached, and the slides were incubated with mice sera diluted 1:100 or with pooled saliva samples diluted 1:5 in 1% BSA-PBS (w/v) or hybridoma culture supernatant in a humidification chamber for 1 hour at 37 °C. After washing three times with PBST, slides were incubated with fluorescently labeled secondary antibodies (Alexa Fluor^{®} 488 AffiniPure Goat Anti-Mouse IgG, Fcγ fragment specific, Jackson ImmunoResearch, Goat anti-Mouse IgM (Heavy chain) Secondary Antibody, Alexa Fluor^{™} 647, invitrogen, Goat Anti-Mouse IgA alpha chain (DyLight^{®} 594), abcam) each diluted 1:400 in blocking buffer.

Slides were incubated for 1 hour at 37 °C in a humidification chamber, washed three times with PBST, rinsed with deionized water, and dried by centrifugation. Fluorescence signals were measured with a Genepix 4300A device (Molecular Devices, Sunnyvale, CA, USA).

The photomultiplier tube (PMT) voltage was adjusted to avoid oversaturation of the signals. Data were analyzed using GenePix Pro 7 software (Molecular Devices, Sunnyvale, CA, USA).

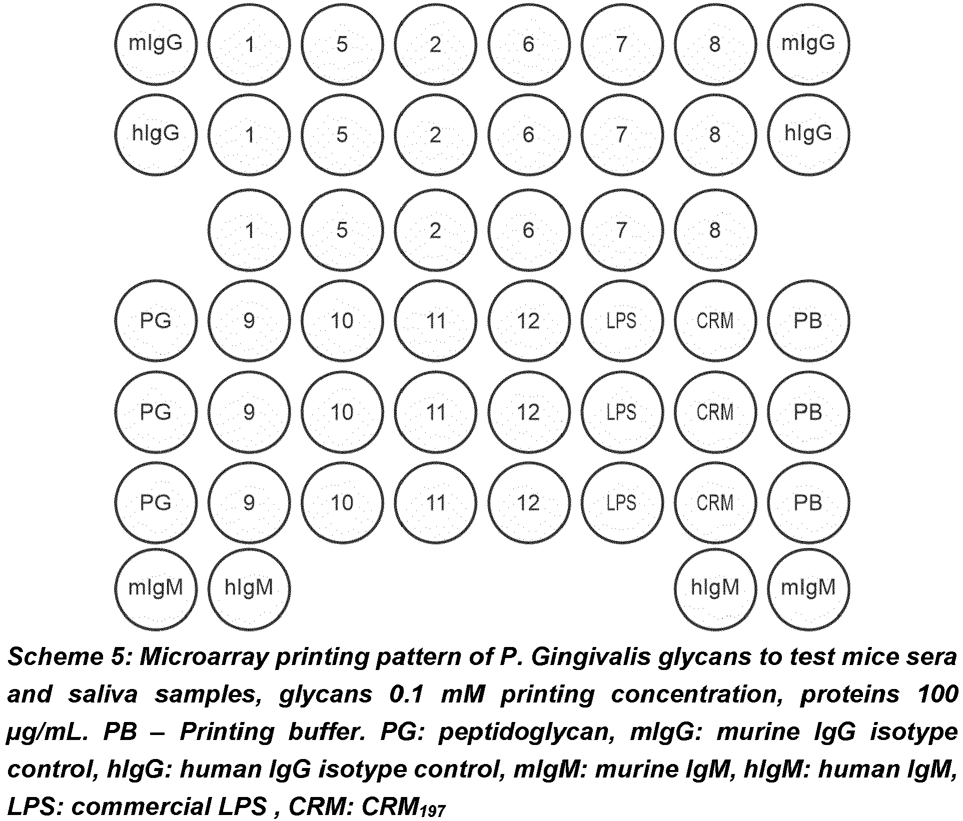

### P. gingivalis W50 culture

*P. gingivalis* W50 (ATCC-53978) bacteria were cultured for 5 to 7 days on Columbia Blood agar plates (VWR, Belgium) under anaerobic conditions. For liquid cultures, bacteria were cultured overnight in Supplemented Tryptic Soy Broth (ATCC medium 2722). Anaerobic conditions were created in an anaerobic culture jar supplemented with a 2.5 l Thermo Scientific^{™} Oxoid^{™} AnaeroGen^{™} pack (Thermo Fisher Scientific). 100 ml of ATCC Medium 2722 is composed of 30.0 g Tryptic Soy Broth, 5.0 g Yeast Extract, 0.5g L-cysteine hydrochloride, 1.0 mL Hemin stock (5µg/mL), 0.5 g Hemin, 1.74 g K₂HPO₄, 0.2 mL Vitamin K3 stock (5mg/mL; final concentration 1.0 µg/mL) and 100 mL distilled water. The medium was autoclaved at 121°C after preparation.

### Bacterial ELISA with P. gingivalis W50

Bacteria were cultured overnight in Supplemented Tryptic Soy Broth to obtain different CDs. Bacteria were grown to an OD of 0.7 (~9.4 x 10¹⁰ cfu) and centrifuged at 3000 g during 5 min. Supernatant was discarded. Bacteria were washed three times with PBS and were afterwards diluted in 10 ml of carbonate bicarbonate coating buffer. High-binding flat bottom ELISA plates (Corning) were coated with 100 ul of diluted bacteria and were incubated at 4 °C overnight. ELISA plates were washed 3 times with PBST. Sera of day 56 after immunization, as well as hybridoma supernatant was diluted 1:100 in PBS/BSA. 50 ul of serum dilution in triplicates were pipetted into corresponding wells and incubated at RT for 2 hours. Plates were washed 3 times with PBST.

Per well 50 ul of goat- anti mouse IgG HRP antibody (Dianova) was added at a dilution of 1:10000 and incubated for 3 hours. Plates were washed 3 times with PBST. 100 ul of substrate TMB was added to each well. After 10 min the reaction was stopped by adding H₂SO₄. The absorbance was read at 450 nm in a CLARIOstar Plus plate reader (BMG Labtech).

### Statistical Analysis

Graphing and statistical analysis (one sample t-test) were performed using GraphPad Prism 9.3.1.

### Glycan Microarray Experiments (human sera, human saliva)

The rapid synthesis of a library of LPS fragments by AGA in combination with following high throughput glycan microarray studies is a fast and efficient tool to identify an active epitope. The determination of the active epitope is essential for the design of conjugate vaccines, because the identified antigen will be responsible for the generation of specific antibodies after immunization in order to produce a protective immune response. In previous studies, *P. gingivalis* infected patients showed to form IgG and IgA antibodies in blood and saliva against pathogen-specific antigens.

Here, human sera and saliva from *P. gingivalis* infected patients were used to screen antibodies against the synthetic glycan library **1-11** to determine whether LPS fragments are potential leads for vaccine development.

Samples were taken from a pool of patients at the Charit6 Zahnklinik (provided by Prof. Dr. Henrik Dommisch). The dental status of three groups of patients was defined as: healthy (20 patients, no clinical indications for CP), infected (16 patients, CP stage III or stage IV) and recovered from CP (six patients). Recovered patients were treated from CP stage III or stage IV at the Charit6 Zahnklinik by resective and regenerative surgery and execution of two to four professional dental cleanings per year.

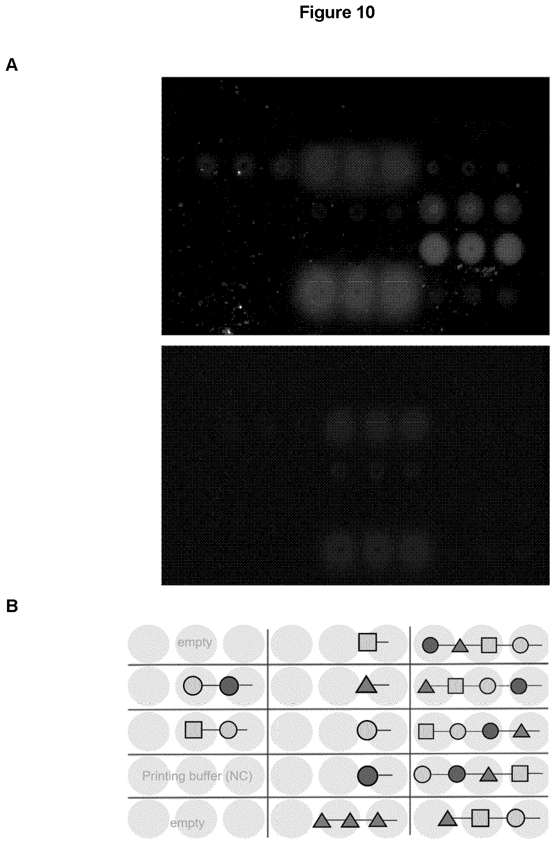

The synthesized LPS fragments of *P. gingivalis* were immobilized at NHS-activated carboxyl-functionalized glass slides in triplicates (Figure 10 A, B). Additionally, a positive glycan control containing the α(1→2)-trirhamnose **(5.06)** was used in the assay, because most humans develop antibodies against this pathogen-associated glycan

### Results

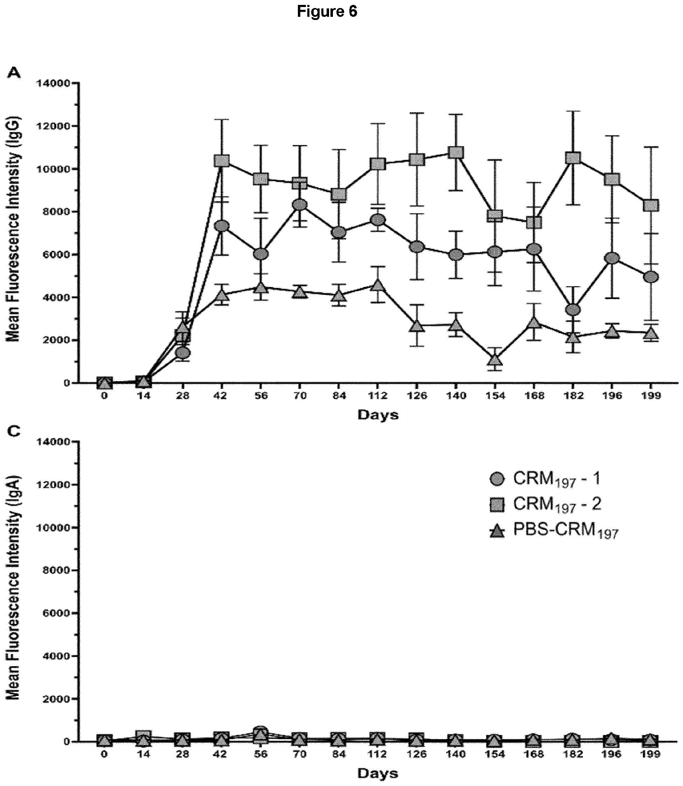

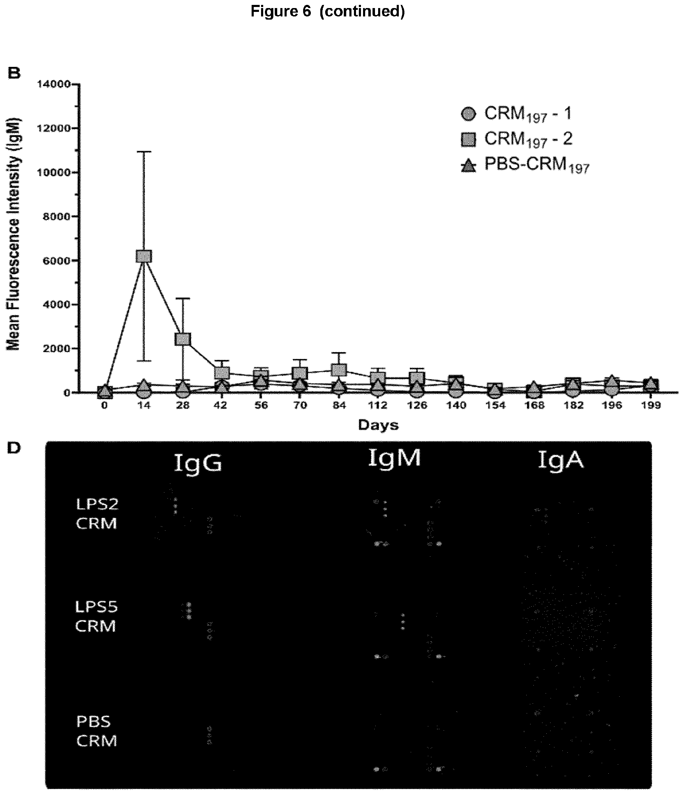

Immunization with the glycoconjugate vaccines lead to the development of glycan specific antibodies in sera of mice. Mice immunized with **1**-CRM197 developed antibodies binding to **1** on the glycan array. Mice immunized with **2**-CRM197 developed an immune response against **2.** IgG antibody levels started to increase 28 days after immunization and stayed at high levels throughout the course of the experiment (Figure 6 A). Antibody levels against **2** in mice immunized with **2-**CRM197 were in general slightly higher than levels against **1** in mice immunized with **1-**CRM197. This could be due to higher loading of **2** antigens onto CRM197. In addition to that, all mice developed an antibody response against CRM197. Control mice immunized with CRM197 only developed stable antibody responses against CRM197 and did not show immune responses against the tested synthetic structures.

The IgM response against **2** peaked at day 14 with still relatively high IgM levels on day 28. IgM-responses against **1** and CRM197 remained at low levels (Figure 6 B).

Serum IgA levels were in general very low with a signal below a MFI of 400 (Figure 6 C). The conjugate could hence not induce strong serum IgA immune responses against **1** and **2.**

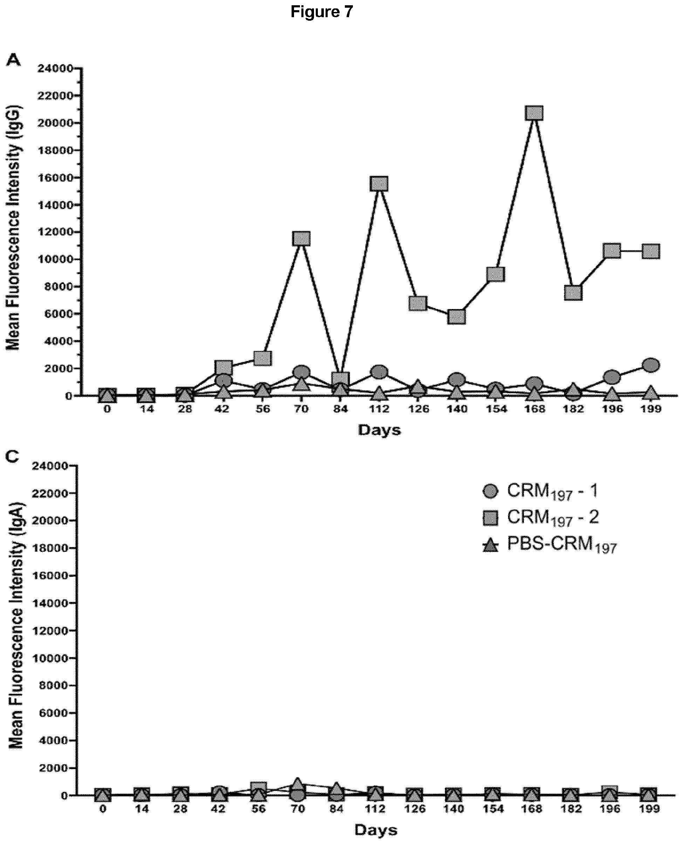

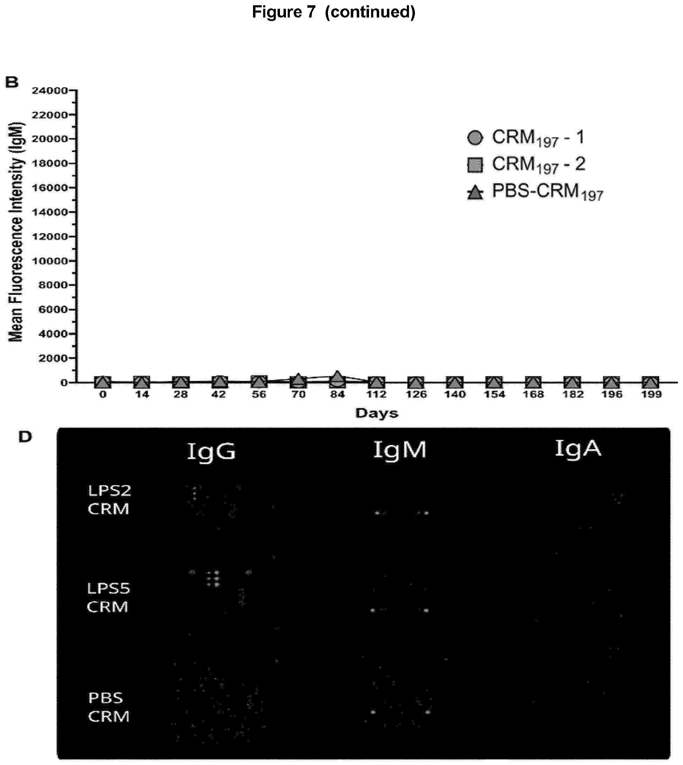

Since *P. gingivalis* primarily affects the oral cavity, antibody titers against the synthetic structures in saliva were tested on the glycan array. Saliva of all 5 mice of each group were pooled and diluted. High mucosal IgG responses against **2** could be determined in the **2**-CRM197 group from day 70 on after immunization (Figure 7 A). 1-CRM197 and PBS-CRM197 did not induce an IgG immune response against **1** or CRM197 respectively. IgM and IgA antibodies were very low across all groups in saliva samples (Figure 7 B and 7 C). Mucosal IgA formation could not be observed on the glycan array. However, antibody titers could be too low to be detected, since only very few microliters of saliva could be recovered per mouse.

Binding of mice sera and mice saliva samples to synthetic glycans was shown by glycan array. A bacterial ELISA was performed to show binding to native LPS structures on *P. gingivalis* W50 as well. Figure 9 shows that serum-IgG of mice immunized with **1**-CRM197 are binding to *P. gingivalis* W50. Antibodies targeting **1** generated by the hybridoma technology were also binding to *P. gingivalis* W50.

This is consistent with the previous findings that IgG in serum of patients infected with *P. gingivalis* binds to synthetic **1**. Binding to native structures is especially important for further vaccine development, since the epitopes can be hidden or presented in a different way on bacteria than on glycan arrays.

Immunization with the **1**-CRM197 glycoconjugate vaccine induces serum IgG-antibodies that are capable of binding bacterial LPS. In addition to that, the monoclonal antibodies generated against **1** are binding to *P. gingivalis* on the array. The **2**-CRM197 glycoconjugate vaccine candidate did not induce antibody binding to native bacterial LPS. The normalized fluorescence was in a similar range as binding of sera from mice vaccinated with the PBS-CRM197 control.

Binding of human sera and human saliva samples to synthetic glycans was shown by glycan array. While *P. gingivalis* was identified in the dental plaque of all patients, the bacterial amount was generally higher for chronical periodontitis patients (mean(cT) of 18.52) and recovered patients (mean(cT) of 21.70) compared to healthy individuals (mean(cT) of 24.71, Table 1).

**Table 1: CT values (PCR) of the three patient groups (dental healthy, chronical periodontitis and recovered from chronical periodontitis). Patients with outlaying high antibody-binding in microarray studies are marked in orange.**

| **Dental status: Healthy** | | **Dental status: CP** | | **Dental status: Recovered from CP** | |
|---|---|---|---|---|---|
| Mean(cT) = 24.71 | | Mean(cT) = 18.52 | | Mean(cT) = 21.70 | |
| **Sample** | **cT** | **Sample** | **cT** | **Sample** | **cT** |
| P-H-00 | 21.82 | P-I-01 | 28.14 | P-T-01 | 21.09 |
| P-H-01 | 21.9 | P-I-02 | 23.42 | P-T-02 | 21.95 |
| P-H-02 | 24.3 | P-I-03 | 16.15 | P-T-03 | 22.13 |
| P-H-03 | 25.32 | P-I-04 | 19.9 | P-T-04 | 23.65 |
| P-H-04 | 25.72 | P-I-05 | 18.55 | P-T-05 | 16.15 |
| P-H-05 | 25.08 | P-I-06 | 22.93 | P-T-06 | 25.22 |
| P-H-06 | 24.94 | P-I-07 | 17.62 | | |
| P-H-07 | 30.85 | P-I-08 | 18.66 | | |
| P-H-08 | 24.52 | P-I-09 | 17.35 | | |
| P-H-09 | 22.15 | P-I-10 | 23.54 | | |
| P-H-10 | 25.74 | P-I-11 | 14.53 | | |
| P-H-11 | 23.33 | P-I-12 | 19.23 | | |
| P-H-12 | 24.17 | P-I-13 | 16.71 | | |
| P-H-13 | 25.25 | P-I-14 | 17.94 | | |
| P-H-14 | 24.72 | P-I-15 | 16.72 | | |
| P-H-15 | 25.01 | P-I-16 | 14.51 | | |
| P-H-16 | 25.04 | | | | |
| P-H-17 | 24.76 | | | | |
| P-H-18 | 25.96 | | | | |
| P-H-19 | 23.59 | | | | |

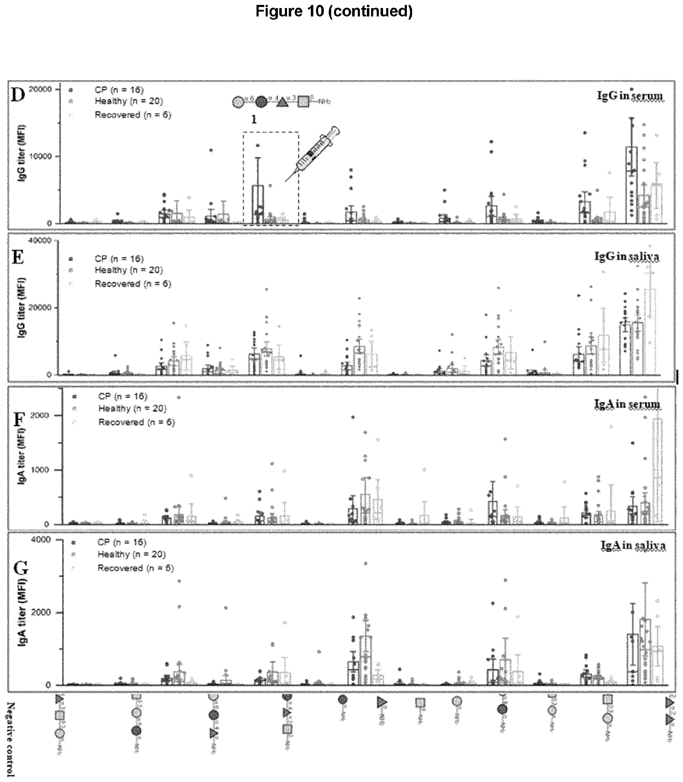

The glycan microarray analysis of human saliva compared to human serum generally showed IgG antibody binding to the same LPS-fragments **5, 3, 9, 7, 4** as well as the positive control **5.06** (Figure 10 D, E). While only saliva of CP patients with high bacterial load show IgG antibody binding to the listed LPS glycan structures, also serum of healthy and treated individuals contains IgG antibodies against those structures. It is generally known, that human serum contains antibodies against rhamnose structures as well as α-galactose structures, because those units are common motifs at bacterial surfaces. The specific binding of antibodies in saliva of chronical periodontitis patients to some LPS-glycan structures is an evidence for the active mucosal IgG antibody production in the advanced affection of *P. gingivalis* in CP. IgG antibodies against positive control **5.06** in saliva were also observed in healthy individuals, but significantly less than in chronical periodontitis patients and recovered chronical periodontitis patients, which supports this result. Generally, it is known, that salivary antibodies reflect both mucosal and systemic immunity.

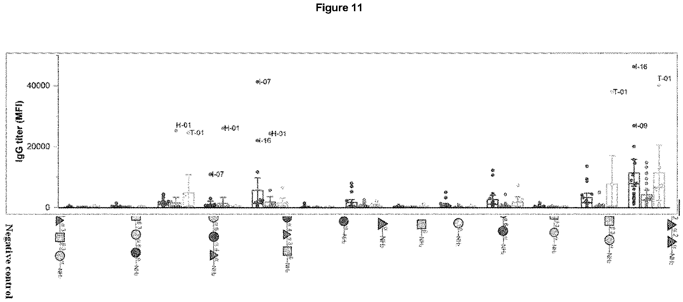

The glycan microarray studies using saliva show a few outlying data points (Figure 11). CP patients I-07, I-09 and I-16 for example have the highest MFI to specific glycans and at the same time the highest bacterial load according to the low cT value (Table 1) as well as particularly distinct clinical indications for CP including significant damage to the attachment apparatus, deep periodontal lesions and deep intrabony defects. This shows that the stage of periodontitis and bacterial load is proportional to the IgG titer. CP treated patient T-01 also shows IgG binding to specific glycans, while the remaining treated patient group is generally comparable with healthy individuals. Interestingly, the frequent professional dental cleaning was skipped for more than ten months in case of T-01. Therefore, the high IgG titer for T-01 could be a first sign of a resurgence of the infection, while clinical indications for CP were not visible yet. Further time-dependent screenings on treated patients before and after the professional dental cleaning and after a longer period of time without such cleaning could give an idea about a potential of early diagnosis for high CP risk or CP resurgence using the here described glycan array studies.

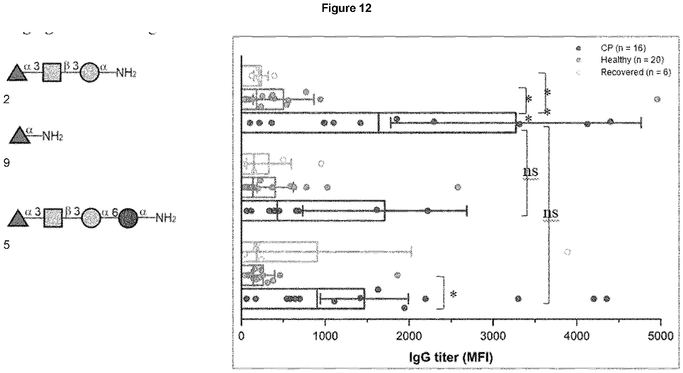

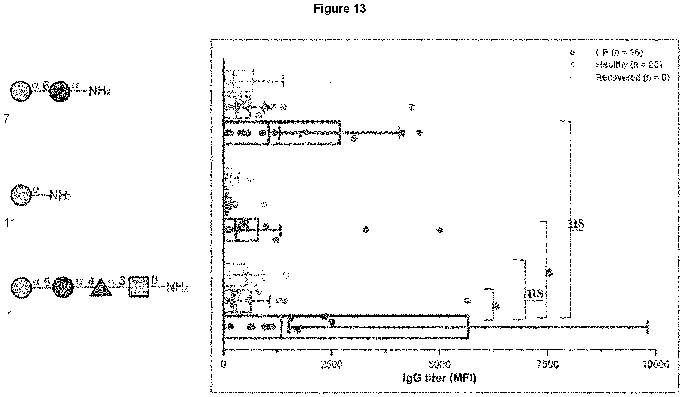

The epitopes that show binding to IgG antibodies in saliva and blood are generally LPS-fragments with a terminal α(1→3)-rhamnose unit (**5, 2** and **9,** Figure 12) or a terminal α(1→6)-galactose unit (**3, 7** and **11,** Figure 13.).

**Fehler! Verweisquelle konnte nicht gefunden werden.** 12 compares the mean fluorescence intensity (MFI) indicating IgG titer of bound human IgG antibodies in saliva to terminal α(1→3)-rhamnose LPS-fragments. Interestingly, **4** trisaccharide shows higher IgG antibody binding than **5** tetrasaccharide, even though higher molecular weight glycan antigens are known to elicit a better immune response than lower molecular weight fragments. On the other hand, **2** trisaccharide shows higher amount of IgG antibody binding than to rhamnose monomer **9.** This indicates, that observed IgG antibody binding to **2** is not only attributed to general antibodies against common bacterial rhamnose motifs, but more likely also to the actual LPS-fragment of *P. gingivalis.* These values, however, have low statistical significance (P ≤ .1), likely due to the different cT values inside each group. More significant results would probably be received in a larger CP group (n > 16). Nevertheless, the statistical significance of the stronger IgG binding observed for CP patients to **2** compared to low IgG binding for healthy and recovered patients is high (P ≤ .01) and is a clear evidence for the active mucosal IgG antibody production in the advanced affection of *P. gingivalis* in CP against **2.**

Figure 13 compares the bound human IgG antibodies in saliva to terminal α(1→6)-galactose LPS-fragments. **1** tetrasaccharide exhibits the strongest IgG interaction observed in these studies. Conversely, disaccharide **7** and monosaccharide **11** bind significantly less IgG (P ≤ .05). This suggests, that all four parts of the LPS repeating unit have an impact on the binding to IgG. The antibody binding not just arises from binding of general α(1→6)-galactose antibodies. Furthermore, IgG binding in CP saliva to **1** is significantly higher than in dental healthy patients (P ≤ .05). This supports the assumption, that epitope **1** is specific for *P. gingivalis.* The glycan microarray studies show that the order of the four tetrasaccharide fragments **1-3** and **5** in the LPS repeating unit of *P. gingivalis* is important for the binding to IgG antibodies. α(1→6)-Galactose terminal LPS-fragment **1** shows the highest amount of bound IgG antibodies in this study.

As IgA antibodies are the predominant mucosal antibody species, it was also screened for IgA antibody binding (Figure 10 F, G). Here, approximately ten-fold lower MFI for IgA antibody binding was observed, compared to IgG binding. Saliva samples showed stronger binding, which is expectable due to the high IgA content in saliva. Only positive control rhamnose structures **9** and **5.06** as well as α-galactose dimer **7** show significant binding of IgA in saliva and serum - strucutures that are common for many bacteria. There is also no significant difference observed between CP patients and dental healthy and recovered individuals. Dental healthy individuals actually show slightly higher IgA levels against the common bacteria glycan structures. The screening for IgG antibodies using a fluorescent-labeled secondary antibody (Fc-AF 647) in glycan microarray studies is commonly used to evaluate the immunogenicity of glycans and has already provided reliable results. The screening of IgA antibodies has been included in very few glycan microarray studies. Therefore, little is known on the IgA binding strength, to the immobilized glycans as well as the competition to IgG binding. However, it was clearly observed that IgA does not bind to antigen structures that are specific for *P. gingivalis* and only IgG antibodies seem to be actively produced against **1** in the case of a severe form of CP.

## Claims

1. A saccharide of general formula **(I)**
**H-Uₓ₊₂-Uₓ₊₁-Uₓ-T-O-L-NH₂** **(I)**
wherein
x is an integer selected from 1 and 3; 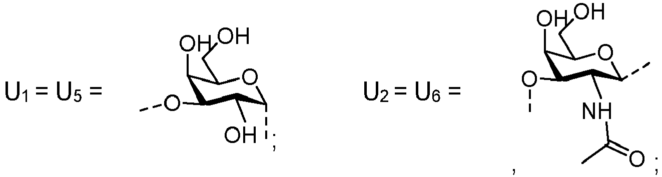 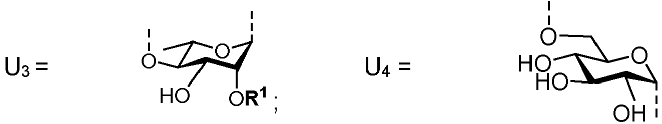
-T- represents a bond, -Uₓ₊₃-, -Uₓ₊₃-Uₓ₊₂-, -Uₓ₊₃-Uₓ₊₂-Uₓ₊₁-, or -[Uₓ₊₃-Uₓ₊₂-Uₓ₊₁-Uₓ]ₙ- with n being an integer selected from 1, 2 and 3;
R¹ represents -H or -PO₂-O-CH₂CH₂-NH₂;
L represents a linker;
or a pharmaceutically acceptable salt thereof.

2. The saccharide according to claim 1, wherein R¹ represents -H, or a pharmaceutically acceptable salt thereof.

3. The saccharide according to claim 1 or 2, wherein -T- represents a bond or
-Uₓ₊₃-, or a pharmaceutically acceptable salt thereof.

4. The saccharide according to claim 1 of general formula **(III)**
**H-Uₓ₊₂-Uₓ₊₁-Uₓ-[Uₓ₊₃-Uₓ₊₂-Uₓ₊₁-Uₓ]ₙ-O-L-NH₂** **(III)**
wherein Uₓ, Uₓ₊₁, Uₓ₊₂, Uₓ₊₃, x, L, and n have the meanings as defined in claim 1.
U₁ = U₅ = 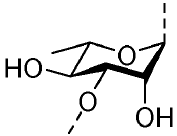 or a pharmaceutically acceptable salt thereof.

5. The saccharide according to any one of the claims 1 to 4, wherein
**-L-** represents **-L^{a}-, -L^{a}-L^{e}-, -L^{a}-L^{b}-L^{e}-,** or **-L^{a}-L^{d}-L^{e}-** ;
**-L^{a}-** represents -(CH₂)ₒ-, -(CH₂-CH₂-O)ₒ-C₂H₄-, or -(CH₂-CH₂-O)ₒ-CH₂ ;
**-L^{b}-** represents -O-, -NH-CO-NH-, -NH-CO-CH₂-NH-, -NH-CO-;
**-L^{d}-** represents -(CH₂)_{q}-, -(CH(OH))_{q}-, -(CF₂)_{q}-, -(CH₂-CH₂-O)_{q}-C_{2H4}-, or -(CH₂-CH₂-O)_{q}-CH₂- ;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ; and
**o, q, p1** and **p2** are independently of each other an integer selected from 1, 2, 3, 4, 5, and 6.

6. The saccharide according to any one of the claims 1 to 4, wherein
**-L-** represents -(CH₂)ₒ- and o is an integer selected from 2, 3, 4, 5, 6, 7 and 8.

7. A conjugate comprising a saccharide of general formula **(I)** according to any of the claims 1 - 6 covalently linked to an immunogenic carrier through the nitrogen atom of the -O-L-NH₂ group.

8. The conjugate according to claim 7, wherein the immunogenic carrier is a carrier protein, a *P. gingivalis* outer membrane protein or a glycosphingolipid.

9. The conjugate according to claim 7 of general formula **(IV)**
**[H-Uₓ₊₂-Uₓ₊₁-Uₓ-T-O-L-NH-W]ₘ-IM** **(IV)**
wherein m is comprised between about 2 and about 18;
-W- is selected from: 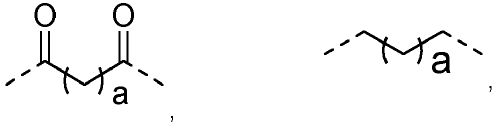 and 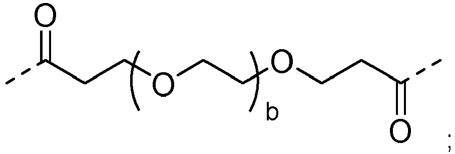
a represents an integer from 1 to 10;
b represents an integer from 1 to 4;
**IM** represents an immunogenic carrier, and
Uₓ, Uₓ₊₁, Uₓ₊₂, Uₓ₊₃, x, n and L have the meanings as defined in any one of the claims 1 - 6.

10. The conjugate according to claim 7 of general formula **(V)**
**[H-Uₓ₊₂-Uₓ₊₁-Uₓ-T-O-L-NH-W]ₘ-CRM₁₉₇** **(V)**
wherein m is comprised between about 2 and about 18;
-W- is selected from: 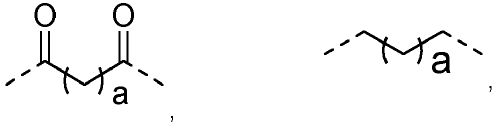 and 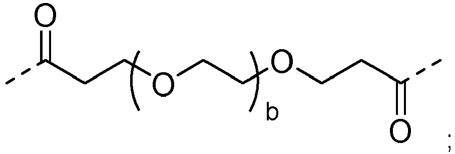
a represents an integer from 1 to 10;
b represents an integer from 1 to 4; and
Uₓ, Uₓ₊₁, Uₓ₊₂, Uₓ₊₃, x, n and L have the meanings as defined in any one of the claims 1 - 6.

11. The conjugate according to claim 9 or 10, wherein
**-L-** represents **-L^{a}-, -L^{a}-L^{e}-, -L^{a}-L^{b}-L^{e}-,** or **-L^{a}-L^{d}-L^{e}-** ;
-L^{a}- represents -(CH₂)ₒ-, -(CH₂-CH₂-O)ₒ-C₂H₄-, or -(CH₂-CH₂-O)ₒ-CH₂;
-L^{b}- represents -O-, -NH-CO-NH-, -NH-CO-CH₂-NH-, -NH-CO-;
-L^{d}- represents -(CH₂)_{q}-, -(CH(OH))_{q}-, -(CF₂)_{q}-, -(CH₂-CH₂-O)_{q}-C₂H₄-, or -(CH₂₋CH₂-O)_{q}-CH₂-;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6.

12. The conjugate according to claim 8, wherein the glycosphingolipid is (2S,3S,4R)-1-(α-D-galactopyranosyl)-2-hexacosanoylaminooctadecane-3,4-diol.

13. A pharmaceutical composition comprising at least one saccharide according to any one of the claims 1 - 6 and/or at least one conjugate according any one of the claims 7 - 12 as an active ingredient together with at least one pharmaceutically acceptable adjuvant and/or excipient.

14. The saccharide according to any one of the claims 1 - 6, the conjugate according to any one of the claims 7 - 12, or the pharmaceutical composition according to claim 13 for use in prevention and/or treatment of disease associated with *Porphyromonas gingivalis,* wherein the disease is periodontal disease, Alzheimer's disease, atherosclerosis, or rheumatoid arthritis.

15. The saccharide according to any one of the claims 1 - 6 for use as a marker in immunological assays for the detection of antibodies against *Porphyromonas gingivalis.*
